# EUROPEAN PATENT APPLICATION

(11) **EP 4 458 364 A1**
(43) Date of publication of application: **06.11.2024**
(21) Application number: 24174068.7
(22) Date of filing: 03.05.2024
(51) Int. Cl.: A61K 35/744, A61K 35/747, A61K 45/06, A61P 35/00, A61P 37/02

(54) **COMPOSITIONS FOR ENHANCING IMMUNOTHERAPY EFFICACY IN COLORECTAL CANCER TREATMENT**

(30) Priority: 05.05.2023 US 202363464325 P
(71) Applicant: The Chinese University Of Hong Kong, New Territories, Hong Kong (HK)
(72) Inventor: YU, Jun, Hong Kong (CN); WU, Ka Kei, Hong Kong (CN); LI, Qing, Hong Kong (CN); KANG, Xing, Hong Kong (CN); FONG, Winnie, Hong Kong (CN)
(74) Representative: Matter IP Limited

(57) **Abstract**

The present invention provides for compositions and methods for improving efficacy of immunotherapy for colorectal cancer.

## Description

### BACKGROUND OF THE INVENTION

Colorectal cancer (CRC) is the third most common cancer and the third leading cause of cancer mortality worldwide, with an estimated incidence of over 1 million new cases and a mortality of more than 500,000 deaths per year. Several intrinsic (*e.g.,* age, male gender, ethnicity, diabetes mellitus, obesity and inflammatory bowel disease) and extrinsic (*e.g.,* cigarette smoking, inadequate intake of fiber, high consumption of alcohol, red meat and high-fat diet) factors are associated with increased risks for colorectal cancer. The epidemiology of colon cancer is under dynamic changes owing to the changing prevalence and distribution of risk factors. In this regard, colon cancer incidence in many developing countries, including Asian countries, has increased 2- to 4-fold over the last two decades and has now reached an alarming rate, with Westernization of diet playing a pivotal role.

About 38 trillion bacteria exist in the human intestine. In view of their symbiotic and co-operative relationship with the human body, these bacteria have a close association with the pathogenesis and progression of colon cancer. The association of colon cancer with altered gut microbiota has been studied in different populations, with certain bacterial species identified for their potential roles, either beneficial or detrimental, in tumourigenesis. Disease management and facilitation of treatment among colon cancer patients by way of modifying the profile of gut microorganisms is a highly desirable means of medical intervention due to its high efficacy, low cost, and low risk of side effects. Immune checkpoint blockade (ICB) therapy has shown great promise in cancer treatment and has seen increased use in colorectal cancer treatment in the past decade. Also known as anti-PD1/PDL1 (programmed cell death protein 1/programmed death ligand 1) and/or anti-CTLA4 (cytotoxic T-lymphocyte-associated antigen-4) therapy, ICB therapy acts by removing the "brake" signal of T cell activation and elicits an anti-cancer immune response against cancer cells in order to suppress disease progression. There are notable limitations associated with this therapeutic approach, however, including drug resistance. Thus, new methods and compositions for the purposes of improving colon cancer treatment efficacy are desired.

### BRIEF SUMMARY OF THE INVENTION

The inventors discovered that certain gut microbial species and their metabolites can suppress the proliferation of colorectal cancer cells, especially when applied in combination with immunotherapeutic agents for treating colon cancer. The identified microorganisms and metabolites provide new methods and compositions for enhancing or improving the therapeutic efficacy of immunotherapy for the treatment of colorectal cancer.

In a first aspect, the present invention provides a composition for use in improving or enhancing immunotherapy efficacy in a human patient being treated for colon cancer. The composition contains an effective amount of (1) any one or any two or three of *Lactobacillus gallinarum, Roseburia intestinalis,* and *Streptococcus salivarius* K12; and (2) one or more physiologically acceptable excipients. In some embodiments, the composition comprises an effective amount of two or three of *L. gallinarum, R. intestinalis,* and *S. salivarius K12.* In some embodiments, the composition comprises a total of about 10⁶ to about 10¹⁴ colony-forming unit (CFU) of any one or any two or all three of *L. gallinarum, R. intestinalis,* and *S*. *salivarius K12.* In some embodiments, the composition further comprises indole-3-carboxylic acid (ICA). In some embodiments, the composition further comprises butyrate. In some embodiments, the composition is formulated for oral ingestion, *e.g.,* in the form of a food or beverage item. In some embodiments, the composition is formulated in a daily dosage comprising about 5 × 10⁹ CFU of *L. gallinarum* per kg patient bodyweight. In some embodiments, the composition is formulated in a daily dosage comprising about 5 × 10⁹ CFU of *R. intestinalis* per kg recipient bodyweight. In some embodiments, the composition is formulated in a daily dosage comprising about 5 × 10⁹ CFU of *S*. *salivarius K12* per kg recipient bodyweight.

In the second aspect, the present invention provides a method for improving or enhancing or augmenting immunotherapy efficacy in the treatment of colon cancer by administering to a subject in need thereof an effective amount of the composition described above and herein, namely containing an effective amount of (1) any one or more of *Lactobacillus gallinarum, Roseburia intestinalis,* and *Streptococcus salivarius* K12; and (2) one or more physiologically acceptable excipients. In some embodiments, the immunotherapy comprises administration of an effective amount of a PD1 inhibitor or a PDL1 inhibitor. In some embodiments, the PD1 or PDL1 inhibitor is an anti-PD1 or an-PDL1 antibody, respectively. In some embodiments, the PD1 inhibitor or PDL1 inhibitor is administered via injection, *e.g.,* via intravenous infusion. In some embodiments, the claimed method includes a step of administering to the subject one composition comprising any one or more of *L. gallinarum, R. intestinalis,* and *S. salivarius K12.* In some embodiments, the method comprises the step of administering to the subject two or more compositions comprising any two or all three of *L. gallinarum, R. intestinalis,* and *S. salivarius K12,* optionally further comprising ICA and/or butyrate. In some embodiments, the administering step comprises oral ingestion of the composition, for example, the administering step comprises oral ingestion prior to or with food intake.

In a related aspect, the present invention provides a novel use of a composition for improving or enhancing immunotherapy efficacy in the treatment of colorectal cancer. The composition contains an effective amount of (1) any one or more of *Lactobacillus gallinarum, Roseburia intestinalis,* and *Streptococcus salivarius* K12; and (2) one or more physiologically acceptable excipients. In some embodiments, the immunotherapy comprises administration of an effective amount of a PD1 inhibitor or a PDL1 inhibitor. In some embodiments, the PD1/PDL1 inhibitor is an anti-PD1/PDL-1 antibody, respectively. In some embodiments, the PD1 inhibitor or PDL1 inhibitor is administered via injection, *e.g.,* via intravenous infusion. In some embodiments, the claimed method includes a step of administering to the subject one composition comprising any one or more of *L. gallinarum, R. intestinalis,* and *S. salivarius K12.* In some embodiments, the method comprises the step of administering to the subject two or more compositions comprising any two or all three of *L. gallinarum, R. intestinalis,* and *S*. *salivarius K12,* optionally further comprising ICA and/or butyrate. In some embodiments, the administering step comprises oral ingestion of the composition, for example, the administering step comprises oral ingestion prior to or with food intake.

In a third aspect, the present invention provides a kit for improving or enhancing immunotherapy efficacy in the treatment of colorectal cancer. The kit comprises a plurality of compositions each comprising an effective amount of one or more of *Lactobacillus gallinarum, Roseburia intestinalis,* and *Streptococcus salivarius* K12, optionally further comprising an effective amount of ICA and/or butyrate. In some embodiments, the kit includes at least two compositions, the first comprising an effective amount of any one or two or three of *L. gallinarum, R. intestinalis,* and *S. salivarius K12,* and the second comprising an effective amount of a PD-1 inhibitor or a PD-L1 inhibitor, *e.g.,* the PD1/PDL1 inhibitor is an anti-PD1/PD-L1 antibody, respectively. In some embodiments, the first composition is formulated for oral ingestion. In some embodiments, the second composition is formulated for injection. In some embodiments, the first composition is formulated in a daily dosage comprising about 10⁸ CFU of any one of *L. gallinarum, R. intestinalis,* or *S. salivarius K12.*

### BRIEF DESCRIPTION OF THE DRAWINGS

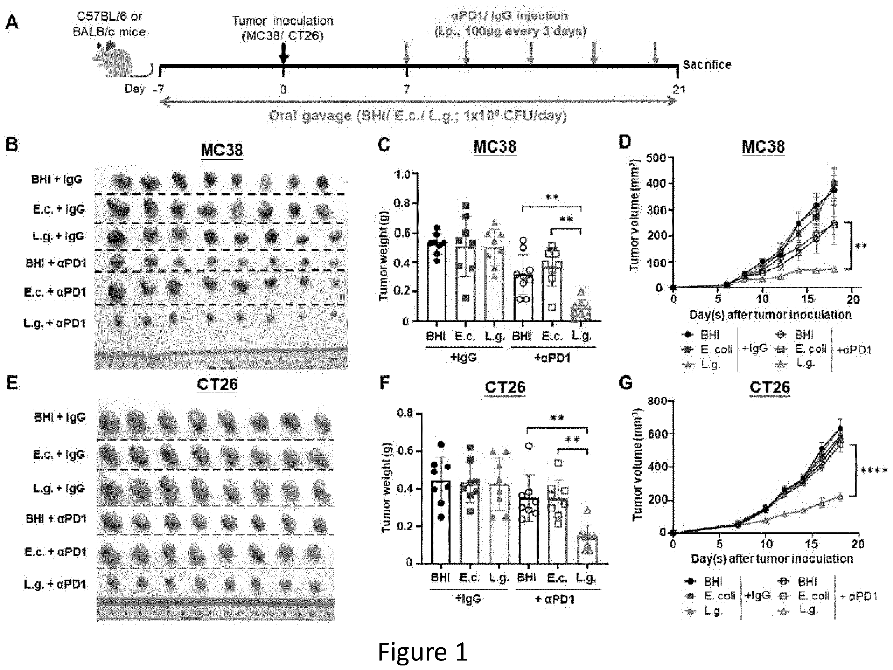

**Figures 1A - 1G** **illustrate *L. gallinarum* improved anti-PD1 efficacy in MC38 and CT26 syngeneic mouse models.** Figure 1A is a schematic diagram of experimental design for syngeneic mouse model. *L. gallinarum,* in combination with anti-PD1 therapy, significantly inhibited tumour growth in MC38 syngeneic mouse model (MSI-H model), as evidenced by representative tumour pictures as illustrated in Figure 1B, tumour weight as illustrated in Figure 1C, and tumour volume as illustrated in Figure 1D. *L. gallinarum* also promoted anti-PD1 efficacy in CT26 syngeneic mouse model, an MSI-L model, as supported by representative tumour pictures as illustrated in Figure 1E, tumour weight as illustrated in Figure 1F, and tumour volume as illustrated in Figure 1G. **P<0.01, ****P<0.0001.

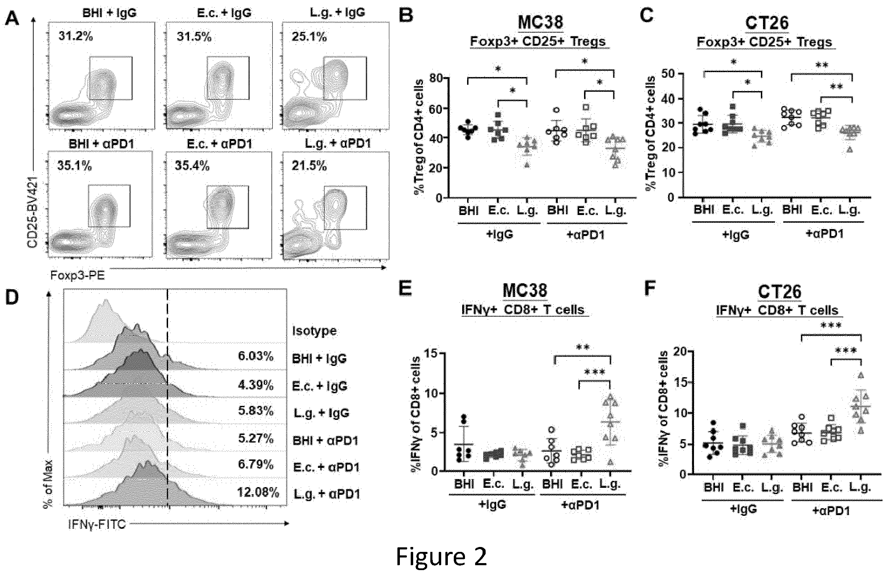

**Figures 2A - 2F** **illustrate *L. gallinarum* reduced Foxp3+ CD25+ Treg infiltration and increased IFNγ+ CD8+ T cells in the tumour microenvironment.** Figure 2A shows representative flow cytometry plots of Foxp3+ CD25+ Treg. *L. gallinarum* significantly reduced Foxp3+ CD25+ Treg infiltration in MC38 tumours as illustrated in Figure 2B, and CT26 tumours as illustrated in Figure 2C. Figure 2D shows representative flow cytometry histogram of IFNγ+ CD8+ T cells. *L. gallinarum,* in combination with anti-PD1, increased IFNγ+ CD8+ T cells in MC38 tumours as illustrated in Figure 2E, and CT26 tumours as illustrated in Figure 2F. *P<0.05, **P<0.01, ***P<0.001.

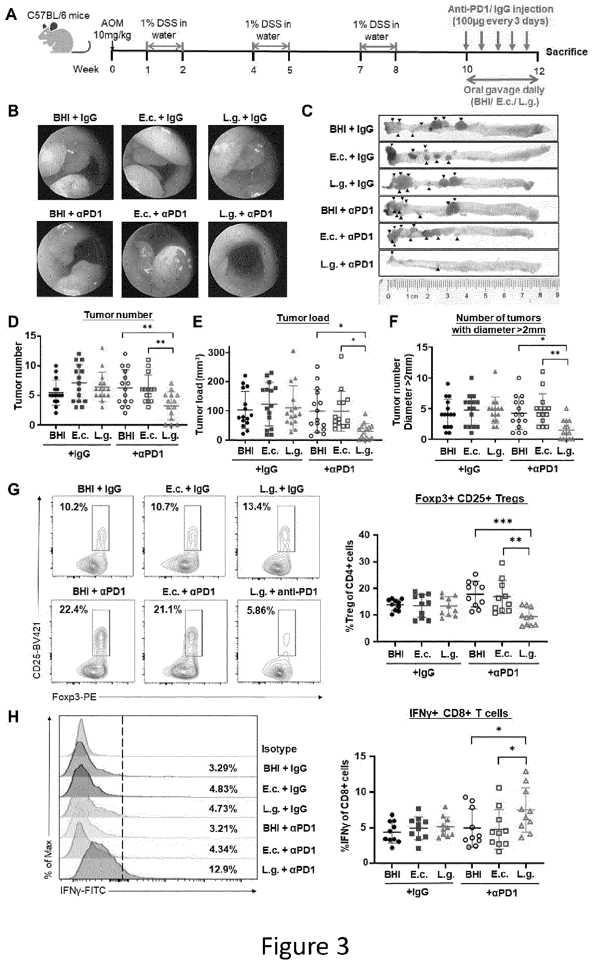

**Figures 3A - 3H** **show *L. gallinarum* improved anti-PD1 efficacy in AOM/DSS-induced CRC mouse model.** Figure 3A is a schematic diagram of experimental design for AOM/DSS-induced CRC model. Figure 3B shows representative colonoscopy images, and Figure 3C shows representative colon images of AOM/DSS-induced CRC tumourigenesis mouse model. *L. gallinarum,* in combination with anti-PD1, reduced tumour number as illustrated in Figure 3D, tumour load as illustrated in Figure 3E, and the number of large tumours (diameter larger than 2mm) as illustrated in Figure 3F. Figure 3G illustrates the percentage of Foxp3+ CD25+ Tregs in colonic tumour tissues. Figure 3H shows the percentage of IFNγ+ CD8+ T cells in colonic tumour tissues. *P<0.05, **P<0.01, ***P<0.001.

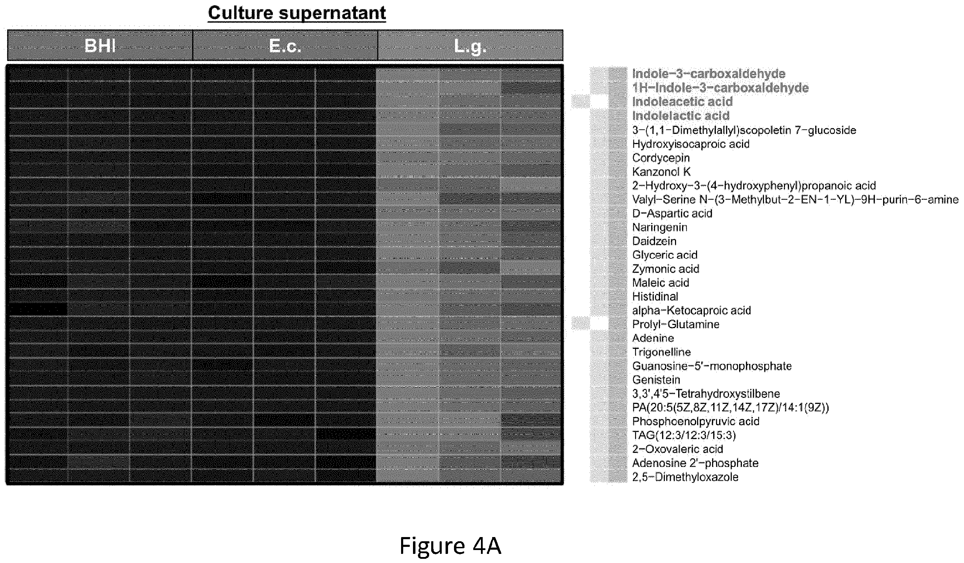

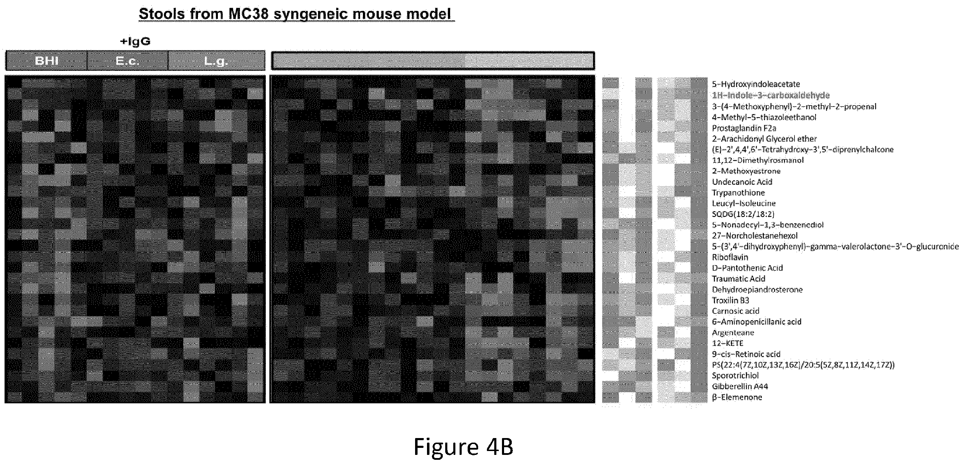

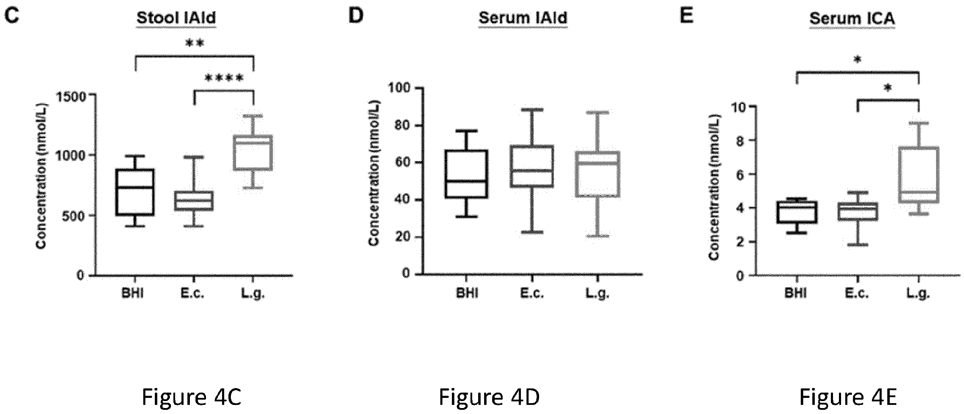

**Figures 4A - 4E** **show *L. gallinarum* produced tryptophan metabolites *in vitro* and *in vivo.*** Figure 4A is a heatmap analysis of BHI, *E. coli* and *L. gallinarum* culture supernatant *in vitro.* Figure 4B is a heatmap analysis of stool samples from MC38 syngeneic mouse model that revealed a differential abundance of metabolites in BHI-, *E. coli-* and *L. gallinarum-*treated mice. Figure 4C shows Indole-3-carboxaldehyde (lAld) was enriched in stool samples of *L. gallinarum-*treated mice in MC38 syngeneic mouse model. No significant difference of indole-3-carboxaldehyde (lAld) was observed in serum samples between groups as shown in Figure 4D. Figure 4E shows Indole-3-carboxylic acid (ICA) was enriched in serum of *L. gallinarum-treated* mice in MC38 syngeneic mouse model. *P<0.05, **P<0.01, ****P<0.0001.

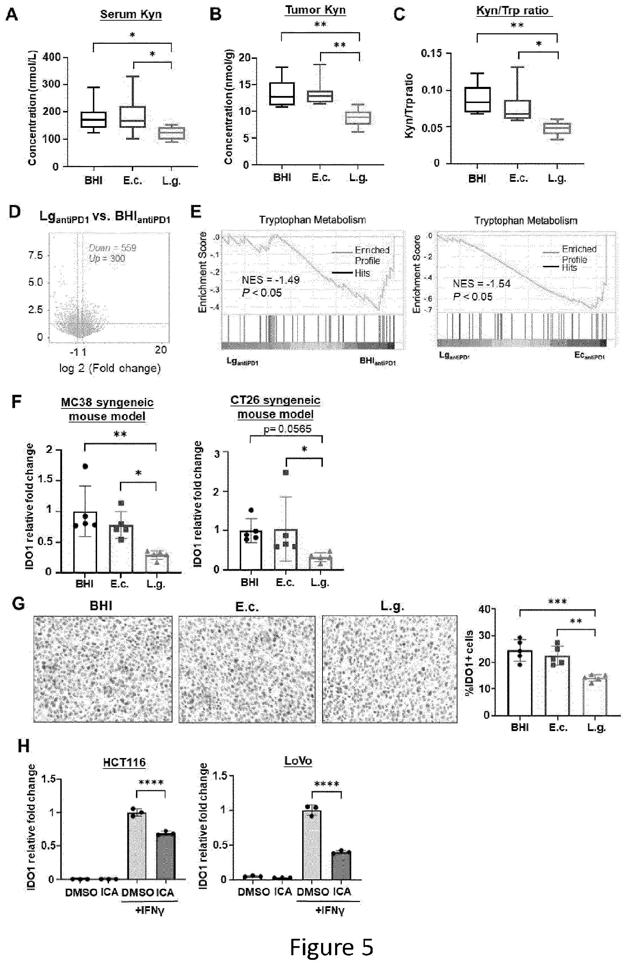

**Figures 5A - 5H** **illustrate *L. gallinarum* and ICA inhibited IDO1 expression and Kyn production in tumour.** *L. gallinarum* reduced serum kynurenine level (Figure 5A), tumour kynurenine level (Figure 5B), kynurenine-to-tryptophan ratio in tumours in MC38 syngeneic mouse model (Figure 5C). RNA sequencing revealed a differential gene expression between BHI plus anti-PD1 group versus *L. gallinarum* plus anti-PD1 group as illustrated in Figure 5D. Figure 5E shows the enrichment plot of tryptophan metabolism pathway. Figure 5F illustrates IDO1 mRNA expression of tumour tissues from MC38 and CT26 syngeneic mouse model. Figure 5G shows immunohistochemical staining of IDO1 in tumour tissues of CT26 syngeneic mouse model. Figure 5H shows the effect of ICA (5µM) on IDO1 mRNA expression in HCT116 and LoVo cell lines. *P<0.05, **P<0.01, ***P<0.001, ****P<0.0001.

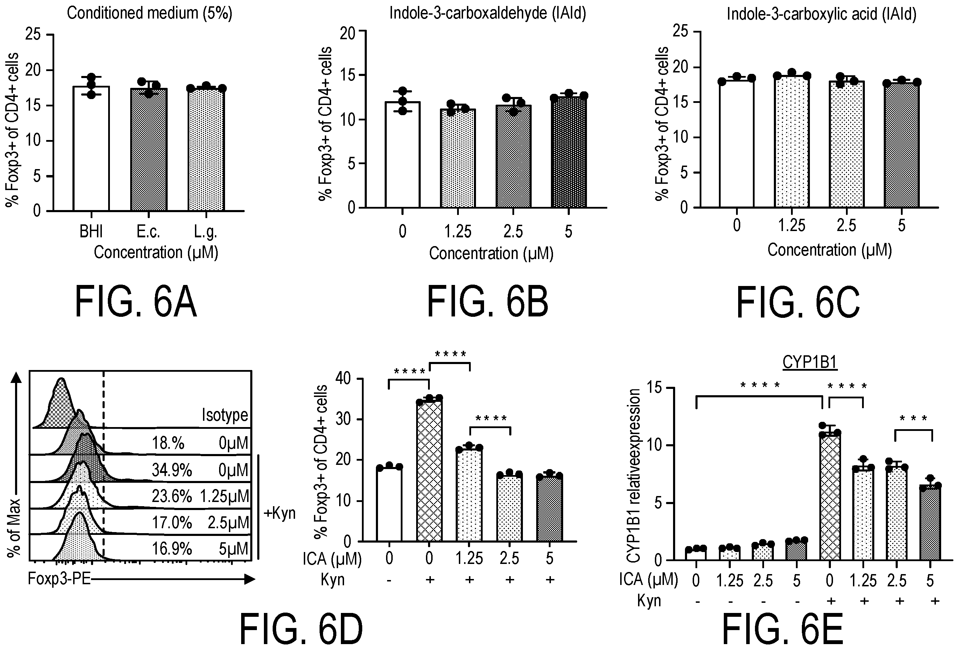

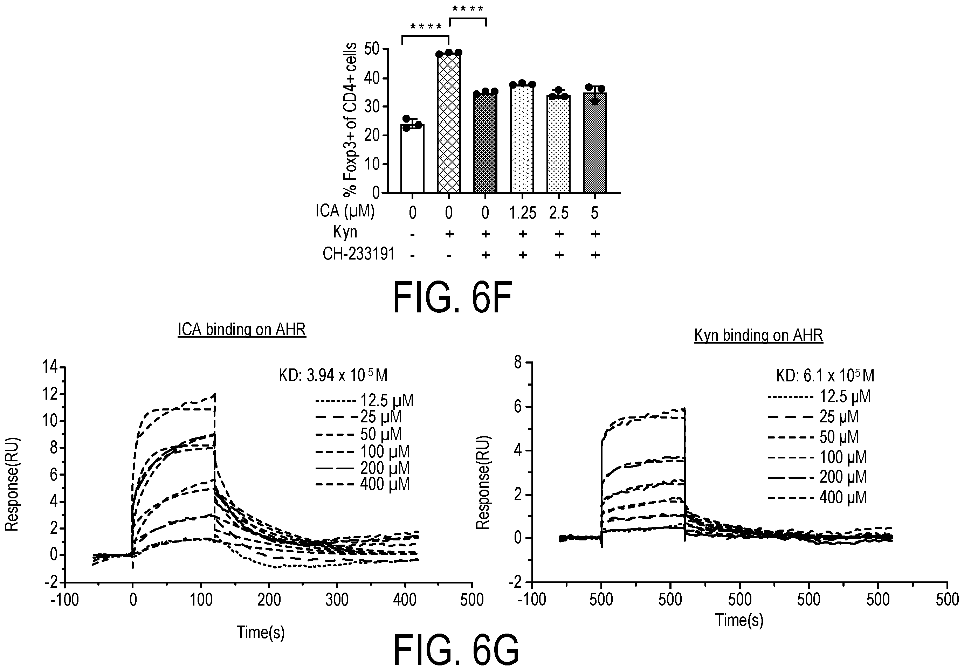

**Figures 6A - 6G** **show ICA outcompeted Kyn and inhibited Kyn-mediated AHR activation on CD4+ T cells.** Effect of *L. gallinarum* conditioned medium (Figure 6A), indole-3-carboxaldhye (lAld) (Figure 6B), and indole-3-carboxylic acid (ICA) on Foxp3+ Treg differentiation (Figure 6C). Figure 6D illustrates the effect of ICA and Kyn (50µM) on Foxp3+ Treg differentiation. ICA antagonized Kyn-mediated Treg differentiation in a dose-dependent manner. Figure 6E shows CYP1B1 expression of CD4+ T cells treated with different dose of ICA and Kyn (50µM). Figure 6F shows CH-233191 (AHR antagonist) (10µM) abolished the effect of ICA and Kyn. Figure 6G is a surface plasmon resonance (SPR) assay of ICA and Kyn on human AHR protein. ***P<0.001, ****P<0.0001.

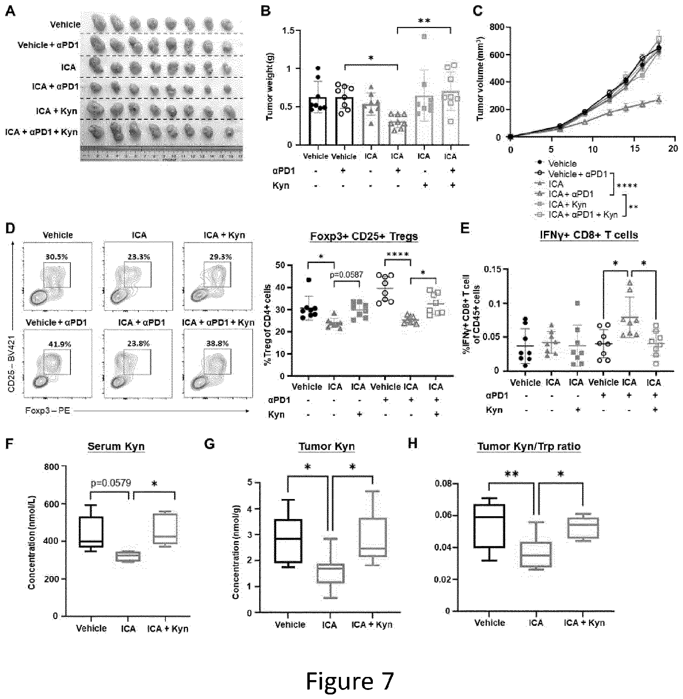

**Figures 7A- 7H** **show ICA improved anti-PD1 efficacy and was reversed by Kyn supplementation.** ICA in combination with anti-PD1 impeded tumour growth, as evidenced by representative tumour pictures as illustrated in Figure 7A, tumour weight as illustrated in Figure 7B, and tumour volume as illustrated in Figure 7C, in CT26 syngeneic mouse model. Figure 7D shows the percentage of Foxp3+ CD25+ Treg in tumour tissues. Figure 7E shows the percentage of IFNγ+ CD8+ T cells of tumour tissues. Figure 7F shows Kynurenine level in serum, Figure 7G shows Kynurenine level in tumour tissues, and Figure 7H shows Kynurenine-to-tryptophan ratio in tumour tissues. *P<0.05, **P<0.01, ***P<0.001.

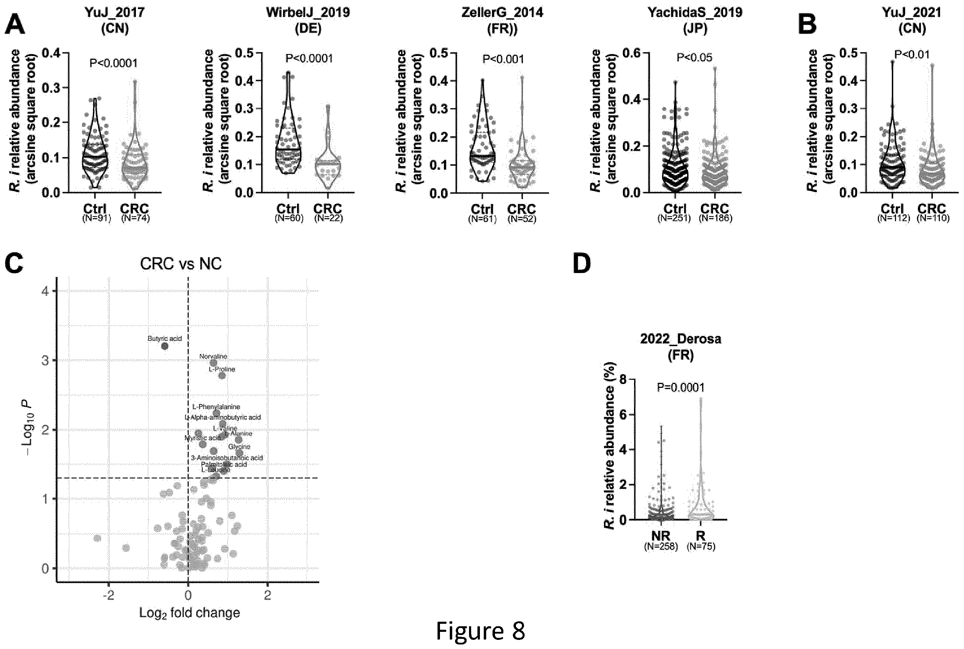

**Figures 8A - 8D** **show *R. intestinalis* and butyric acid are depleted in faecal samples of CRC patients.** Arcsine square root transferred relative abundance of *R*. *intestinalis* in published metagenomic cohorts (Figure 8A) and in-house metagenomic cohort (Figure 8B) of colorectal cancer patients and healthy controls. CN, China; DE, Germany; FR, France; JP, Japan. Figure 8C is volcano plots of significantly altered human faecal metabolites between CRC and NC. Enriched metabolites are labelled as red dots and depleted ones are labelled as blue. -Logic P (0.05) was indicated with the horizontal dash line. CRC, colorectal cancer. NC, normal control. Figure 8D is a graph showing the relative abundance of *R. intestinalis* in a France cohort of responders and non-responders to immune checkpoint inhibitors.

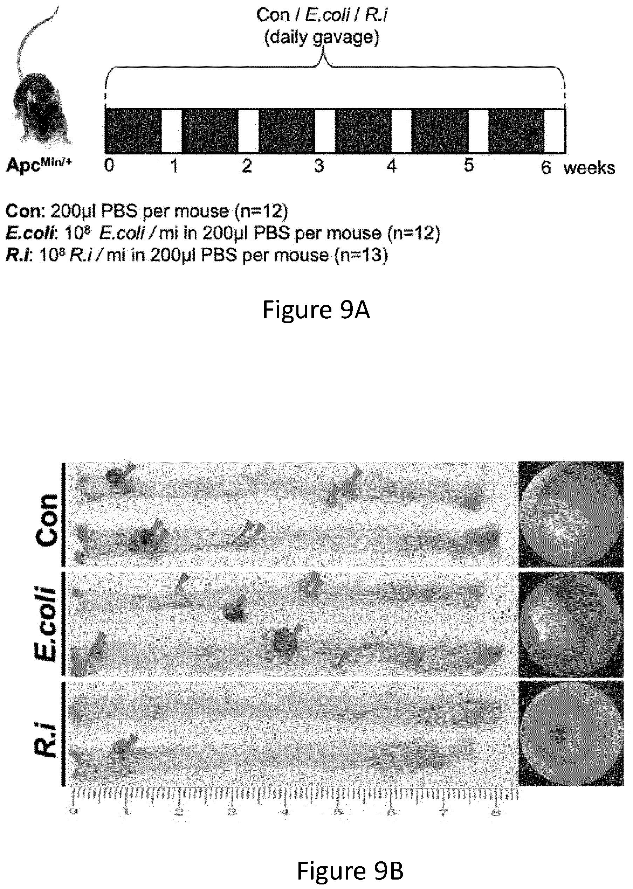

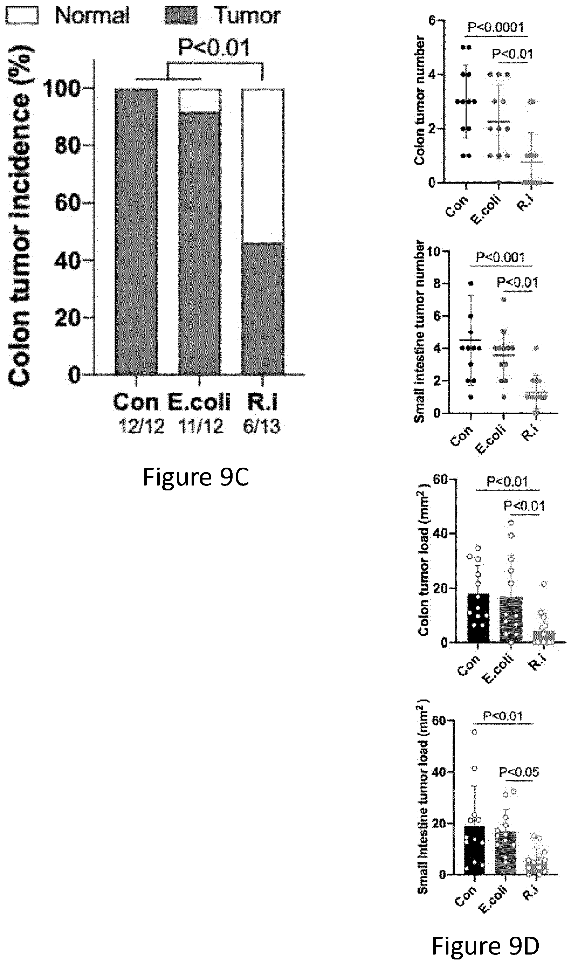

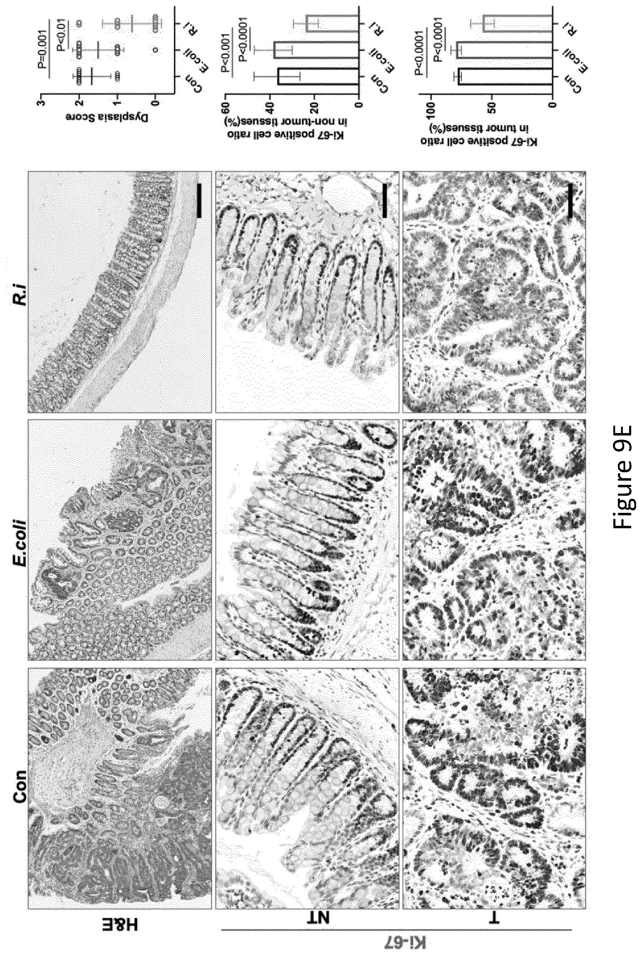

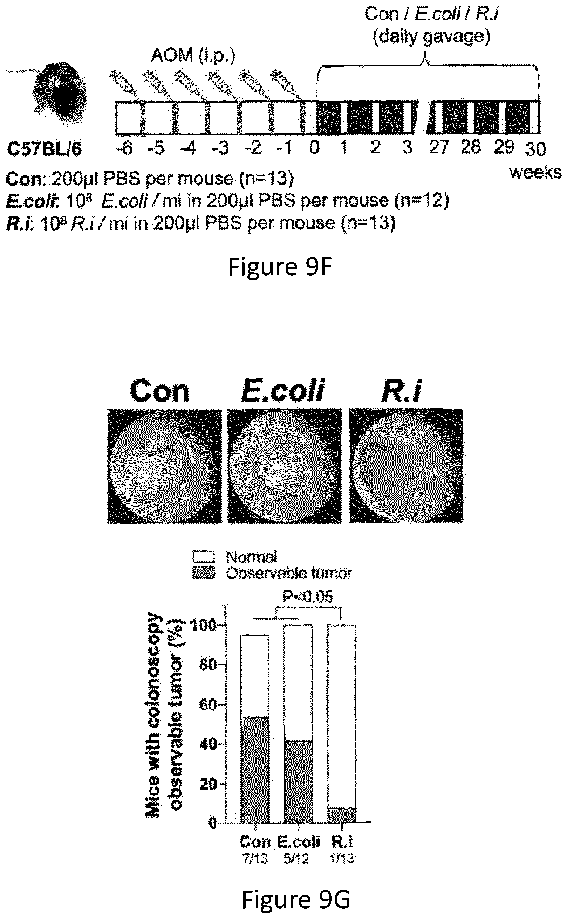

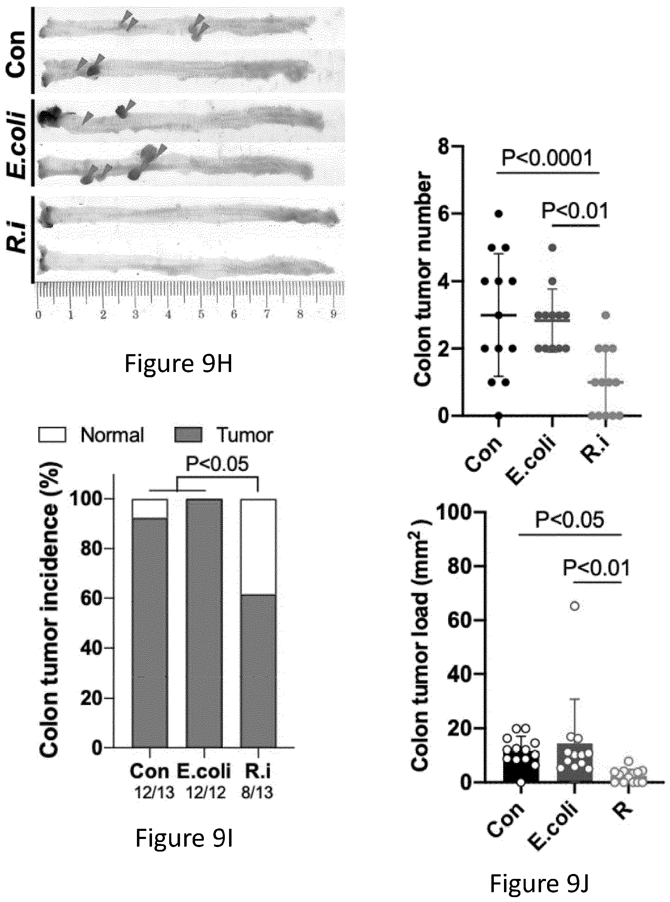

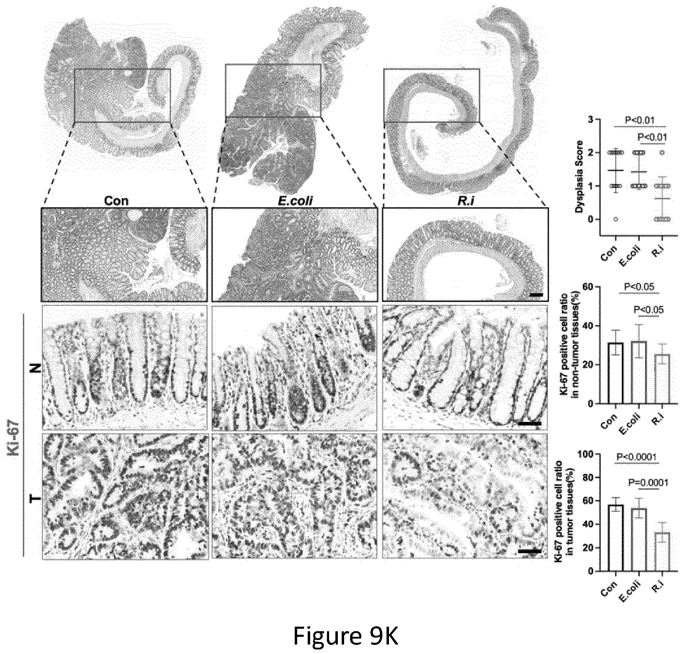

**Figures 9A - 9K** **show *R. intestinalis* reduces colorectal carcinogenesis in *Apc*^{*Min*/*+*} and AOM-induced mice.** Figure 9A is schematic diagram showing the experimental design and timeline of *Apc*^{*Min*/*+*} mice. Figure 9B shows representative colonic morphologies (left panel) and colonoscopy video captures (right panel) of *Apc*^{*Min*/*+*} mice. Colon tumour incidence (Figure 9C), colon and small intestine tumour number and tumour load (total tumour area, mm²) (Figure 9D) of *Apc*^{*Min*/*+*} mice. Figure 9E are representative H & E staining images (scale bar=200µm) and Ki-67 IHC staining (scale bar=50µm) of *Apc*^{*Min*/*+*} mice. Figure 9F is a schematic diagram showing the experimental design and timeline of AOM-induced mice. Figure 9G are representative colonoscopy video captures (upper panel) and incidence of AOM-induced mice with colonoscopy observable tumour (down panel). Figure 9H shows representative colonic morphologies of AOM-induced mice. Figure 9I shows colon tumour incidence, colon tumour number and colon tumour load (total tumour area, mm²) (Figure 9J) of AOM-induced mice. Figure 9K shows representative H & E staining images (scale bar=200µm) and Ki-67 IHC staining (scale bar=50µm) of AOM-induced mice.

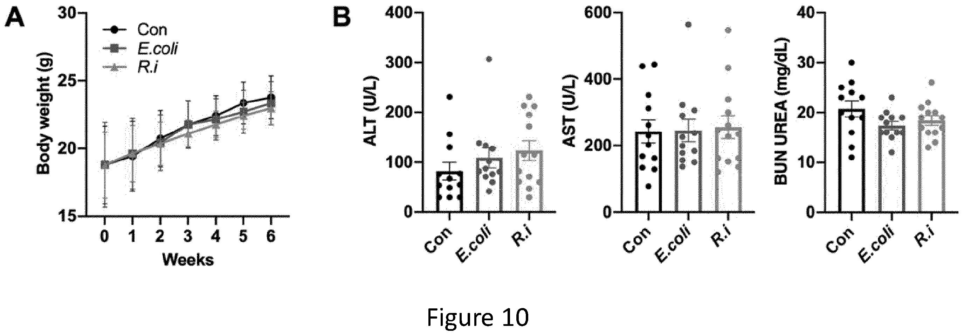

**Figures 10A - 10B** **illustrate safety assessment of *R. intestinalis* on *APC*^{*Min*/*+*} mice.** The dosage and frequency of *R. intestinalis* treatment on mouse body weight **(****Figure 10A****),** live (ALT, AST) and renal function (BUN) **(****Figure 10B****).** ALT, Alanine transaminase; AST, aspartate aminotransferase; BUN, blood urea nitrogen; CREA, creatinine, all the samples were under 0.3mg/dL.

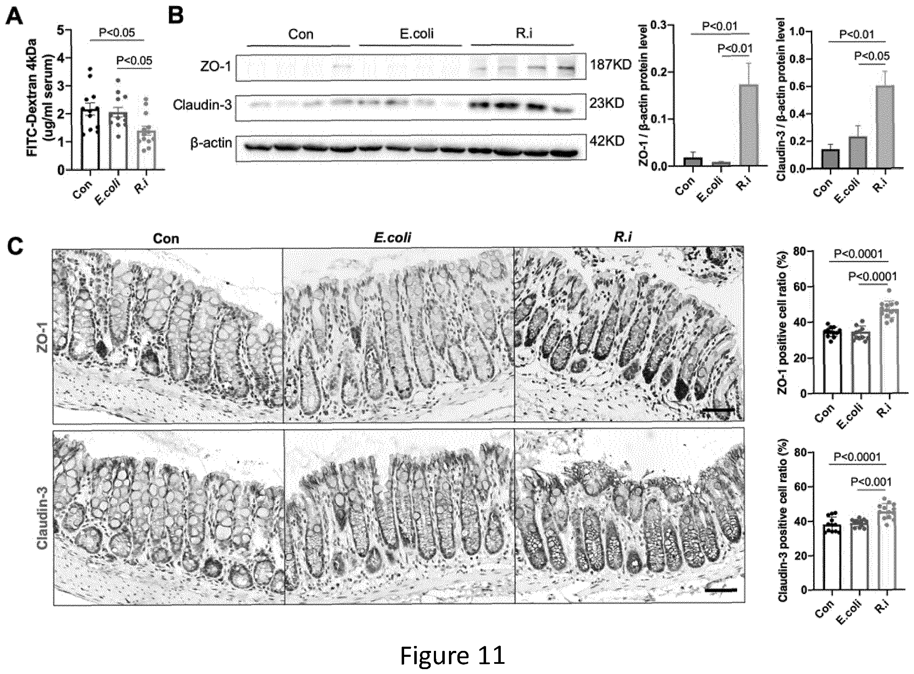

**Figures 11A - 11C** **show *R. intestinalis* enhances epithelial barrier functions in *Apc*^{*Min*/*+*} mice.** Figure 11A shows FITC-dextran (4KD) concentration (ng/ml) in *Apc*^{*Min*/*+*} mice serum. Figure 11B shows expression levels of tight junction proteins ZO-1 and Claudin-3 determined by western-blot in colon tissues of *Apc*^{*Min*/*+*} mice. Figure 11C is representative ZO-1 and Claudin-3 IHC staining images (scale bar=50µm).

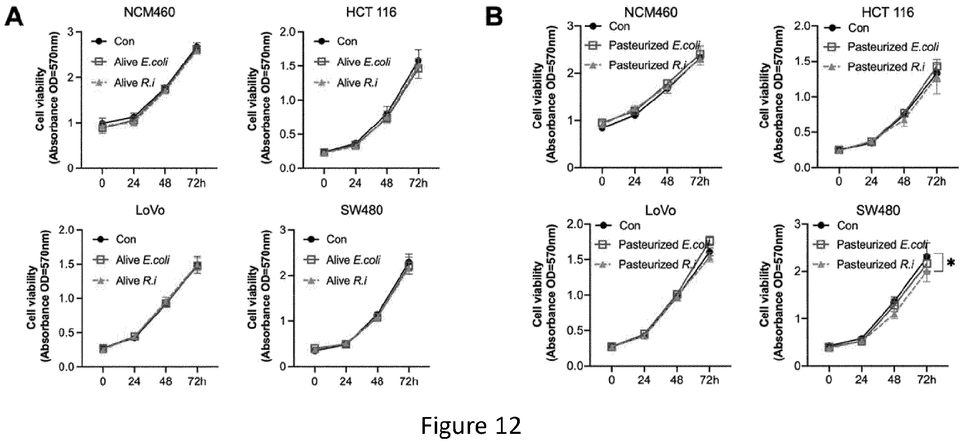

**Figures 12A and 12B** **show alive and pasteurized *R. intestinalis* have no tumour-suppressive effects *in vitro.*** Both alive *R.i* (CFU=10⁶/ml) (Figure 12A), and pasteurized (70 °C, 30min) *R.i* (Figure 12B) had no effects on colonic cell viability.

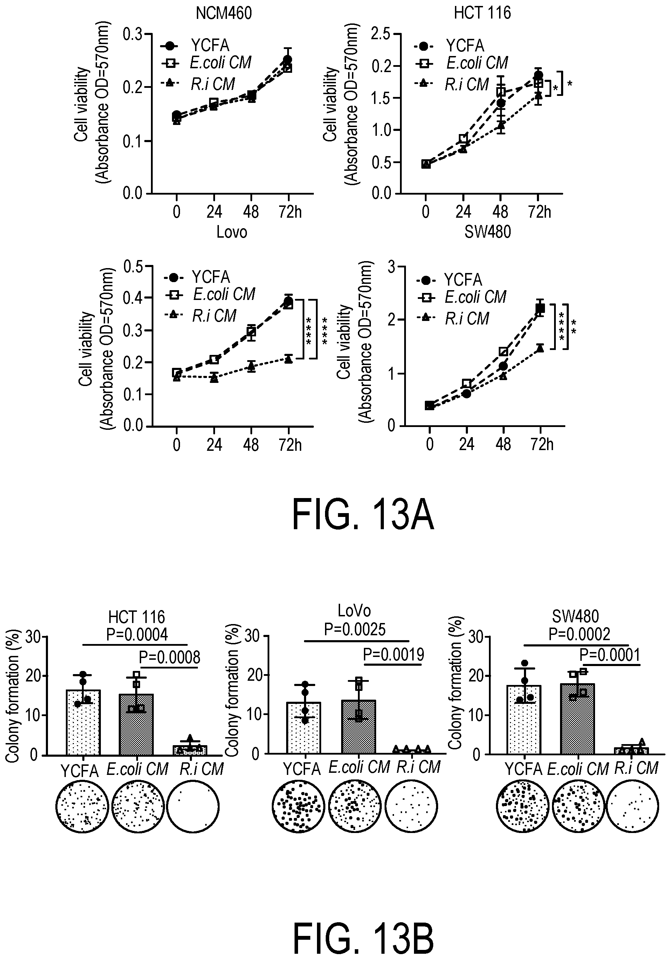

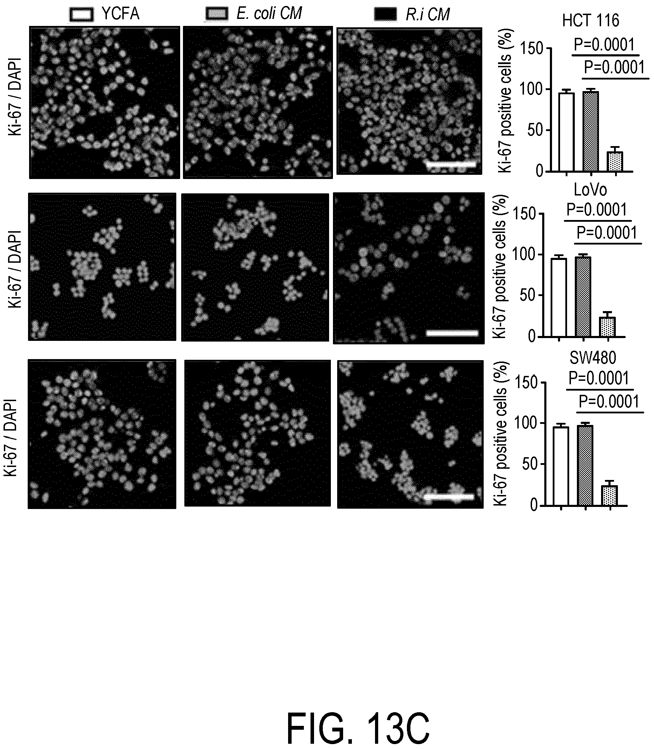

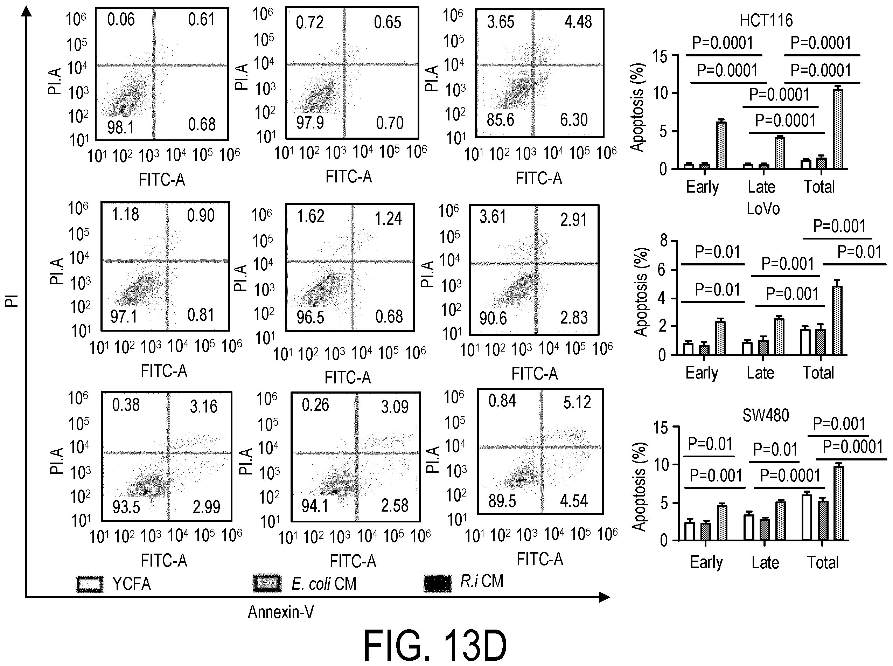

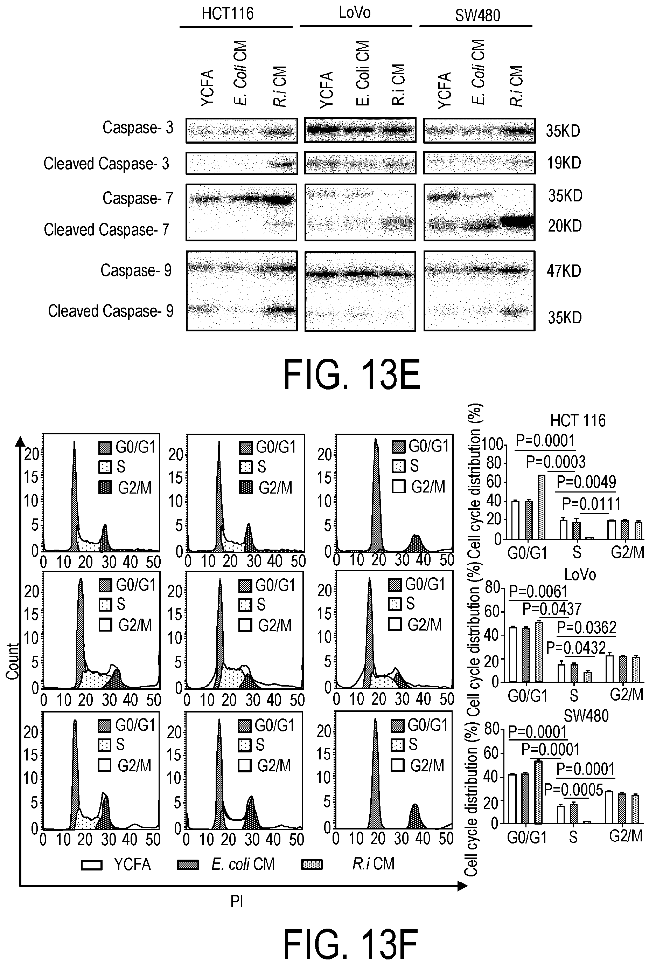

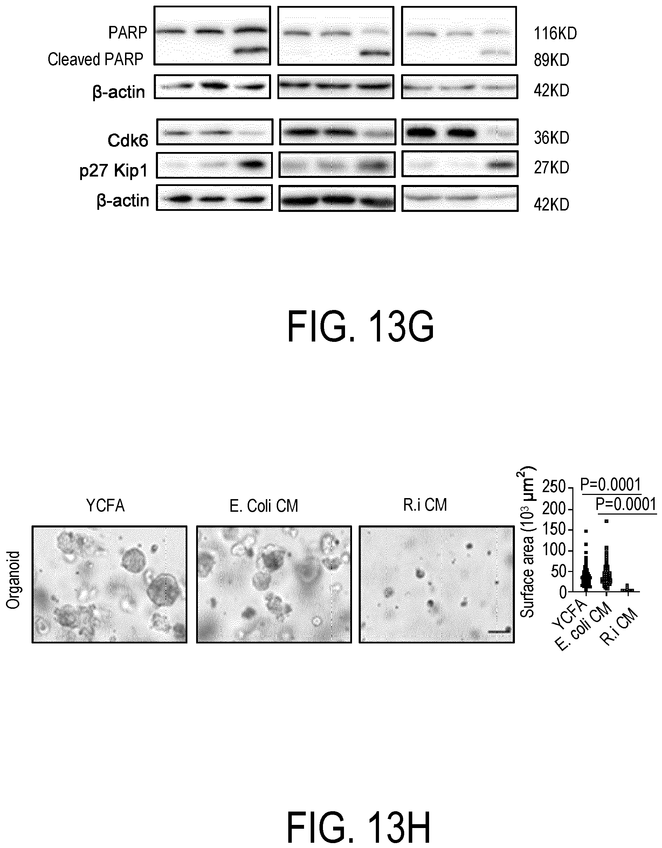

**Figures 13A - 13H** **illustrates *R.i* CM exhibits tumour-suppressive effects *in vitro.*** As shown in Figure 13A *R.i* CM (5%) reduced the colonic cell viability, except NCM460. *Escherichia coli* conditioned medium (*E.i* CM) and YCFA were used as control. As shown in Figure 13B *R.i* CM (5%) suppressed the colony formation of CRC cells. As shown in Figure 13C *R.i* CM reduced CRC cell Ki-67 expression levels determined by immunocytochemistry (ICC). As shown in Figure 13D *R.i* CM increased CRC cell apoptosis. Figure 13E shows *R.i* CM induced CRC cell apoptosis of CRC cells by cleaved caspase-3, cleaved caspase-7, cleaved caspase-9, and cleaved PARP expression. Figure 13F shows *R.i* CM inhibited CRC cell cycle G1/S transition. Figure 13G shows *R.i* CM induced G1 arrest by Cdk6 downregulation and p27 Kip1 upregulation. Figure 13H shows *R.i* CM inhibited the growth of the organoid derived from a CRC patient.

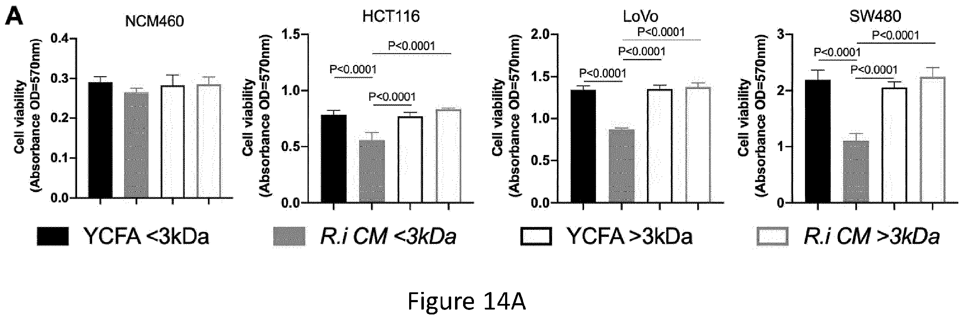

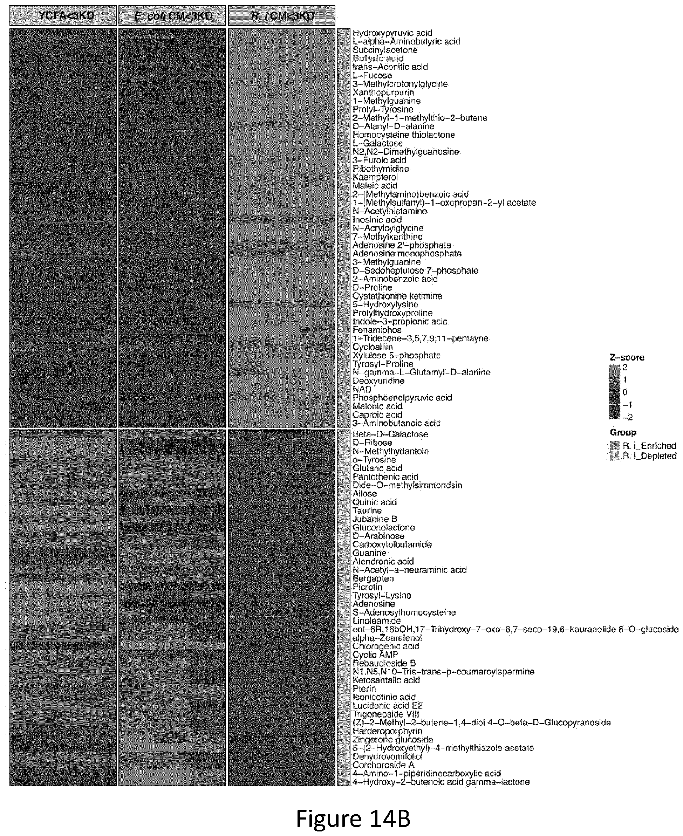

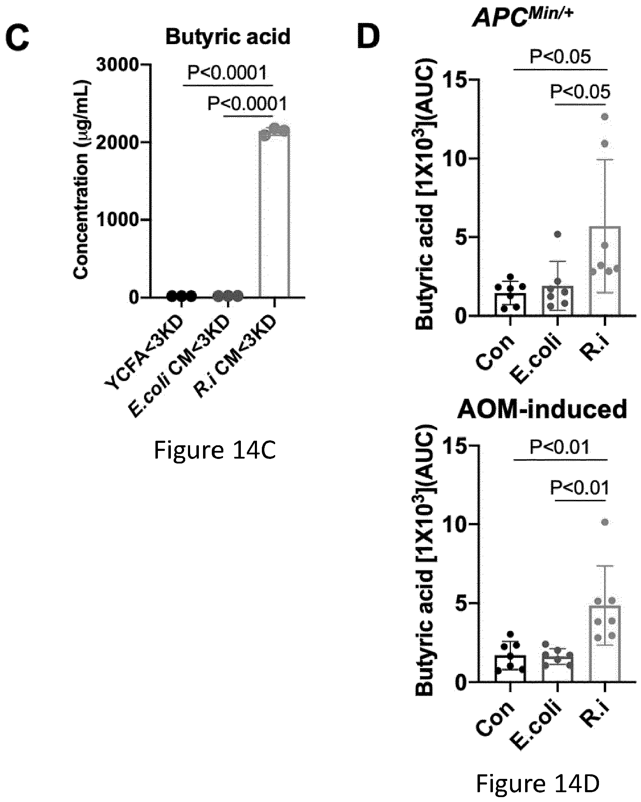

**Figures 14A - 14D** **show the tumour suppressive component produced by *R*. *intestinalis* is Butyric acid.** As shown in 14A, the efficient components in *R.i* CM are less than 3KD. As shown in Figure 14B, the *R.i* CM <3KD fraction was assessed by untargeted LC-MS/MS analysis and butyric acid was identified as the candidate tumour suppressive molecule. Figure 14C shows butyrate acid concentration (µg/ml) in *R.i* CM <3KD was determined by targeted short chain fatty acid GC-MS analysis. Figure 14D shows relative concentration of butyrate acid (area under curve, AUC) in *Apc*^{*Min*/}*⁺ and* AOM-induced mice feces was determined by GC-MS analysis.

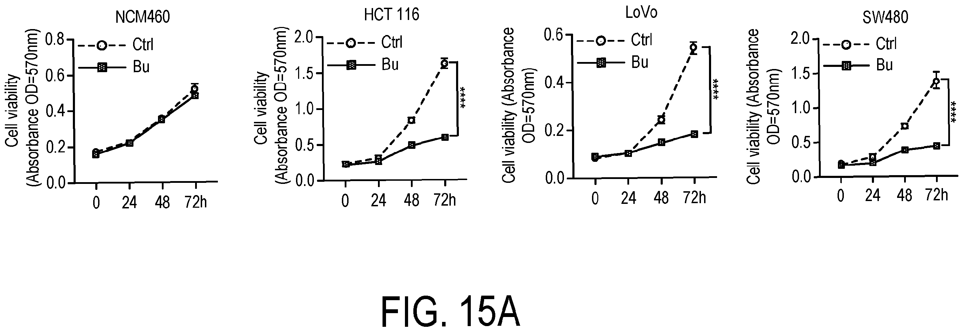

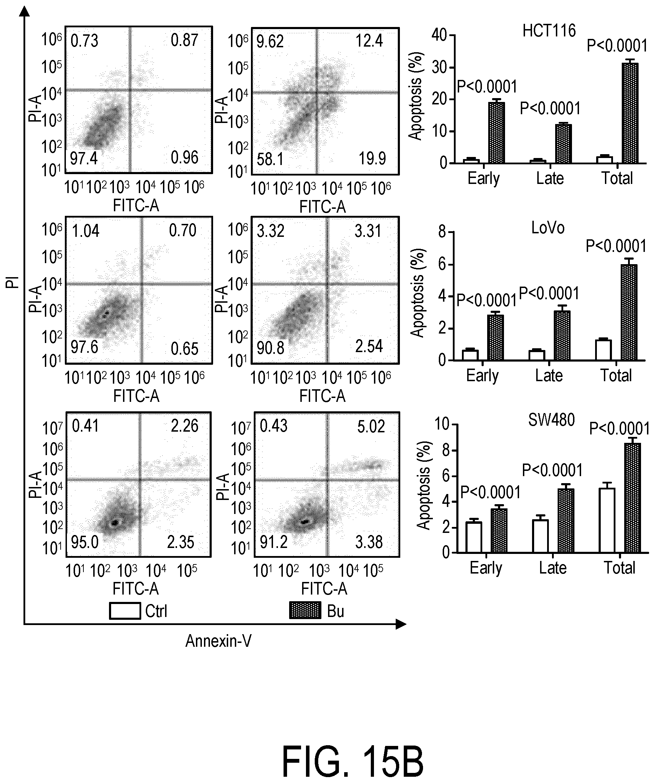

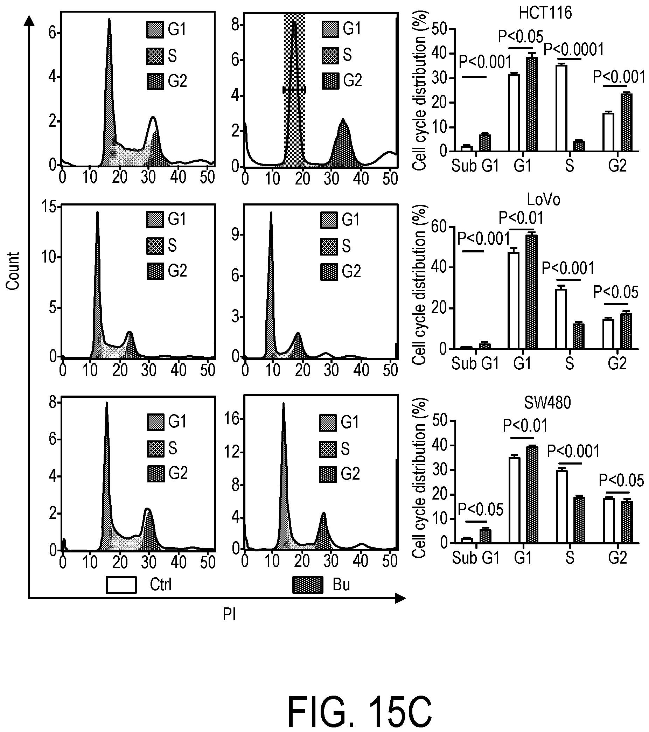

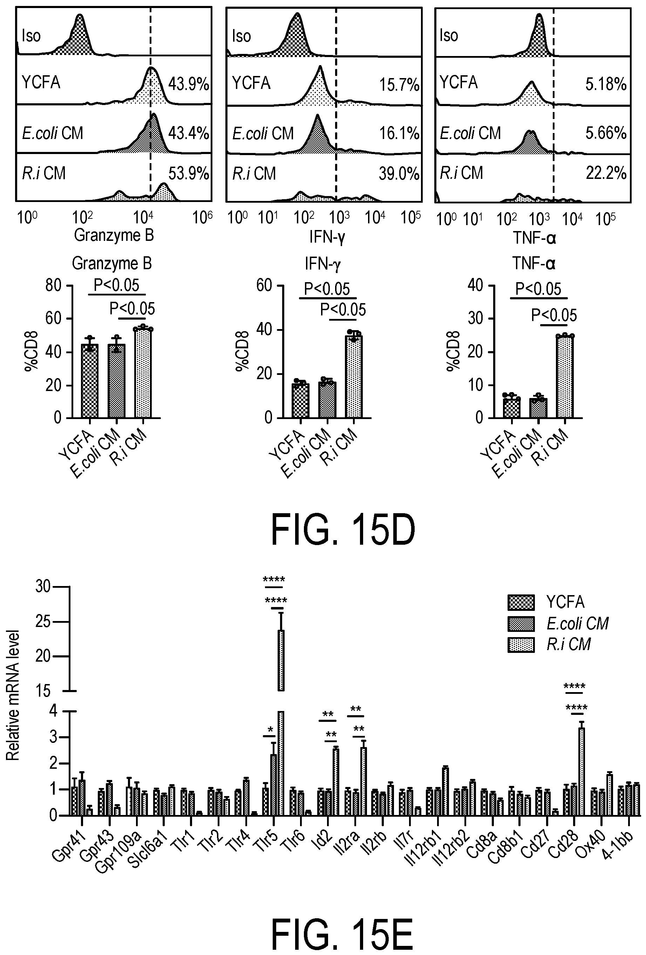

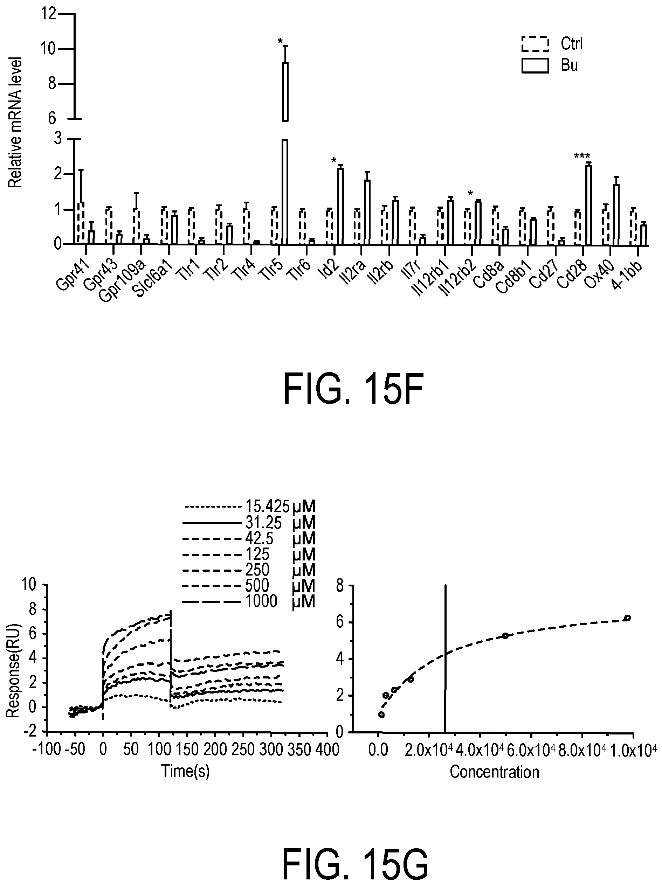

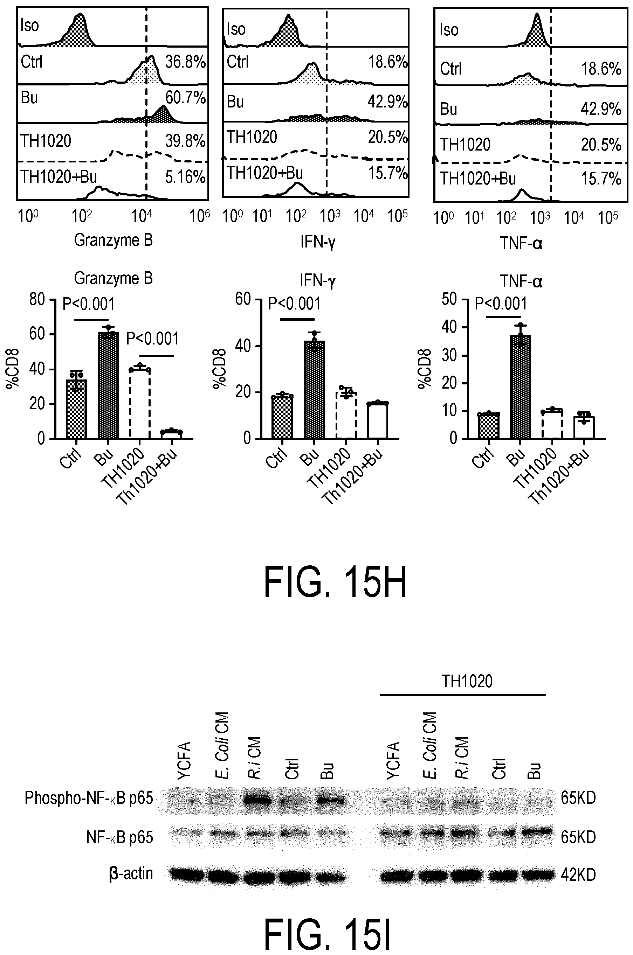

**Figures 15A - 15I** **show comparable dosage of butyrate in *R.i* CM suppresses colorectal cancer cells by directly antitumour effects and CD8⁺ T cell boosting *in vitro.*** Figure 15A shows comparable dosage (1mM) of butyrate in *R.i* CM reduced the colonic cell viability, except NCM460. Figure 15B shows butyrate induced CRC cell apoptosis. Figure 15C shows butyrate inhibited CRC cell cycle G1/S transition. Figure 15D shows *R.i* CM directly boosted Granzyme B⁺, IFN-γ⁺, TNF-α⁺ CD8⁺ T cells isolated from human PBMC. The mRNA expression level of associated receptors on CD8⁺ T cells after treatment with *R. i* CM is shown in Figure 15E, and Butyrate (Figure 15F). Figure 15G shows the binding affinity between TLR5 and butyric acid was measured by surface plasmon resonance (SPR) (left), affinity of TLR5 to butyric acid was 264 µM (right). Figure 15H shows Granzyme B⁺, IFN-γ⁺, TNF-α⁺ CD8⁺ T cells isolated from human PBMC were directly boosted by comparable dosage of butyrate and abolished by TLR5 antagonist TH1020. Figure 15I shows Phospho-NF-κB p65 protein expression level of αCD3/αCD28/IL2 activated Jurkat E6.1 T cells after *R.i* CM and butyrate treatment with or without TH1020 inhibition.

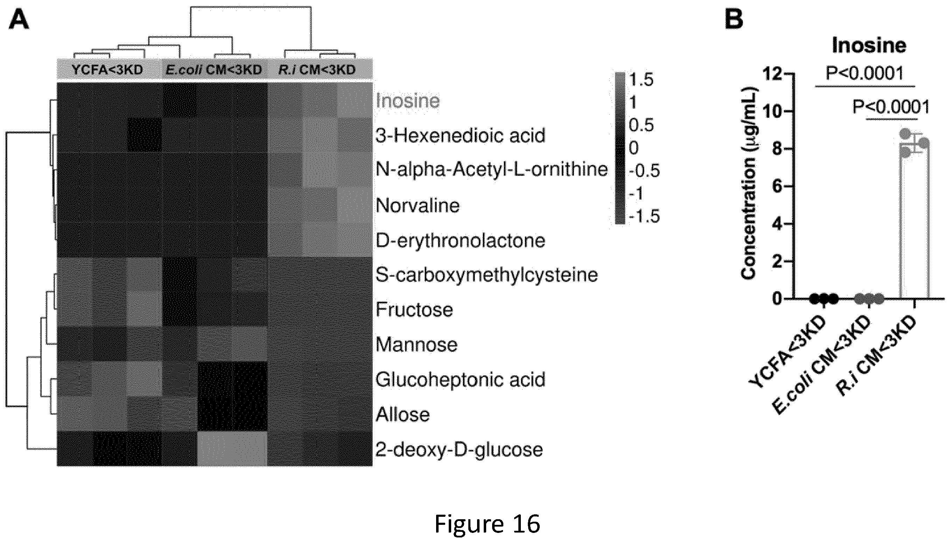

**Figures 16A and 16B** **shows Inosine might be another tumour suppressive component produced by *R.i.*** Figure 16A shows the *R.i* CM <3KD fraction was assessed by untargeted GC-MS/MS analysis and inosine was identified as the candidate tumour suppressive molecule. As shown in Figure 16B, inosine concentration (µg/ml) in *R.i* CM <3KD was determined by targeted LC-MS analysis.

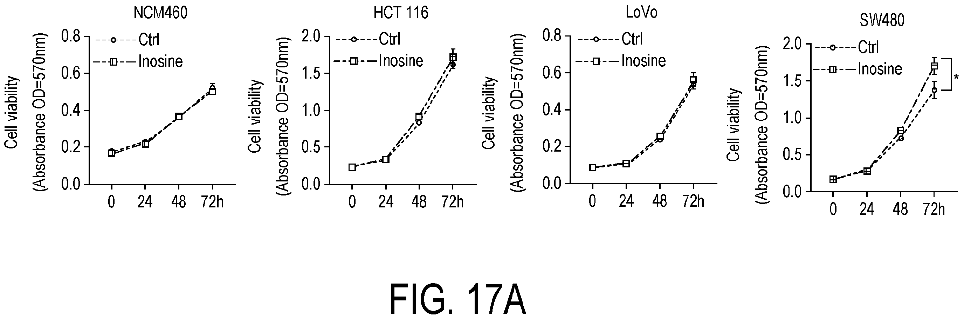

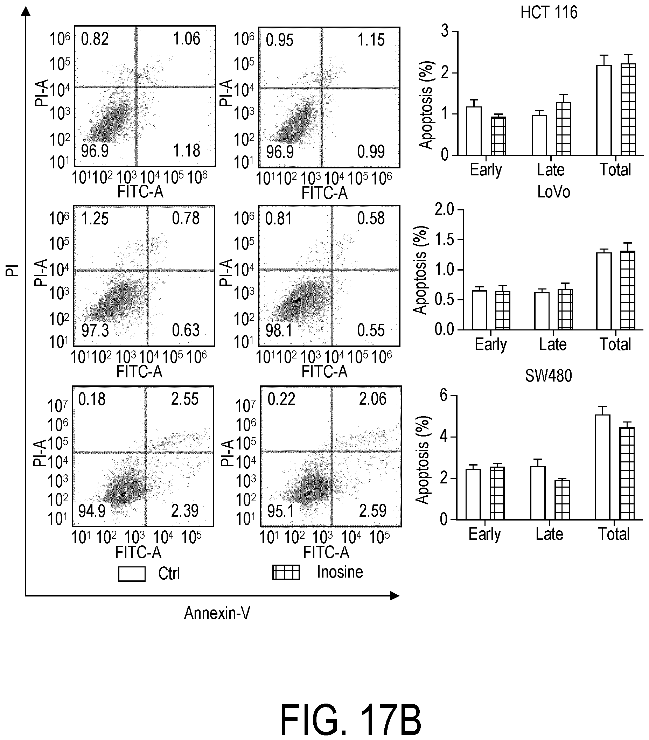

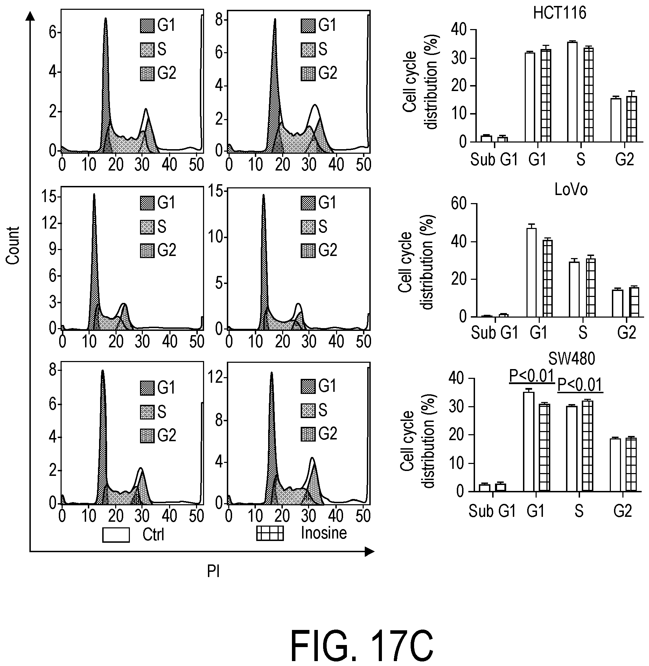

**Figures 17A - 17D** **show the comparable dosage of inosine in *R.i* CM has no anti-tumour effects *in vitro.*** Comparable dosage of inosine in *R.i* CM (0.4µg/ml) had no suppressive effects on colonic cell viability (Figure 17A), cell apoptosis (Figure 17B) and cell cycle (Figure 17C). Comparable dosage of inosine had no effects on Granzyme B⁺, IFN-γ⁺, TNF-α⁺ CD8⁺ T cells isolated from C57BL/6 mouse spleen.

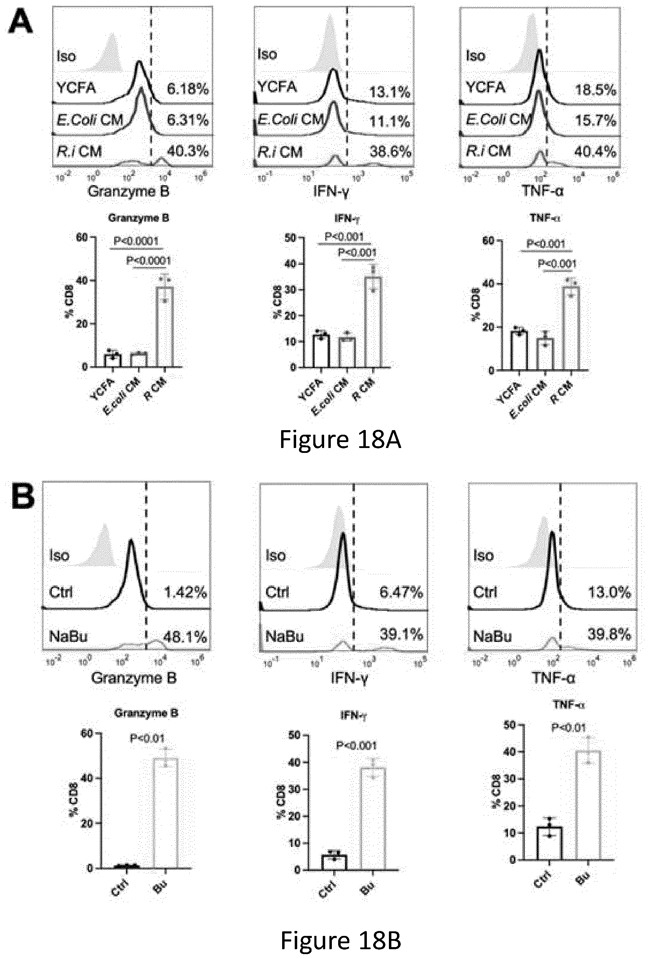

**Figures 18A and 18B** **show comparable dosage of butyrate in *R.i* CM suppresses colorectal cancer cells by CD8⁺ T cell boosting** ***in vitro.** R.i* CM (Figure 18A) and comparable dosage (1mM) of butyrate (Figure 18B) directly boosted Granzyme B⁺, IFN-γ⁺, TNF-α⁺ CD8⁺ T cells isolated from C5TBL/6 mouse spleen.

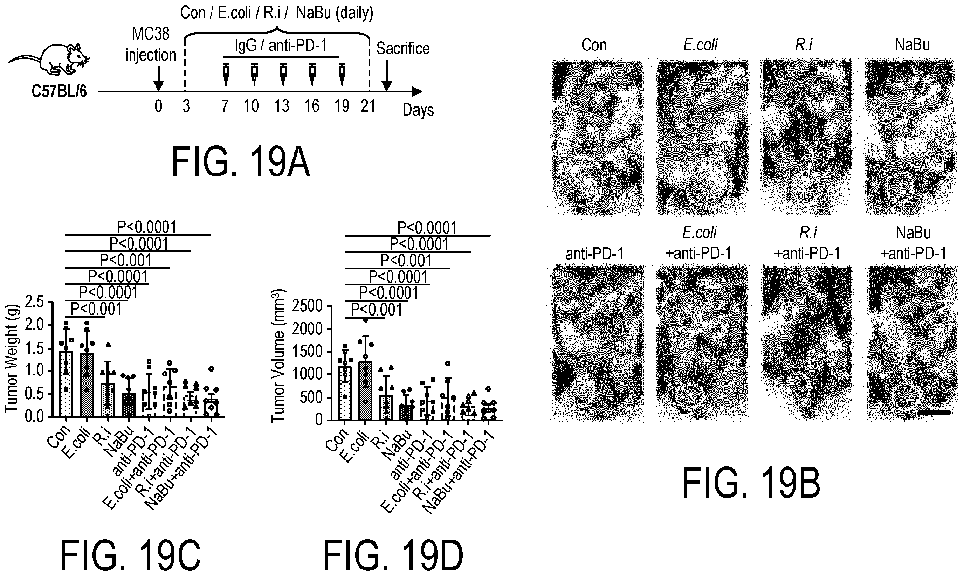

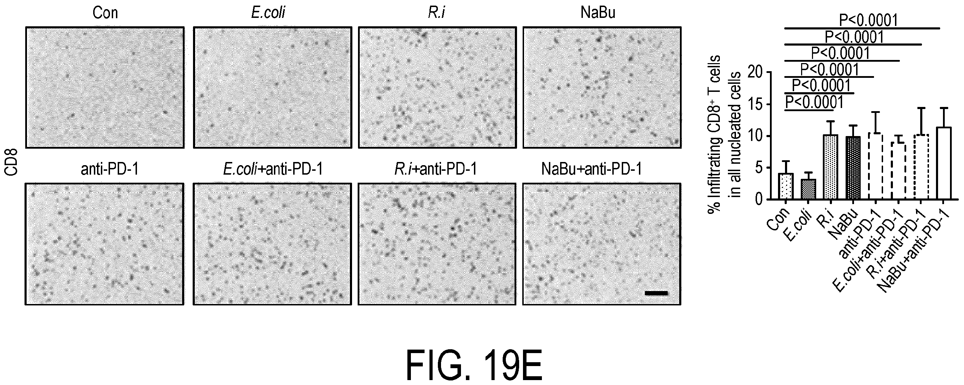

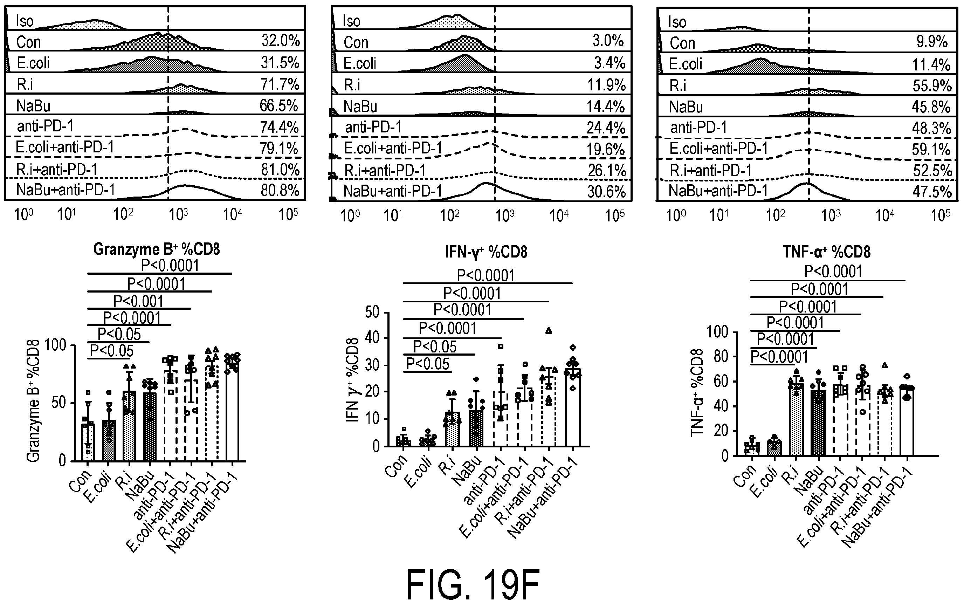

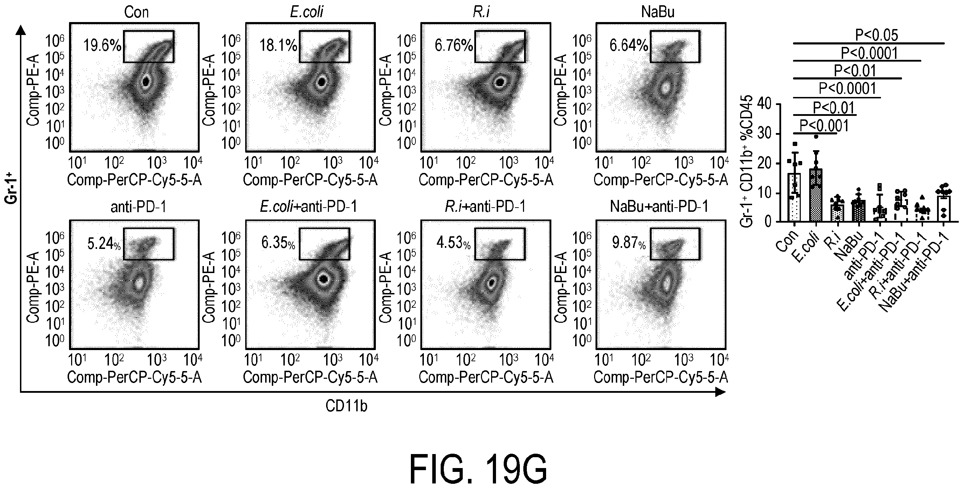

**Figures 19A - 19G** **show *R. intestinalis* and butyrate restrict MC38 orthotopic tumour growth by directly boosting CD8⁺ T cells.** Figure 19A is a schematic diagram showing the experimental design and timeline of MC38 orthotopic model. Figure 19B shows representative MC38 rectum orthotopic tumour morphologies. Figure 19C shows tumor volume (mm³) and Figure 19D shows tumour weight. Figure 19E shows the proportion of tumour infiltrating CD8⁺ T cells in all nucleated cells of MC-38 orthotopic tumour tissues determined by IHC. Figure 19F shows frequencies of granzyme B, IFN-γ, TNF-α within CD8+ populations of tumours from control, *E. coli* and *R.i* treated C57BL/6J mice. Figure 19G shows the frequencies of MDSC from tumour tissues in control, *E. coli* and *R.i* treated C57BL/6J mice measured by flow cytometry.

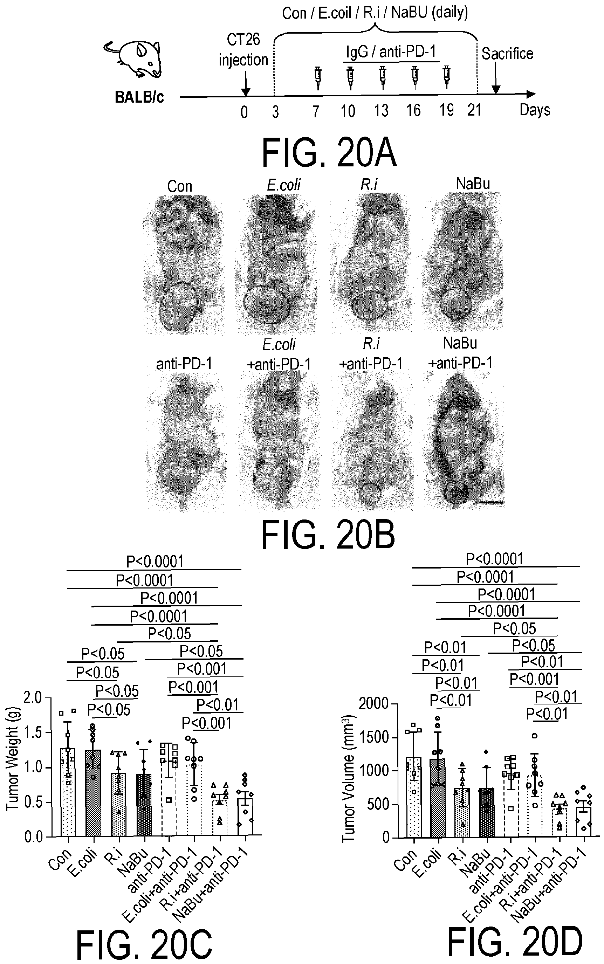

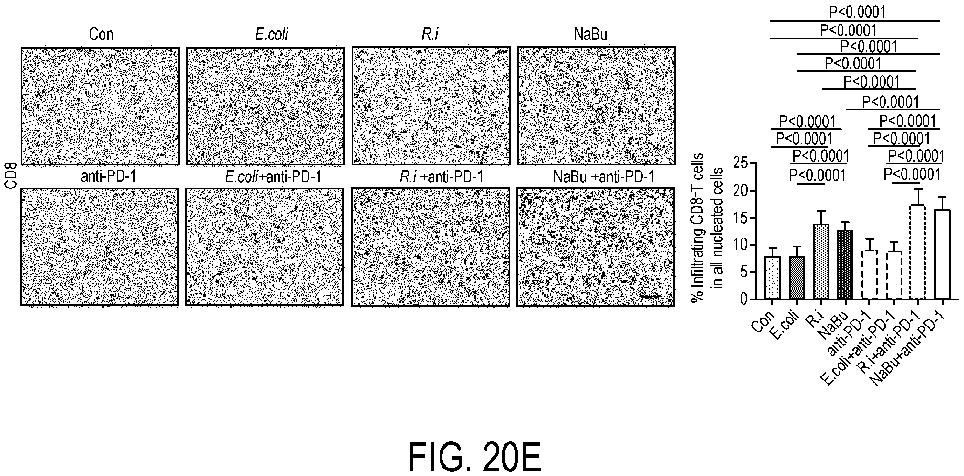

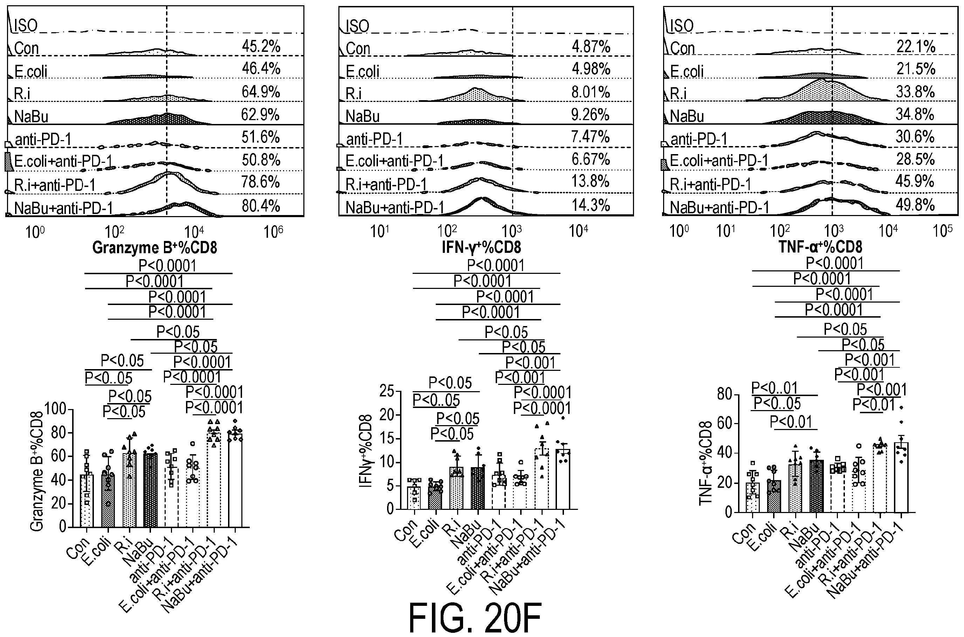

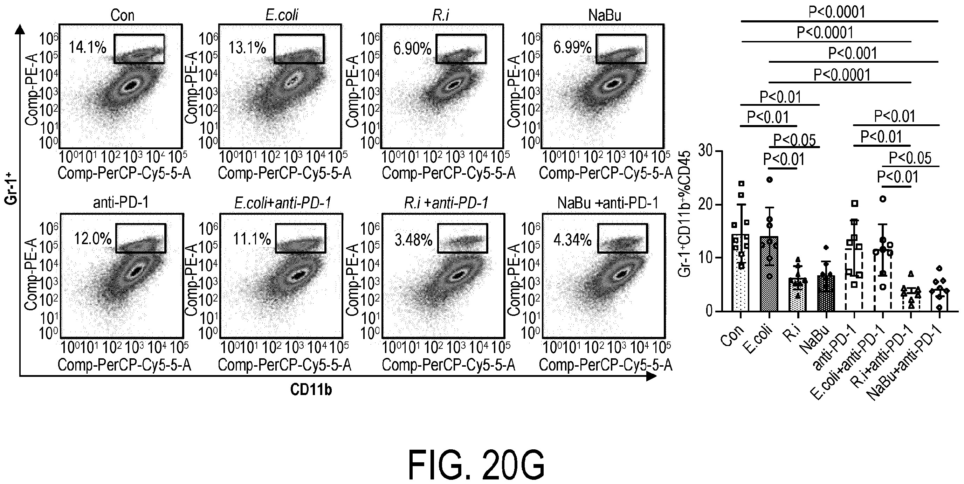

**Figures 20A - 20G** **show *R. intestinalis* and butyrate treatments are effective in CT-26 orthotopic tumour via CD8⁺ T cells independently from anti-PD-1 therapy.** Figure 20A is a schematic diagram showing the experimental design and timeline of CT26 orthotopic model. Figure 20B shows representative CT26 rectum orthotopic tumour morphologies. Figure 20C shows tumour volume (mm³) and Figure 20D shows tumour weight. Figure 20E shows a proportion of tumour infiltrating CD8⁺ T cells of CT-26 orthotopic tumour in all nucleated cells determined by IHC. Figure 20F shows frequencies of granzyme B, IFN-γ, TNF-α within CD8+ populations of tumours from control, *E. coli* and *R.i* treated BALB/c mice. Figure 20G shows frequencies of MDSC from tumour tissues in control, *E. coli* and R.i treated BALB/c mice measured by flow cytometry.

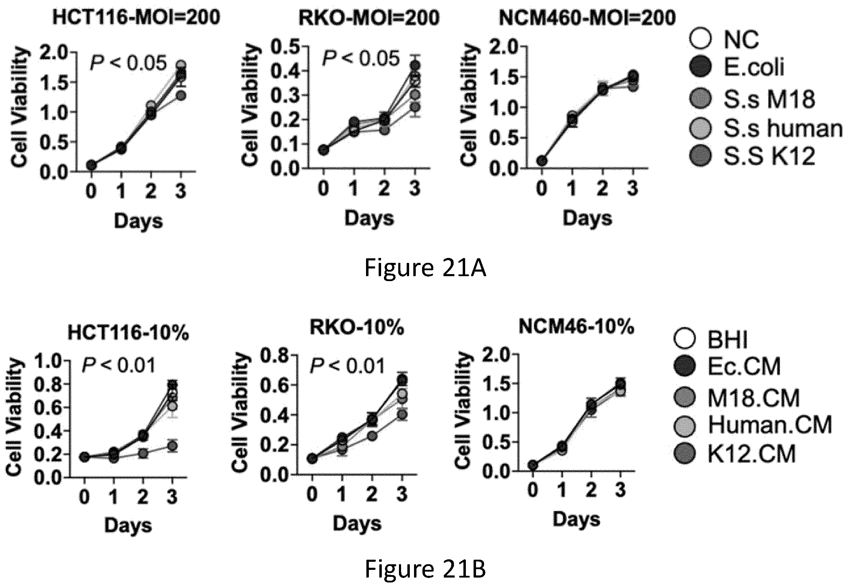

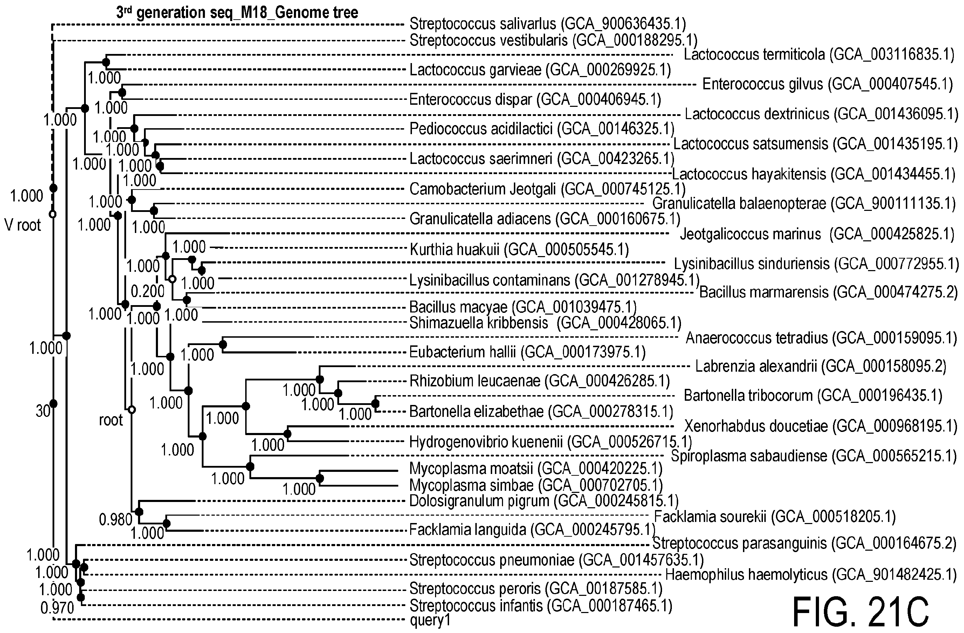

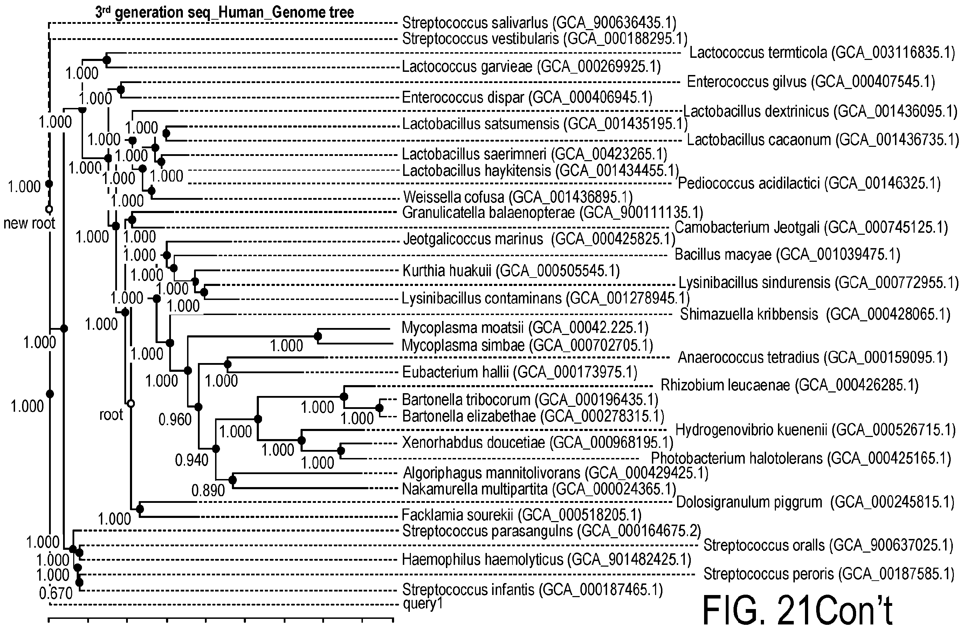

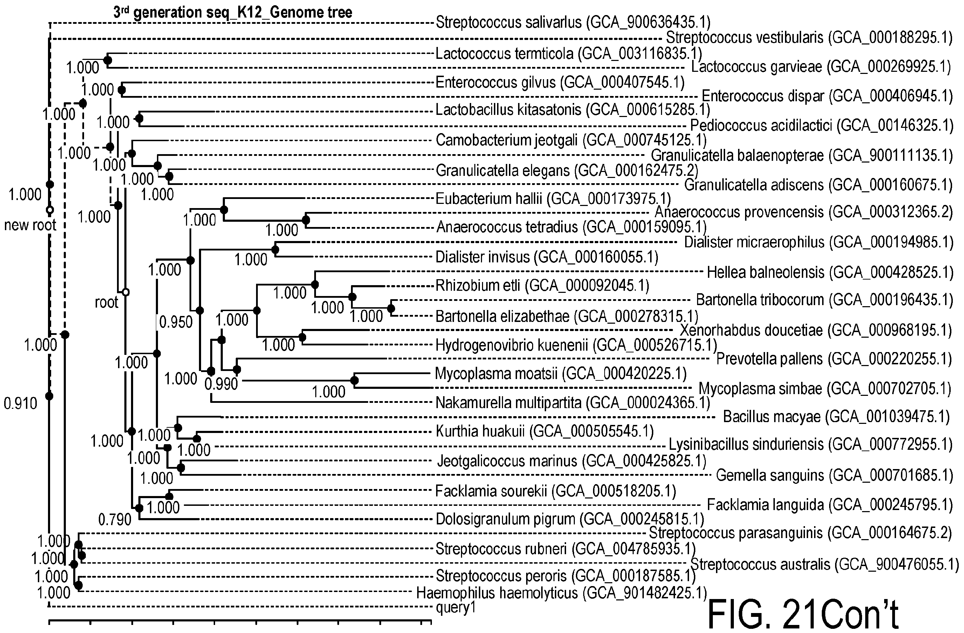

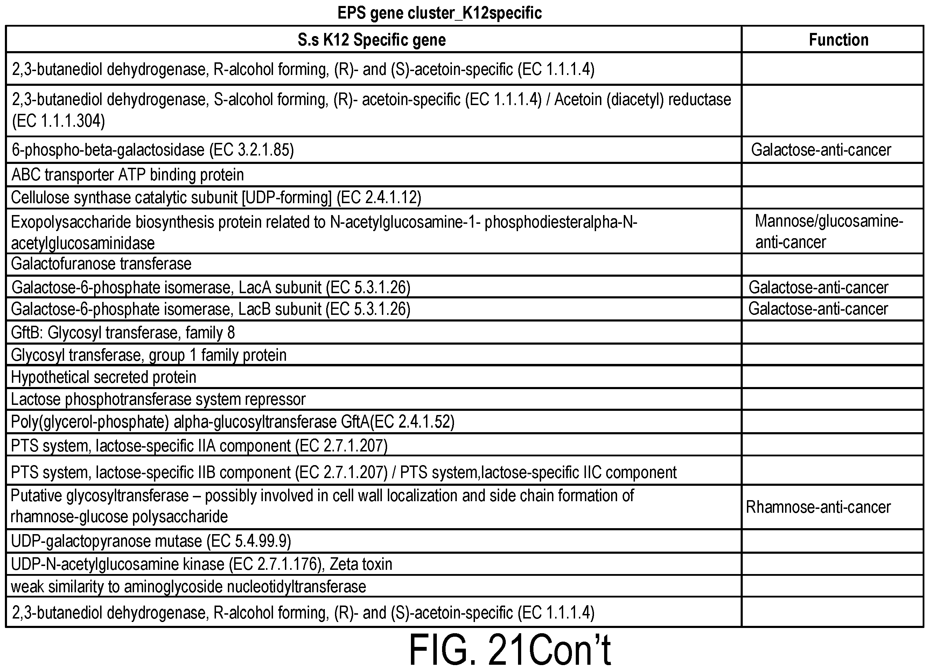

**Figures 21A - 21C** **show only *S. salivarius* K12 inhibits the viability of colon cancer cells.** Figure 21A shows the viability of CRC cells (HCT116, Lovo) and normal colon cells (NCM460) after co-incubation with or without 3 different strains *of S. salivarius* (K12, M18, Human). *E. coli* was used as a bacteria control. Figure 21B shows the viability of CRC cells (HCT116, Lovo) and normal colon cells (NCM460) after co-incubation with or without the conditioned medium of 3 different strains of *S. salivarius* (K12, M18, Human). The conditioned medium of *E. coli* (Ec.CM) was used as a bacteria control. BHI was used as broth control. Figure 21C shows the genome tree form of 3 different strains of *S*. *salivarius* (K12, M18, Human), and the specific EPS gene cluster from *S. salivarius* K12. Results are presented as mean ± S.D. Statistical significance was determined by one-way ANOVA or two-way ANOVA where appropriate. S.s, *Streptococcus salivarius*; CM, conditioned medium; MOI, multiplicity of infection; EPS, exopolysaccharides.

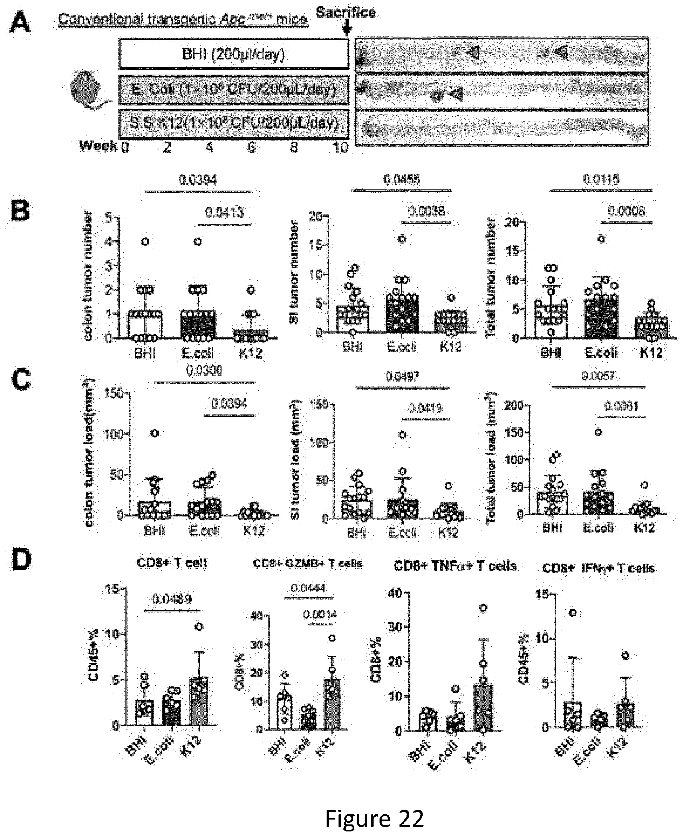

**Figures 22A - 22D** **show *S. salivarius* K12 protects against intestinal tumourigenesis and modulates anti-tumour immunity in *Apc*^{min/*+*} mice.** Figure 22A is a schematic diagram showing the experimental design, timeline, and representative colonic morphologies of *Apc*^{min/*+*} mice under different treatments. Figure 22B shows colonic, small intestine, and total (Colon + small intestine) tumour number of *Apc*^{*min*/*+*} mice under different treatments. Figure 22C shows colonic, small intestine, and total (Colon + small intestine) tumour load of *Apc*^{*min*/*+*} mice under different treatments. Figure 22D shows the proportion of tumour infiltrating CD8+ T cells in *Apc*^{*min*/*+*} mice under different treatments. Results are presented as mean ± S.D. Statistical significance was determined by one-way ANOVA. S.s, *Streptococcus salivarius*; SI, small intestine.

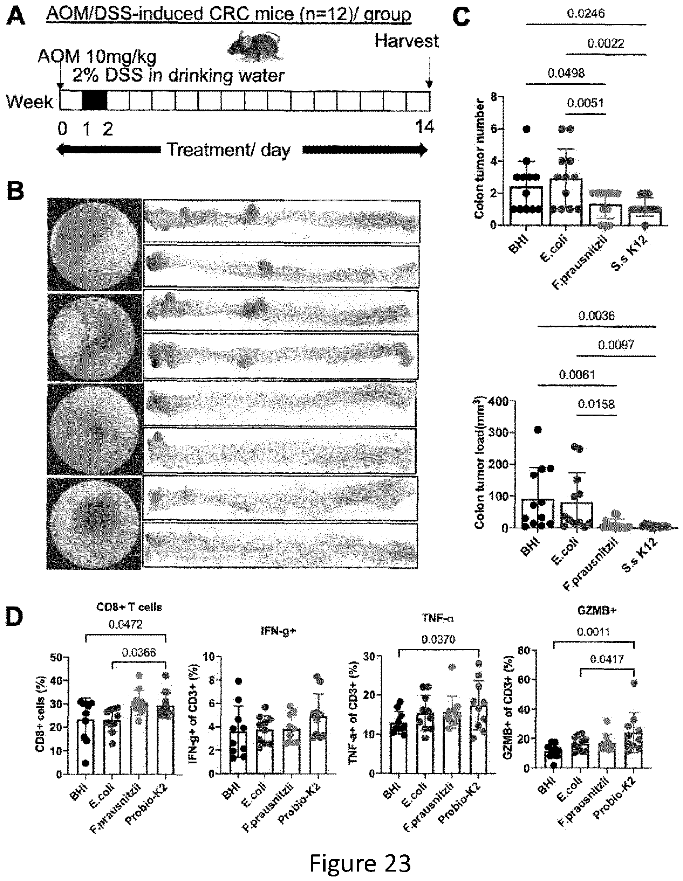

**Figures 23A** - **23D** **shows *S. salivarius* K12 protects against intestinal tumourigenesis and modulates anti-tumour immunity in AOM/DSS mouse model.** Figure 23A is a schematic diagram showing the experimental design, timeline, and representative colonic morphologies of AOM/DSS-induced CRC mice under different treatments. Figure 23B shows representative images of colon tumour from AOM/DSS mouse model. Figure 23C shows colon tumour number and tumour size in AOM/DSS mice under different treatments. Figure 23D shows the proportion of tumour infiltrating CD8+ T cells in AOM/DSS mouse model under different treatments. Results are presented as mean ± S.D. Statistical significance was determined by one-way ANOVA. S.s K12, *Streptococcus salivarius* K12; AOM, azoxymethane.

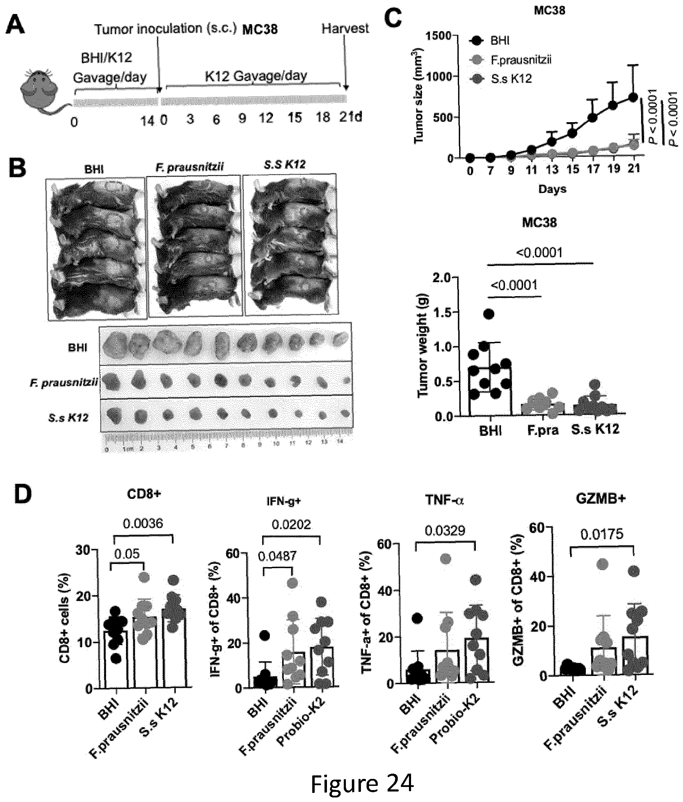

**Figures 24A** - **24D** **shows *S. salivarius* K12 protects against intestinal tumourigenesis and modulates anti-tumour immunity in the MC38 syngeneic mouse model.** Figure 24A is a schematic diagram showing the experimental design, and timeline of MC38 syngeneic mouse model under different treatments. Figure 24B shows representative images of tumours from MC38 syngeneic mouse model under different treatments. Figure 24C shows tumour size and tumour weight in MC38 syngeneic mouse model under different treatments. Figure 24D shows the proportion of tumour infiltrating CD8+ T cells in MC38 syngeneic mouse model under different treatments. Results are presented as mean ± S.D. Statistical significance was determined by one-way ANOVA or two-way ANOVA where appropriate. S.s K12, *Streptococcus salivarius* K12.

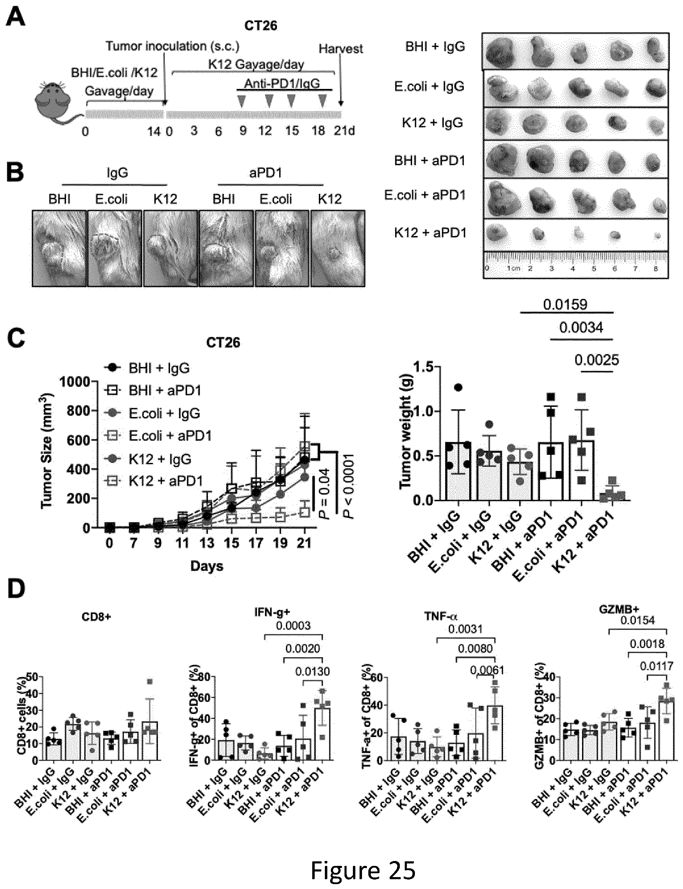

**Figures 25A** - **25D** **shows *S. salivarius* K12 protects against intestinal tumourigenesis and modulates anti-tumour immunity in the CT26 syngeneic mouse model.** Figure 25A is a schematic diagram showing the experimental design, and timeline of CT26 syngeneic mouse model under different treatments. Figure 25B shows representative images of tumours from Ct26 syngeneic mouse model under different treatments. Figure 25C tumour size and tumour weight in CT26 syngeneic mouse model under different treatments. Figure 25D shows the proportion of tumour infiltrating CD8+ T cells in CT26 syngeneic mouse model under different treatments. Results are presented as mean ± S.D. Statistical significance was determined by one-way ANOVA or two-way ANOVA where appropriate. K12, *Streptococcus salivarius* K12; aPD1, anti-PD1.

### DEFINITIONS

In this disclosure, the terms "colorectal cancer (CRC)" and "colon cancer" have the same meaning and refer to a cancer of the large intestine (colon), the lower part of human digestive system, although rectal cancer often more specifically refers to a cancer of the last several inches of the colon, the rectum. A "colorectal cancer cell" is a colon epithelial cell possessing characteristics of colon cancer and encompasses a precancerous cell, which is in the early stages of conversion to a cancer cell or which is predisposed for conversion to a cancer cell. Such cells may exhibit one or more phenotypic traits characteristic of the cancerous cells.

The term "inhibiting" or "inhibition," as used herein, refers to any detectable negative effect on a target biological process, such as RNA/protein expression of a target gene, the biological activity of a target protein, cellular signal transduction, cell proliferation, presence/level of an organism especially a micro-organism, any measurable biomarker, bio-parameter, or symptom in a subject, and the like. Typically, an inhibition is reflected in a decrease of at least 10%, 20%, 30%, 40%, 50%, 60%, 70%, 80%, 90% or greater in the target process (e.g., a subject's bodyweight, or the blood glucose/ cholesterol level, or any measurable symptom or biomarker in a subject, such as an infection rate among subjects by a pathogenic infectious agent), or any one of the downstream parameters mentioned above, when compared to a control. "Inhibition" further includes a 100% reduction, i.e., a complete elimination, prevention, or abolition of a target biological process or signal. The other relative terms such as "suppressing," "suppression," "reducing," and "reduction" are used in a similar fashion in this disclosure to refer to decreases to different levels (e.g., at least 10%, 20%, 30%, 40%, 50%, 60%, 70%, 80%, 90% or greater decrease compared to a control level) up to complete elimination of a target biological process or signal. On the other hand, terms such as "activate," "activating," "activation," "increase," "increasing," "promote," "promoting," "enhance," "enhancing," or "enhancement" are used in this disclosure to encompass positive changes at different levels (e.g., at least about 5%, 10%, 20%, 30%, 40%, 50%, 60%, 70%, 80%, 90%, 100%, 200%, or greater such as 3, 5, 8, 10, 20-fold increase compared to a control level in a target process, signal, or parameter.

As used herein, the term "treatment" or "treating" includes both therapeutic and preventative measures taken to address the presence of a disease or condition or the risk of developing such disease or condition at a later time. It encompasses therapeutic or preventive measures for alleviating ongoing symptoms, inhibiting or slowing disease progression, delaying of onset of symptoms, or eliminating or reducing side-effects caused by such disease or condition. A preventive measure in this context and its variations do not require 100% elimination of the occurrence of an event; rather, they refer to a suppression or reduction in the likelihood or severity of such occurrence or a delay in such occurrence.

The term "severity" of a disease refers to the level and extent to which a disease progresses to cause detrimental effects on the well-being and health of a patient suffering from the disease, such as short-term and long-term physical, mental, and psychological disability, up to and including death of the patient. Severity of a disease can be reflected in the nature and quantity of the necessary therapeutic and maintenance measures, the time duration required for patient recovery, the extent of possible recovery, the percentage of patient full recovery, the percentage of patients in need of long-term care, and mortality rate.

A **"patient"** or **"subject"** receiving the composition or treatment method of this invention is a human, including both adult and juvenile human, of any age, gender, and ethnic background, who have been diagnosed with colon cancer and receiving immunotherapy for the condition and are therefore in need of enhancing efficacy of the treatment. Typically, the patient or subject receiving treatment according to the method of this invention to improve immunotherapy efficacy is not otherwise in need of treatment by the same therapeutic agents. For example, if a subject is receiving the synbiotic composition according to the claimed method, the subject is not suffering from any other disease that is known to be treated by the same therapeutic agents. Although a patient may be of any age, in some cases the patient is at least 40, 45, 50, 55, 60, 65, 70, 75, 80, or 85 years of age; in some cases, a patient may be between 40 and 45 years old, or between 50 and 65 years of age, or between 65 and 85 years of age. A "child" subject is one under the age of 18 years, e.g., about 5-17, 9 or 10-17, or 12-17 years old, including an "infant," who is younger than about 12 months old, e.g., younger than about 10, 8, 6, 4, or 2 months old, whereas an "adult" subject is one who is 18 years or older.

The term **"immunotherapy,"** as used in the context of colon cancer treatment, refers to any treatment methodology that achieves inhibition of cancer cell proliferation, as indicated by tumour mass increase and/or metastasis, by way of inducing, stimulating, or enhancing a patient's immune system functions to suppress or kill cancer cells. For example, **anti-PD1 immunotherapy** is a frequently used cancer treatment strategy that targets the PD1 and PDL1 interaction between cancer cells and immune cells. PDL1 (Programmed Cell Death Ligand 1) is a protein found on the surface of some cancer cells that interacts with the PD1 (Programmed Cell Death 1) protein on T cells, which are a part of the immune system normally capable of immune surveillance and initial elimination of newly arisen cancer cells. The PDL1 to PD1 interaction acts to thwart an attack on the cancer cells by a normal immune system. It's one of the molecular mechanisms that cancer cells are known to rely on to escape detection and destruction by the immune system and thus to continue their uncontrolled growth and proliferation. The use of PDL1 or PD1 inhibitors, the so-called immune checkpoint inhibitors (ICIs), for cancer treatment is therefore known as anti-PD1 immunotherapy for the inhibitors' ability to restore the anti-cancer capability of a patient's immune system. Although many known PDL1 or PD1 inhibitors are antibodies (especially human or humanized antibodies) against the PDL1 or PD1 protein, any other molecules, including small molecules, can also be employed in a PD1 immunotherapy if they are capable of interacting with either PDL1 or PD1 and therefore disrupting the interaction between the two proteins.

The term **"effective amount,"** as used herein, refers to an amount that produces intended (e.g., therapeutic or prophylactic) effects for which a substance is administered. The effects include the prevention, correction, or inhibition of progression of the symptoms of a particular disease/condition and related complications to any detectable extent, *e.g.,* incidence of disease, progression rate, severity of disease, including tumour mass, distant metastasis, and up to mortality. The exact amount will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); and Pickar, Dosage Calculations (1999)).

The term **"about"** when used in reference to a given value denotes a range encompassing ±10% of the value.

A **"pharmaceutically acceptable"** or **"pharmacologically acceptable"** excipient is a substance that is not biologically harmful or otherwise undesirable, *i.e.*, the excipient may be administered to an individual along with a bioactive agent without causing any undesirable biological effects. Neither would the excipient interact in a deleterious manner with any of the components of the composition in which it is contained.

The term **"excipient"** refers to any essentially accessory substance that may be present in the finished dosage form of the composition of this invention. For example, the term "excipient" includes vehicles, binders, disintegrants, fillers (diluents), lubricants, glidants (flow enhancers), compression aids, colours, sweeteners, preservatives, suspending/dispersing agents, film formers/coatings, flavours, and printing inks.

The term **"consisting essentially of**," when used in the context of describing a composition containing an active ingredient or multiple active ingredients, refer to the fact that the composition does not contain other ingredients possessing any similar or relevant biological activity of the active ingredient(s) or capable of enhancing or suppressing the activity, whereas one or more inactive ingredients such as physiological or pharmaceutically acceptable excipients may be present in the composition. For example, a composition consisting essentially of active agents (for instance, one or more of *Lactobacillus gallinarum, Roseburia intestinalis,* and *Streptococcus salivarius* K12) effective for enhancing or improving efficacy of immunotherapy for treating colon cancer in a subject is a composition that does not contain any other agents that may have any detectable positive or negative effect on the same target process (*e.g*., anti-colon cancer efficacy) or that may increase or decrease to any measurable extent of the disease severity or outcome among the receiving subjects.

### DETAILED DESCRIPTION OF THE INVENTION

### I. Introduction

This invention describes the use of certain beneficial gut bacterial species (*e.g.*, one or more of *Lactobacillus gallinarum, Roseburia intestinalis,* and *Streptococcus salivarius* K12), optionally in further combination with certain prebiotics (*e.g*., ICA and butyrate), for enhancing or improving the therapeutic efficacy of an anti-colon cancer treatment that is concurrently administered to a patient suffering from the disease, especially for enhancing or improving the efficacy of an immunotherapy such as the anti-PD1 therapy. The practical use of the invention includes development and manufacturing of commercial food products or health supplements containing the probiotics and/or prebiotics, for example, in the form of a powder, tablet, capsule, or liquid, which can be taken alone or added to food or beverages in connection with anti-colon cancer treatment efforts at or around the same time.

### II. Pharmaceutical Compositions and Administration

The present invention provides pharmaceutical compositions comprising an effective amount of one or more of *Lactobacillus gallinarum, Roseburia intestinalis,* and *Streptococcus salivarius* K12, optionally in further combination with ICA and/or butyrate for enhancing or improving therapeutic efficacy in a colon cancer patient currently receiving immunotherapy such as anti-PD1 therapy for treating the disease. Pharmaceutical compositions of the invention are suitable for use in a variety of drug delivery systems.

The pharmaceutical compositions of the present invention can be administered by various routes, *e.g.,* systemic administration via oral ingestion or local delivery using a rectal suppository. The preferred route of administering the pharmaceutical compositions is oral administration at daily doses of about 10⁶ to about 10¹⁴ CFU, *e.g.,* about 5 × 10⁹ CFU, for each of one or more of *L. gallinarum, R. intestinalis,* and *S. salivarius* K12, at a roughly equal weight ratio, or up to about 3 to 1 weight ratio between the bacterial species at the highest concentration and the lowest concentration. Optionally, the prebiotic combination of ICA and butyrate is further administered to the subject, either in one single composition or in multiple compositions. Typically, ICA and butyrate may be present in the composition in the weight ratio of about 1 to about 4, about 1 to about 2, about 1 to about 1, about 1 to about 2, or about 1 to about 4. For example, ICA and/or butyrate may be present in total amount of about 0.1 to about 4 grams, preferably about 0.6 to about 2.6 grams. Additionally, the composition may be formulated in a daily dosage format comprising ICA and/or butyrate in the total amount of about 0.01 to about 50 mg/kg, about 0.1 to about 25 mg/kg, about 1 to about 20 mg/kg, or about 2 to about 10 mg/kg of the patient. The appropriate dose may be administered in a single daily dose or as divided doses presented at appropriate intervals, for example as two, three, four, or more subdoses per day. The duration of administration may range from about 1 or 2 weeks to about 4 or 8 weeks, *e.g.,* about 1 week to about 2 weeks, or about 1 month to about 2 months, just prior to, during, or shortly after (for example, within about 1, 2, 3, 4, 5 days or a week before or after) an immunotherapy (*e.g.,* anti-PD1 therapy) is administered to the patient.

For preparing pharmaceutical compositions containing one or more of *L. gallinarum, R. intestinalis,* and *S. salivarius* K12, optionally with prebiotics ICA and/or butyrate, one or more inert and pharmaceutically acceptable carriers may be used. The pharmaceutical carrier can be either solid or liquid. Solid form preparations include, for example, powders, tablets, dispersible granules, capsules, cachets, and suppositories. A solid carrier can be one or more substances that can also act as diluents, flavoring agents, solubilizers, lubricants, suspending agents, binders, or tablet disintegrating agents; it can also be an encapsulating material.

In powders, the carrier is generally a finely divided solid that is in a mixture with the finely divided active component, *e.g.,* one or more of *L. gallinarum, R. intestinalis,* and *S*. *salivarius* K12, optionally further in combination with ICA and/or butyrate. In tablets, the active ingredient is mixed with the carrier having the necessary binding properties in suitable proportions and compacted in the shape and size desired.

For preparing pharmaceutical compositions in the form of suppositories, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

Powders and tablets preferably contain between about 5% to about 100% by weight of the active ingredient(s) (*e.g.,* one or more of *L. gallinarum, R. intestinalis,* and *S. salivarius* K12, optionally further in combination with ICA and/or butyrate). Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The pharmaceutical compositions can include the formulation of the active ingredient(s) *e.g.,* one or more of *L. gallinarum, R. intestinalis,* and *S. salivarius* K12, optionally further in combination with ICA and/or butyrate, with encapsulating material as a carrier providing a capsule in which the active ingredient(s) (with or without other carriers) is surrounded by the carrier, such that the carrier is thus in association with the active ingredient(s). In a similar manner, sachets can also be included. Tablets, powders, sachets, and capsules can be used as solid dosage forms suitable for oral administration.

Liquid pharmaceutical compositions include, for example, solutions suitable for oral administration or local delivery, suspensions, and emulsions suitable for oral administration. Sterile water solutions of the active component (*e.g.,* one or more of *L. gallinarum, R. intestinalis,* and *S. salivarius* K12, optionally further in combination with ICA and/or butyrate) or sterile solutions of the active component in solvents comprising water, buffered water, saline, PBS, ethanol, or propylene glycol are examples of liquid or semi-liquid compositions suitable for oral administration or local delivery such as by rectal suppository. The compositions may contain pharmaceutically acceptable auxiliary substances as required to approximate physiological conditions, such as pH adjusting and buffering agents, tonicity adjusting agents, wetting agents, detergents, and the like.

Sterile solutions can be prepared by dissolving the active component (*e.g.,* one or more of *L. gallinarum, R. intestinalis,* and *S. salivarius* K12, optionally further with ICA and/or butyrate) in a previously sterilized solvent under sterile conditions. In some cases, the composition will consist essentially of one or more of *L. gallinarum, R. intestinalis,* and *S*. *salivarius* K12, optionally with ICA and/or butyrate, plus one or more physiological excipients, without another microorganism present at a detectable level, which is defined as undetectable by means of a polymerase chain reaction. The resulting aqueous solutions may be packaged for use as is, or lyophilized, the lyophilized preparation being combined with a sterile aqueous carrier prior to administration. The pH of the preparations typically will be between 3 and 11, more preferably from 5 to 9, and most preferably from 7 to 8.

Single or multiple administrations of the compositions can be carried out with dose levels and pattern being selected by the treating physician. In any event, the pharmaceutical formulations should provide a quantity of an active agent sufficient to effectively enhance the efficacy of a vaccine and/or reduce or eliminate undesirable adverse effects of a vaccine.

### III. Additional Therapeutic Agents

Additional known therapeutic agent or agents may be used in combination with an active agent such as one or more of *L. gallinarum, R. intestinalis,* and *S. salivarius* K12, optionally further in combination with prebiotics such as ICA and/or butyrate, in the practice of the present invention for the purpose of enhancing or improving the efficacy of an immunotherapy for treating colon cancer in a patient in need thereof. In such applications, one or more of these previously known effective anti-cancer therapeutic agents can be administered to patients concurrently with an effective amount of the active agent(s) either together in a single composition or separately in two or more different compositions.

For example, therapeutic agents that are known to be effective for use to treat cancer, especially colon cancer, include Camptosar (irinotecan hydrochloride), capecitabine, Eloxatin (oxaliplatin), 5-fluorouracil, leucovorin calcium, Lonsurf (trifluridine and tipiracil hydrochloride), Stivarga (regorafenib), Tukysa (tucatinib), Zaltrap (ziv-aflibercept), and their combinations may be used along with the active agents (such as the probiotics, optionally with the prebiotics) of the present invention to enhance an immunotherapy's effectiveness, reduce potential disease severity (including morbidity and mortality), prevent/reduce risk of metastasis or recurrence, and improve patient survival and recovery from the disease.

### IV. Kits

The invention also provides kits for improving therapeutic efficacy in an individual receiving immunotherapy for the treatment of colon cancer according to the method disclosed herein. The kits typically include a plurality of containers, each containing a composition comprising one or more of beneficial bacterial species *Lactobacillus gallinarum, Roseburia intestinalis,* and *Streptococcus salivarius* K12. Optionally, additional container(s) may be included in the kit providing composition(s) comprising ICA or butyrate or both. Further, additional agents or drugs that are known to be therapeutically effective for treating colon cancer, including for ameliorating the symptoms and reducing the severity of the disease, as well as for facilitating survival/recovery from the disease (such as those described in the last section or otherwise known in the pertinent technical field) may be included in the kit. The plurality of containers of the kit each may contain a different active agent/drug or a distinct combination of two or more of the active agents or drugs. Also included in the kit, in some cases, is one or more compositions containing agent(s) used in immunotherapy for treating colon cancer, such as any one of PD1 inhibitors (*e.g.,* Pembrolizumab, Nivolumab, Cemiplimab, Dostarlimab, Vopratelimab, Spartalizumab, Camrelizumab, Sintilimab (IBI308), Tislelizumab, Toripalimab and Acrixolimab) or PDL1 inhibitors (*e.g.,* Atezolizumab, Avelumab, Durvalumab, and Cosibelimab) or any combination thereof.

The kit may further include informational material providing instructions on how to dispense the pharmaceutical composition(s), including description of the type of patients who may be treated (*e.g.,* human patients who have been diagnosed with colon cancer and are therefore receiving immunotherapy to treat the disease, such as anti-PD 1 therapy), and in some cases information about the type of patients not to be included in the claimed method (*e.g.,* those who have been diagnosed with a pre-existing condition that already requires the administration of the active components such as one or more of *L. gallinarum, R. intestinalis,* and *S. salivarius* K12, optionally along with ICA and/or butyrate), as well as the dosage, frequency, and manner of administration, and the like.

### EXAMPLES

The following examples are provided by way of illustration only and not by way of limitation. Those of skill in the art will readily recognize a variety of non-critical parameters that could be changed or modified to yield essentially the same or similar results.

### EXAMPLE 1: Lactobacillus gallinarum and Its Derived Metabolite Improve Anti-PD1 Efficacy in Colorectal Cancer by Modulating the IDO1/Kyn/AHR Metabolic Zxis

### BACKGROUND OF THE INVENTION

The introduction of immune checkpoint blockade (ICB) therapy has revolutionized the paradigm of cancer treatment over the past decade. Harnessing the immune system, ICB therapy removes the "brake" signal of T cell activation and elicits antitumour response. ICB therapy, namely anti-PD1/PDL1 (programmed cell death protein 1/ programmed death ligand 1) and anti-CTLA4 (cytotoxic T-lymphocyte-associated protein 4), has yielded great success in clinical trials and is now approved to treat different cancers. Nevertheless, in colorectal cancer (CRC), clinical use of ICB therapy remains highly limited and is only approved in CRC patients with high level microsatellite instability (MSI-H) or mismatch repair deficiency, making up merely 15% of CRC patients. Meanwhile, drug resistance persists as an unresolved challenge - a large portion of patients, despite their eligibility to receive ICB, may be refractory or even fail to respond to anti-PD1 therapy. Therefore, there is a dire need to identify novel adjuvants to overcome ICB resistance and improve treatment efficacy.

Gut microbiota has emerged as an important factor dictating ICB response in various cancers. By using metagenomic profiling, several studies reported a distinct microbiota composition between ICB responders and non-responders in melanoma patients, while supplementing bacteria species enriched in responders or faecal microbiota transplantation to non-responders dramatically improved their ICB efficacy in patients with in melanoma or epithelial cancer. A recent study also demonstrated that a consortium of 11 bacterial strains from healthy donors effectively induced IFNγ+ CD8+ T cells, which boosted antitumour immunity and synergized with ICB therapy.

While earlier studies focus on the correlation between microbiota and host response, the immunoregulatory molecules/components and mechanisms remain largely unknown. In view of this, recent publications have characterized the bacteria-derived immunomodulatory components, which are mostly bacteria metabolites or cell wall fragments such as inosine, exopolysaccharides, phospholipids and peptidoglycan. These have highlighted the potential of leveraging probiotics or bacteria-based therapeutics as a next-generation adjuvant to improve cancer immunotherapy.

We demonstrated that *Lactobacillus gallinarum,* a probiotic depleted in CRC patients, inhibited colorectal tumourigenesis by secreting indole-3-lactic acid (ILA), a tryptophan metabolite. Tryptophan metabolites are known as a class of immunoregulatory metabolites, which could induce Th17 polarization and promote differentiation of CD4+CD8αα+ double-positive intraepithelial lymphocytes. Therefore, we sought to explore the translational potential of this probiotic beyond cancer prevention - whether *L. gallinarum* may modulate antitumour immunity and improve ICB treatment in CRC. Here, we report that *L. gallinarum-*derived indole-3-carboxylic acid (ICA) significantly improved anti-PD1 therapy in CRC. We revealed that ICA reduced regulatory T cell (Treg) infiltration and enhanced the effector function of CD8+ T cells, mechanistically by modulating the indoleamine 2,3-dioxygenase 1 (IDO1)/ Kynurenine (Kyn)/ aryl hydrocarbon receptor (AHR) metabolic axis.

### MATERIALS AND METHODS

### Bacteria strains and growth conditions

*L. gallinarum* (ATCC 33199) and *E. coli* strain MG1655 (ATCC 700926) were purchased from American Type Culture Collection (ATCC; Manassas, VA). *E. coli* strain MG1655 is a non-pathogenic human commensal gut bacterium and was used as a negative control in this study. *L. gallinarum* and *E. coli* were cultured in MRS or BHI broth as appropriate in a shaking incubator at 37°C under aerobic condition, and were resuspended in BHI broth before gavage.

### Cell culture

Murine MC38 (microsatellite instability high, MSI-H) and CT26 (microsatellite stable, MSS) and human (HCT116 and LoVo) CRC cell lines were purchased from ATCC. All cells were cultured in Dulbecco's modified Eagle's medium (Gibco BRL, Grand Island, NY) supplemented with 10% fetal bovine serum (FBS) and 1% penicillin/streptomycin, and maintained at 37°C in a humidified incubator with 5% carbon dioxide.

### Syngeneic mouse model

Male C57BL/6 or BALB/c mice at 5-6 weeks old were randomized and daily gavaged with (1) brain heart infusion (BHI) broth, (2) *Escherichia coli* MG1655, or (3) *Lactobacillus gallinarum* (1×10⁸ colony-forming unit/100µL per mouse). After one week, MC38 (5×10⁵ cells) or CT26 (1×10⁶ cells) were subcutaneously injected into the dorsal flank of C57BL/6 and BALB/c mice, respectively. When the tumour size reached 50-100 mm³, anti-mouse programmed cell death protein 1 (anti-PD1) monoclonal antibody (BE0146, Bio X Cell, Lebanon, NH) or IgG isotype control (BE0089, Bio X Cell) was administered to mice by intraperitoneal injection every 3 days (100µg per mouse). Tumour size was measured by caliper every other day.

For indolecarboxylic acid (ICA) model, ICA (18mg/kg, Sigma-Aldrich, St. Louis, MO) was gavaged to mice daily. Tumour inoculation and dosing schedule of anti-PD1 or isotype control is the same as described above. After the outgrowth of palpable tumours, kynurenine (Kyn, 10mg/kg, Sigma-Aldrich) was intraperitoneally injected into mice twice weekly.

### Carcinogen-induced colon cancer model

Male C57BL/6 mice at 5-6 weeks old were intraperitoneally injected with a single dose of azoxymethane (AOM; Merck, Darmstadt, Germany), followed by 1 week of 1% dextran sulphate sodium (DSS; MP Biomedicals, Solon, OH) in drinking water and a 2-week recovery period. A total of 3 DSS cycles were given. After the third DSS cycle, the mice were randomized and gavaged with BHI broth, *E. coli* or *L. gallinarum* daily. Anti-PD1 or isotype control (100µg) were given to mice by intraperitoneal injection every 3 days after one week of bacterial gavage. Mouse colonoscopy (Karl Storz Endoskope, Tuttlingen, Germany) was performed before sacrifice. All animal studies were approved by the Animal Experimentation Ethics Committee of The Chinese University of Hong Kong.

### Metabolomics profiling and metabolites analysis

Metabolomics was performed by BIOTREE, Shanghai, China. The metabolites were transferred to a fresh glass vial for liquid chromatography-tandem mass spectrometry (LC-MS/MS) analysis. Ultra-high performance liquid chromatography (UHPLC) separation was carried out using a 1290 Infinity series UHPLC System (Agilent Technologies, Palo Alto, CA), equipped with a UPLC BEH Amide column. The TripleTOF 6600 mass spectrometry (AB Sciex, Foster City, California) was used to acquire tandem mass spectrometry (MS/MS) spectra on an information-dependent basis during LC-MS/MS experiment. The metabolite identification was based on in-house MS2 database, Human Metabolome Database (HMDB, website: hmdb.ca), and METLIN metabolite database (metlin.scripps.edu).

### Multicolor flow cytometry analysis

Tumour tissues were dissected into small pieces and digested with 1% bovine serum albumin (BSA) containing 0.5mg/ml collagenase D (Roche, Basel, Switzerland) and 0.25mg/ml DNase I (Roche) for 45 minutes at 37°C on a shaking platform. The cell suspension was then filtered through a 70-µm cell strainer and centrifuged at 700 g for 10 minutes. The cell pellets were resuspended in 1% BSA for surface marker staining. Nuclear antigen staining (Foxp3) was performed using Foxp3 staining buffer set (eBioscience, San Diego, CA) according to manufacturer's instructions. For intracellular cytokine staining, the cell suspension was first stimulated with phorbol myristate acetate, ionomycin and monensin for 4 hours at 37°C before surface marker staining. Then, the cells were fixed and permeabilized by Foxp3 staining buffer (eBioscience), followed by antibody staining.

### Immunohistochemistry staining

Formalin-fixed, paraffin-embedded tissues were sectioned in 4µm thickness. Heat-mediated antigen retrieval was performed using 10mM sodium citrate buffer (pH 6.0) for 20 minutes. Immunohistochemistry (IHC) staining was conducted using the IHC Select HRP/DAB kit (Millipore, Burlington, MA) according to manufacturer's instructions. Tissue sections were stained with IDO1 antibody (ab106134, Abcam, Cambridge, UK) at 1:100 dilution.

### T cell isolation and in vitro experiments

T cells were isolated from spleens of tumour-bearing mice by negative selection using magnetic cell separation (EasySep mouse T cell isolation kit, CD8+ T cell isolation kit or CD4+ CD25+ Regulatory T cell Isolation Kit II; STEMCELL Technologies, Vancouver, Canada). Isolated T cells were cultured in Roswell Park Memorial Institute 1640 medium supplemented with 10% FBS, 50 µM β-mercaptoethanol, 10ng/mL mouse recombinant interleukin-2 (IL-2), 0.1ng/mL transforming growth factor β (TGFβ) and activated with Dynabeads mouse anti-CD3/CD28 beads (Thermo Fisher Scientific, Waltham, MA) for 72 hours. Cells were then collected for analysis.

### Reverse transcription-quantitative PCR (RT-qPCR)

Total RNAs were extracted from cell pellets or colonic tissues using TRIzol Reagent (Life Technologies). Complementary DNA (cDNA) was prepared using Prime Script RT Reagent Kit with gDNA Eraser (Takara, Shiga, Japan). The relative level of specific genes was determined by QuantStudio^{™} 7 Flex Real-Time PCR System (Thermo Fisher Scientific). All gene expression values were normalized to β-actin and calculated using the 2-ΔΔCt method.

### Statistical analysis

Values are expressed as mean ± standard deviation (SD) for both *in vivo* and *in vitro* experiments. Comparisons between two groups were performed using a two-sided Student's *t*-test. Analysis of variance (ANOVA) was used to compare differences among multiple groups, and post-hoc analysis was performed by Tukey's multiple comparisons test. *P*-values < 0.05 indicate statistical significance.

### RESULTS

### L. gallinarum improved anti-PD1 efficacy in syngeneic mouse models

To investigate whether *L. gallinarum* supplementation improves anti-PD1 efficacy, we first established a syngeneic mouse model by inoculating a murine MSI-H CRC cell line (MC38) into mice with daily gavage of either BHI (broth control), *E. coli* (bacterial negative control) or *L. gallinarum* **(****Figure 1A****).** Once tumours were developed, anti-PD1 antibody or IgG isotype control was intraperitoneally injected to mice. *L. gallinarum* remarkably improved anti-PD1 efficacy, as tumour growth was significantly more impeded by the combination of anti-PD1 and *L. gallinarum,* compared to anti-PD1 combined with BHI or *E. coli* control (both *P <* 0.01) **(****Figure 1B** - **D****).**

MC38 syngeneic model is a highly immunogenic model with good immunotherapy response. We next sought to examine whether *L*. *gallinarum* could also be effective in a murine MSS CRC cell line (CT26), a model with low microsatellite stability (MSI-low) and inherited immunotherapy resistance. As expected, anti-PD1 combined with BHI or *E. coli* showed minimal effects on tumour growth in CT26 syngeneic mice **(****Figure 1E** - **G****).** In comparison, *L. gallinarum* supplementation significantly improved anti-PD1 efficacy, and the combination of anti-PD1 and *L*. *gallinarum* induced a marked reduction in tumour weight (*P* < 0.01) **(****Figure 1E & F****)** and volume (*P* < 0.0001) **(****Figure 1G****).** Altogether, these results indicate that *L*. *gallinarum* enhanced anti-PD1 efficacy to suppress tumour growth.

### L. gallinarum reduced Treg infiltration and enhanced effector function of CD8+ T cells

Anti-PD1 efficacy is closely associated with immune cell infiltration. We therefore performed multicolor flow cytometric analysis and examined the change of immune landscape induced by *L. gallinarum.* In MC38 syngeneic mouse model, *L. gallinarum* monotherapy, despite its inefficacy in impeding tumour growth, reduced Foxp3+ CD25+ Treg infiltration, compared to BHI and E. coli group (both P < 0.05) **(****Figure 2B****).** In mice receiving *L*. *gallinarum* plus anti-PD1 therapy, we observed a consistent reduction of Foxp3+ CD25+ Treg infiltration (both P < 0.05) **(****Figure 2B****),** and also a significant increase of IFNγ+ CD8+ T cells (P < 0.01 for BHI, P < 0.001 for E. coli) **(****Figure 2E****),** which is an indicator of improved CD8+ effector T cell function and strengthened antitumour immunity. We then validated our findings in the CT26 syngeneic mouse model. Consistently, *L. gallinarum* reduced Treg infiltration, both in monotherapy (both P < 0.05) and combination with anti-PD1 therapy (both P < 0.01) **(****Figure 2A and 2C****),** and enhanced IFNγ+ CD8+ T cells (both P < 0.001) compared to BHI and E. coli controls **(****Figure 2D and 2F****).**

### L. gallinarum sensitized mice to anti-PD1 therapy in CRC tumourigenesis induced by AOM/DSS

To explore the translational potential of *L. gallinarum* in improving immunotherapy, we next established a carcinogen AOM/DSS-induced CRC tumourigenesis mouse model, which could mimic physiological CRC development in humans. We initiated *L. gallinarum* supplementation after the completion of three DSS cycles, one week before the start of anti-PD1 treatment, so as to better explore the role of *L. gallinarum* as a potential adjuvant to anti-PD1 **(****Figure 3A****).** Consistent with syngeneic mouse models, combining *L. gallinarum* and anti-PD1 significantly inhibited tumour formation as evidenced by colonoscopy images prior to sacrifice **(****Figure 3B****),** and the representative colon images after sacrifice **(****Figure 3C****).** Compared to anti-PD1-treated mice receiving BHI or *E. coli* control, *L. gallinarum* plus anti-PD1 therapy significantly reduced tumour number (both *P* < 0.01) **(****Figure 3D****),** tumour load (both *P* < 0.05) **(****Figure 3E****),** and number of large tumours (diameter ≥ 2mm) (*P* < 0.05 for BHI; *P* < 0.01 for *E. coli*) **(****Figure 3F****),** indicating *L. gallinarum* boosts the efficacy of anti-PD1 therapy in CRC tumourigenesis. Colonic tumours were collected for immune cell analysis by flow cytometric. Consistent with the findings from the MC38 and CT 26 allograft models, compared to anti-PD1-treated mice plus BHI or *E. coli* control, the combination of *L. gallinarum* and anti-PD1 reduced Treg infiltration (*P* < 0.01 for *E. coli*; *P <* 0.001 for BHI) **(****Figure 3G****)** and enhanced CD8+ effector T cell function (both *P* < 0.05) **(****Figure 3H****).** Collectively, *L. gallinarum* synergizes with anti-PD1 therapy in CRC treatment in mice.

### L. gallinarum produced tryptophan as potential functional metabolite in vitro and in vivo

We further characterized the metabolomic profile of *L. gallinarum* to identify functional metabolites that could modulate antitumour immunity. Untargeted metabolomic profiling revealed the significant enrichment of multiple tryptophan metabolites, including indole-3-carboxaldehyde (IAld), indoleacetic acid (IAA) and indolelactic acid (ILA) in *L. gallinarum* culture supernatant **(****Figure 4A****).** In the stool samples of our MC38 syngeneic mouse model, we consistently observed an elevated level of IAld in *L. gallinarum-treated* mice compared to BHI and *E*. *coli*-treated mice **(****Figure 4B****).** For validation, we then performed targeted metabolomic profiling on 32 different tryptophan-related metabolites and confirmed the elevated IAld level in stool samples **(****Figure 4C****).** These results incdicate that *L*. *gallinarum* secreted tryptophan metabolites, specifically lAld, both *in vitro* and *in vivo.*

Given that the tumours of syngeneic mice were located at their dorsal flank which were distant from the intestines, we hypothesized that *L. gallinarum-derived* metabolites were first produced in the gut, then entered the bloodstream and finally reached the tumour tissue. No significant difference in serum lAld level was found between groups **(****Figure 4D****),** however, indicating that lAld may not be the endpoint metabolite modulating antitumour immunity. In contrast, a significant enrichment of indole-3-carboxylic acid (ICA), the downstream metabolite of IAld ²⁰, was observed in serum samples of *L. gallinarum-treated* mice, compared to controls (both *P* < 0.05) **(****Figure 4E****).** Therefore, we postulated that *L. gallinarum* first produced lAld, the precursor of ICA in the gut, which was then converted to ICA in the bloodstream. ICA may be the functional metabolite that played an immunoregulatory role.

### L. gallinarum and ICA inhibited tumour tissue IDO1 expression

In our metabolomic panel of tryptophan metabolites, apart from elevated ICA level, we also observed a decreased Kyn level in both serum (both *P* < 0.05) and tumour tissues (both *P* < 0.01) of MC38 syngeneic mice with *L*. *gallinarum* treatment, compared to controls **(****Figure 5A and 5B****).** A decrease of kynurenine-to-tryptophan (Kyn/Trp) ratio in tumour tissues of*L. gallinarum-*treated mice was also identified (*P* < 0.01 for BHI; *P* < 0.05 for *E*. *coli*) **(****Figure 5C****).** Kyn is a host-derived metabolite notorious for promoting Treg development and immune evasion, and is primarily produced by indoleamine 2,3-dioxygenase (IDO) through tryptophan conversion ²¹. Based on these findings, we hypothesize that *L. gallinarum* suppresses IDO expression and/or activity in tumours, which subsequently reduces Kyn production.

We therefore performed RNA sequencing on tumour tissues from MC38 syngeneic mouse model **(****Figure 5D****).** The tryptophan metabolism pathway was significantly downregulated in *L. gallinarum* plus anti-PD1 group, compared to BHI plus anti-PD1 and *E*. *coli* plus anti-PD1 groups (both *P* < 0.05) **(****Figure 5E****).** We then performed qPCR to validate the sequencing results, and confirmed in both MC38 and CT26 syngeneic mouse models that IDO1 expression in tumour tissues was significantly reduced in *L. gallinarum-treated* mice **(****Figure 5F****).** Immunohistochemical (IHC) staining also confirmed the reduced IDO1 expression in tumour tissues (*P* < 0.01 for *E. coli, P <* 0.001 for BHI) **(****Figure 5G****).** These results suggest that *L. gallinarum* suppressed IDO1 expression in tumour tissues of syngeneic mouse model and reduced Kyn production.

Given that ICA was identified as the functional metabolite of *L. gallinarum* **(****Figure 4****),** we postulated that ICA could be an IDO1 inhibitor. We added IFNγ (100ng/mL) to induce IDO1 expression. Indeed, ICA significantly suppressed IDO1 expression in two human CRC cell lines (HCT116 and LoVo) **(****Figure 5H****).** Taken together, these results indicate that *L*. *gallinarum* or ICA inhibits IDO1 expression and reduces Kyn production in tumour.

### ICA antagonized Kyn-mediated Treg differentiation by inhibiting AHR activation

To explore the impact of *L. gallinarum-*derived metabolites on Treg differentiation, we treated naive CD4+ T cells with *L. gallinarum* culture supernatant, lAld or ICA. However, none significantly affected Treg differentiation was observed by each of these treatments, hinting an indirect effect of ICA on Treg differentiation **(****Figure 6A - C****).** ICA and other tryptophan metabolites are known to activate AHR, while AHR activation is associated with Treg differentiation and immune escape. Therefore, ICA may compete with Kyn, a host-derived AHR agonist highly expressed in tumour tissues, and consequently antagonized Kyn-mediated AHR activation. We simultaneously treated CD4+ T cells with Kyn and ICA. Kyn (50µM) significantly induced Treg differentiation (*P* < 0.0001), while the addition of ICA, at a much lower dose (as low as 1.25µM), antagonized Kyn-mediated Treg differentiation in a dose-dependent manner **(****Figure 6D****).** We then measured the expression of AHR target gene CYP1B1 on CD4+ T cells. ICA alone weakly promoted CYP1B1 expression, indicating ICA as a weak AHR agonist. However, the addition of ICA to Kyn markedly suppressed Kyn-induced CYP1B1 expression **(****Figure 6E****).** The use of CH-233191, an AHR antagonist, also abolished both Kyn and ICA effects in mediating Treg differentiation **(****Figure 6F****).**

We also performed the surface plasma resonance (SPR) assay to explore the interaction between ICA/Kyn with the human AHR receptor. Both ICA and Kyn binds to the human receptor, whereas ICA has a higher receptor affinity (KD value ~39.4µM) to AHR receptor than Kyn (61.0 µM) **(****Figure 6G****).** These support our hypothesis that ICA displaces Kyn from AHR receptor and inhibited Kyn-mediated Treg differentiation through receptor competition.

### ICA phenocopied L. gallinarum effect in vivo, reversed by Kyn supplementation

To confirm the effect of ICA in modulating antitumour immunity *in vivo,* we established a syngeneic mouse model with daily gavage of ICA with or without anti-PD1. ICA significantly promoted anti-PD1 efficacy and phenocopied the effect of *L. gallinarum,* as evidenced by the reduced tumour weight and tumour volume, compared to vehicle control, while Kyn supplementation reversed the effect of ICA **(****Figure 7A-C****).** Consistent with the results of *L. gallinarum-treated* mice **(****Figure 2****),** ICA reduced Treg infiltration and increased IFNγ+ CD8+ T cells, which were also reversed by Kyn supplementation **(****Figure 7D&E****).** ICA also significantly reduced Kyn level in both serum and tumour **(****Figure 7F-G****),** as well as the Kyn/Trp ratio in tumour **(****Figure 7H****).**

Collectively, our results demonstrate that *L. gallinarum* and its derived ICA improve anti-PD1 therapy in a two-fold mechanism: (1) inhibiting IDO1 expression and Kyn production, (2) inhibiting Kyn binding on AHR receptor through receptor competition.

### DISCUSSION

In this study, we report that *L. gallinarum* supplementation sensitizes mice to anti-PD1 therapy to both syngeneic mouse model and CRC tumourigenesis mouse model. We identified ICA, a tryptophan metabolite secreted by *L. gallinarum,* as the key player in remodeling TME through modulating the IDO1/Kyn/AHR metabolic axis. On top of other publications, our results indicate a novel mechanism of how a probiotic species modulates antitumour immunity and improved ICB response.

We first demonstrated that *L. gallinarum* significantly augmented anti-PD1 efficacy and induced a remarkable tumour shrinkage. Accumulating evidence suggested that gut microbiota composition shapes the outcome of cancer immunotherapy, whilst microbiota modulation arises as a promising approach to improve ICB response. For example, a recent study reported that probiotic *Lactobacillus acidophilus* improved ICB efficacy by reducing intratumoural Treg and enhanced effector CD8+ T cells. Similarly, we revealed that *L*. *gallinarum* reduced Foxp3+ CD25+ Treg infiltration in TME and enhanced effector function of CD8+ T cells when combined with anti-PD1, indicating the strengthened antitumour immunity. Notably, *L. gallinarum* was effective in improving anti-PD1 efficacy in CT26 syngeneic mouse model, an MSI-L model known to have minimal response to immunotherapy. Treg recruitment and infiltration is one of the key mechanisms accounting for ICB resistance, which contributed to the immunosuppressive TME and prevented the cytotoxic activities of effector T cells. Meanwhile, to better mimic human CRC development, we also established a carcinogen AOM/DSS-induced CRC mouse model and revealed that *L. gallinarum* also significantly improved anti-PD1 therapy with a marked reduction of tumour number and load. Taken together, our results from different mouse models suggested an immense translational potential of leveraging probiotic *L. gallinarum* in reshaping the immunosuppressive TME and reversing ICB resistance in clinical practice.

Through metabolomic profiling, we identified ICA as the functional immunomodulatory component that improved anti-PD1 therapy. We initially focused on lAld, given its enrichment in *L. gallinarum* culture supernatant and stool samples of *L. gallinarum-*treated mice. However, metabolomics revealed no significant difference in serum lAld level, but a significant enrichment of ICA, the downstream metabolite of IAld, between *L. gallinarum* and control groups. Therefore, we reasoned that *L. gallinarum* first produced lAld, which was further converted to ICA after entering the bloodstream, and ICA functioned as the endpoint metabolite that modulated antitumour immunity. Our previous work demonstrated that *L*. *gallinarum* produced L-tryptophan, while ILA, its downstream catabolites, exerted anticancer effect and inhibited colorectal tumourigenesis. Concerning the highly dynamic nature of microbial tryptophan metabolism, we posited that the tryptophan metabolism pathway may be diverted to a specific pathway *in vivo,* probably subject to multiple factors including species, disease model and treatment model, therefore accounting for the discrepancy of metabolites identified. Of note, the tryptophan metabolite family shares many common biological features, such as binding to AHR, improving gut barrier function and regulating inflammation. In view of this, we reasonably believe that ICA is not the sole metabolite that modulated antitumour immunity, but many different tryptophan metabolites may also play a role in remodeling tumour microenvironment. We also acknowledge that indole metabolites can be produced by a wide variety of gut bacteria. Therefore, instead of pinpointing *L. gallinarum* as the "star" probiotics, it is likely that many gut bacteria also promotes ICB response through a similar mechanism.

We identified that *L. gallinarum* and its derived ICA regulated the host tryptophan metabolism pathway and inhibited IDO1 expression in tumour tissues, leading to a reduced production of Kyn, a host-derived metabolite contributing to tumour progression, immune escape, and Treg development. The conversion of tryptophan to Kyn is regulated by IDO1, which is highly expressed in multiple human cancers and has been an attractive pharmaceutical target. In line with our observations, several studies reported the role of probiotics in inhibiting IDO1 expression and lowering Kyn concentration *in vivo.* Kyn/Trp ratio is also known to be a prognosis marker predicting ICB response. Here we report an effector mechanism of how gut microbiota modulated ICB response through host-bacteria metabolic crosstalk. In fact, tryptophan metabolism involves three major pathways, which are modulated by host, epithelial/ immune cells and enterochromaffin cells respectively. It is also known that modulation of one of these metabolic pathways profoundly disturb the others. Collectively, our results indicate that *L. gallinarum* and its derived ICA, exert the immunomodulatory effect, at least in part, by modulating the host tryptophan metabolism pathway and inhibiting Kyn production in the TME.

Apart from interfering with the upstream IDO1 enzyme, a downstream mechanism was also reported involving a receptor competition between ICA and Kyn for the AHR binding site of T cells, which subsequently inhibited Treg differentiation. We first found that *L*. *gallinarum-*derived ICA or lAld had insignificant immunomodulatory effect on Treg differentiation, indicating that ICA improves ICB response through an indirect manner. Indeed, our results confirm that ICA can suppress Kyn-mediated Treg differentiation through an AHR-dependent manner. Tryptophan metabolites including both indoles and Kyn are widely reported as AHR binding ligands. Several studies reported the antagonistic activity of indole metabolites - while indole metabolites alone function as a weak AHR agonists, they also exhibited antagonistic activity and inhibited agonist-induced AHR activation. AHR agonists or indole metabolites are known to play a pleotropic role in cancer. While AHR agonists alleviated colonic inflammation and thus potentially prevented colitis-associated CRC, they also promoted immune evasion in TME in the context of cancer. In a recent publication, *Lactobacillus-*derived indole metabolites were reported to drive immunosuppression by activating AHR on tumour-associated macrophages to promote tumour progression. In our study, we demonstrated a competitive nature between host- and microbiota-derived AHR ligands, showing that *L. gallinarum-derived* ICA (a weak AHR agonist) outcompeted Kyn and inhibited Kyn-mediated AHR activation. In fact, the use of partial agonist is not a new concept in drug development. Several drug classes in clinical use today, such as opioids (for chronic pain relief) and beta blockers (for treating cardiovascular disease), also include members with partial agonistic activity. Partial agonists bind and activate receptors, but at a much weaker extent compared to a full agonist. In this regard, they prevented full receptor activation, meanwhile maintaining physiological homeostasis. Our results collectively indicate that gut microbiota-derived AHR ligands function as a partial AHR agonist and suppress AHR activation in TME in the presence of Kyn, a potent AHR agonist.

In conclusion, we have demonstrated that *L. gallinarum* and its derived ICA can improve anti-PD1 efficacy in CRC. Such actions are associated with the inhibition of IDO1/Kyn metabolic circuit, as well as the antagonism of Kyn binding on AHR receptors on T cells to inhibit Treg differentiation. Our results reveal a novel mechanism underlining host-microbiota metabolic crosstalk and highlight the translational potential of leveraging *L*. *gallinarum* as an adjuvant therapy to improve ICB response.

### EXAMPLE 2: Butyrate Producing Bacteria Roseburia intestinalis Mediates Cytotoxic cd8⁺ T Cell Immunity against Colorectal Cancer

### BACKGROUND OF THE INVENTION

Colorectal cancer (CRC) is the third major cancer type and remains the third guiding cause of cancer-related death worldwide. Despite significant clinical responses to immunological checkpoint inhibitors in melanoma and non-small cell lung cancer patients, the role of immunotherapy on CRC treatment is limited. Clinical evidence demonstrated that only microsatellite instable (MSI) CRC patients responded to PD-1 inhibitor, while only 10-15% of CRC tumours are MSI. The treatment of most microsatellite stable (MSS) CRC patients leaves a major challenge for CRC immunotherapy. CD8⁺ T cells play an essential role in tumour immunity, therefore, the promotion of functional CD8⁺ T cell infiltration to tumour microenvironment might improve the CRC therapy efficiency, especially MSS type.

Microbiota contributes to antitumour efficiency by modulating host immune response, working alone or facilitating immunotherapy. Microbiota depletion by antibiotic consumption or germ-free mice was related with nonresponse to immunotherapy and oral supplementation of bacteria or faecal microbial transplantation to antibiotic-treated or germ-free mice restored the immunotherapy response. Though the necessity of microbiota in immunotherapy was confirmed, whether and how some certain bacteria improve the antitumour efficiency in CRC patients, especially in MSS-type are still not fully understood.

Gut microbiota affected cancer development by producing metabolites. Butyrate, as one of the essential bacterial metabolites in colon, its concentration is negatively correlated to CRC incidence. Butyrate producing bacteria including five *Roseburia* OTUs were depleted, and Butyryl-coenzyme CoA transferase genes were reduced in the microbiota of CRC patients. These data prompted us to investigate whether *R. intestinalis* and its metabolite butyrate can contribute to the efficacy of antitumour immunity, either alone or in combination with cancer therapy.

*Roseburia intestinalis,* a gram-positive, obligate anaerobic, butyrate-producing bacterium, firstly isolated from human faecal material in 2002. Previously, we established a probe-based duplex quantification PCR panel of bacteria biomarkers to diagnose colorectal cancer based on two Asian cohorts. *R. intestinalis* is one of the bacterial markers and depleted in CRC patients, but whether the altered abundance of *R. intestinalis* is a cause or consequence in CRC development needed further confirmation.

The protect role of *R. intestinalis* in colorectal cancer was invested in two spontaneous AOM-induced and *Apc*^{*Min*/*+*} murine models and *in vitro. R. intestinalis* producing butyrate directly boosting cytotoxic CD8⁺ T cells through Toll-like receptor 5 (TLR5) dependent NF-κB signalling. *R. intestinalis* restricted MSI-type MC-38 and MSS-type CT-26 orthotopic tumour growth by modulating antitumour CD8⁺ T cell response, indicating that *R*. *intestinalis* and its metabolite butyrate can be supplied to improve the anti-colorectal cancer therapy efficacy.

### MATERIALS AND METHODS

### Metagenomic cohorts of colorectal cancer patients and healthy controls

Four published metagenomic data cohorts from China, Germany, France, and Japan, including 334 CRC patients and 463 healthy individuals, were used to evaluate the abundance of *R. intestinalis.* Metagenomic data sets were downloaded from the European Nucleotide Archive (ENA) with the following ENA identifiers: PRJEB10878 for YuJ_2017, PRJEB27928 for WirbelJ_2019, ERP005534 for ZellerG_2014 and DNA Data Bank of Japan for YachidaS_2019 with accession number: DRA006684 and DRA008156.

Another independent Chinese cohort named as "YuJ_2021" of 110 CRC patients and 112 healthy controls were recruited from the participants under colonoscopy screening at the Jockey Club Bowl Cancer Education Centre, The Chinese University of Hong Kong. The clinical study protocol was approved by Joint Chinese University of Hong Kong-New Territories East Cluster Clinical Research Ethics Committee. All participants signed an informed consent form. The mycobioa partial of the metagenomic data was published by our team and the details of sample collection was shown in that study.

### Metagenomic cohorts of responders and non-responders to immune checkpoint inhibitors

Metagenomic sequences data to analyze the relative abundance of *R. intestinalis* in responders and non-responders to immune checkpoint inhibitors (ICI) were downloaded from SRA under the Bioproject accession PRJNA751792 and PRJNA782662. This France cohort included 333 non-small-cell lung cancer patients (258 non-responders and 75 responders). Stool samples were collected before the ICI therapy start.

### Targeted metabolomics cohort of colorectal cancer patients and negative controls

A published metabolomics cohort including 118 CRC patients and 128 negative control individuals was performed gas chromatography coupled to time-of-flight mass spectrometer (GC-TOFMS) targeted a human gut microbiota-host co-metabolism panel by the authors and the significantly altered metabolites between CRC and NC were downloaded from Pubmed. Metabolites with variable importance in projection (VIP) score >1 and P value <0.05 were considered as significant.

### Bacterial strain culture

*Roseburia intestinalis* (DSM 14610) was purchased from Leibniz Institute DSMZ-German Collection of Microorganisms and Cell Cultures GmbH (Braunschweig, DE) and cultured at 37 °C in modified Yeast Casitone Fatty Acids (YCFA) Broth with Hungate bottles filled with N₂. *E. coli* MG1665 was used as a bacteria control and cultured at the same condition with *R. intestinalis.* When the absorbance of *R. intestinalis* reached to the optical density 600 (OD₆₀₀) of 1.0, the cultured medium was centrifuged and filtered with 0.22 µm pore size filter to get the *R. intestinalis* cultured medium (*R.i* CM). We obtained the *E. coli* cultured medium (*E. coli* CM) at the same condition. Fractions larger than 3KD and less than 3KD were separated with 3,000 Dalton molecular weight cutoff filters (UFC9003, Merck KGaA, Darmstadt, DE).

### Experimental animals

The intestinal adenoma spontaneous mouse strain C57BL/6J-*Apc^{Min}*/J (The Jackson Laboratory, Bar Harbor, US) was orally treated with 1×10⁸ colony forming unit (CFU) *R. intestinalis* daily at the age of 6 weeks, and they are raised for the following 6 weeks. Same amount of *E. coli* MG1665 was used as bacterial control and same volume of phosphate buffered saline (PBS) was used as negative control.

6 dosages of azoxymethane (AOM, A5486, Sigma-Aldrich, Darmstadt, DE) were injected into 6-week-old C57BL/6 mice to induce sporadic tumour development. The same treatment with the *Apc*^{*Min*/*+*} model was used one week after the last dosage of AOM injection and lasted for 30 weeks continuously.

A MSI type mouse CRC cell line, MC-38 cell mix [5×10⁵ cells in 10µL Matrigel (354248, Corning, Manassas, US) per mouse] was orthotopically implanted into the rectum of C57BL/6. When the tumour was established or three days after implantation, mice were started with *R*. *intestinalis, E. coli* or sodium butyrate (NaBu, 303410, Sigma-Aldrich, Darmstadt, DE) treatment and lasted for three weeks. 1×10⁸ CFU *R. intestinalis* or *E. coli* were given by oral gavage daily; 150 mM NaBu was provided in the drinking water and refreshed every two days. Mice in other groups received saline in the same amount of sodium as control. αPD-1 antibody (100µg per mouse, BP0146, Colone: RMP1-14, Bio X Cell, West Lebanon, US) or control IgG (100µg per mouse, BP0089, Colone: 2A-3, Bio X Cell) was injected intraperitoneally into MC-38 bearing mice, and followed by total five injections with three days interval one week after MC38 implantation.

CT-26 tumour-bearing BALB/c mice with the same setting and treatment of MC-38 orthotopic model was developed to evaluate the efficacy of αPD-1 antibody immunotherapy on microsatellite stable (MSS)-type colorectal cancer. All experimental procedures were approved by the Chinese University of Hong Kong Animal Ethics Committee.

### Histology Evaluation

Formalin-fixed paraffin embedded colon tissues from each *Apc*^{*Min*/*+*} and AOM-induced mouse were stained with hematoxylin and eosin (H & E) and sent to pathologists for histology evaluation. Benign tissues were scored as "0"; tissues with low grade dysplasia were scored as "1"; high grade dysplasia got "2".

### Immunohistochemistry (IHC) Staining

To analyze the expression of Ki-67, ZO-1 and Claudin-3, colon tissue slides were deparaffinized and rehydrated, followed with antigen unmasking and blocking. Slides were then incubated with primary antibodies Ki-67 (D3B5) Rabbit mAb (12202, Cell Signalling Technology, Danvers, US, 1:200), anti-ZO-1 antibody (ab96587, abcam, Cambridge, UK, 1:100) or Claudin-3 polyclonal antibody (34-1700, Thermo Fisher Scientific, Waltham, US, 1:100) at 4 °C overnight, biotinylated secondary antibodies and streptavidin HRP at room temperature for 30 minutes each. Signals were developed with DAB chromogen. High-power fields were randomly shoot and the positive cells were calculated by ImageJ 1.53a with IHC Profiler plugin.

### Fluorescein Isothiocyanate-Dextran intestinal permeability assay

Each mouse was orally gavaged with 150 µL of 80 mg/mL 4KDa FITC-dextran (68059, Sigma-Aldrich, Darmstadt, DE) after 6 hours-fasting. About 50 µL blood was collected 4 hours after FITC-dextran gavage. 100 µL of PBS diluted (1:5) plasma and standards were transferred to a blank opaque-bottom 96-well plate and the fluorescence was determined at 528 nm with 485 nm excitation. Permeability was expressed as relative fluorescence units between groups.

### Western blotting analysis

To determine the expression of tight junction related proteins, total proteins from mice colon tissues were extracted and separated by SDS-PAGE gel, then transferred onto 0.2 µm Nitrocellulose Membrane (1620112, Bio-Rad, Redmond, US). The membrane was blocked, incubated at 4°C overnight with primary antibodies anti-ZO1 tight junction protein antibody (ab96587, abcam, Cambridge, UK, 1:500) and Claudin-3 polyclonal antibody (34-1700, Thermo Fisher Scientific, Waltham, US, 1:1000), β-actin (13E5) Rabbit mAb (4970, Cell Signalling Technology, Danvers, US, 1:1000) was used as internal control, and followed with one-hour secondary antibody incubation at room temperature.

Primary antibodies Caspase-3 (8G10) Rabbit mAb (9665, 1:1000), Cleaved Caspase-3 (Asp175) antibody (9661, 1:1000), Caspase-7 antibody (9492, 1:1000), Cleaved Caspase-7 (Asp198) antibody (9491, 1:1000), Caspase-9 (C9) Mouse mAb (9508, 1:1000), PARP (46D11) Rabbit mAb (9532, 1:1000), Cleaved PARP (Asp214) Rabbit mAb (5625, 1:1000), Cdk6 (D4S8S) Rabbit mAb (13331, 1:1000), p27 Kip1 (D69C12) (3686, 1:1000) purchased from Cell Signalling Technology (CST, Danvers, US) were used to analyse apoptosis and cell cycle related protein expression on CRC cells HCT116, LoVo and SW480.

Phospho-NF-κB p65 (Ser536) Rabbit mAb (3033, Cell Signalling Technology, Danvers, US, 1:1000) and NF-κB p65 (D14E12) Rabbit mAb (8242, Cell Signalling Technology, Danvers, US, 1:1000) were used to measure NF-xB nuclear translocation in αCD3/αCD28/IL2 activated Jurkat E6.1 T cells.

### Cell culture

Normal human epithelial cell NCM460, human CRC cell lines HCT116, LoVo, SW480, and mouse CRC cell lines MC38 and CT26 were cultured in Dulbecco's Modified Eagle's Medium (DMEM) with 10% fetal bovine serum (FBS) and 1% penicillin-streptomycin (P/S). Jurkat E6.1 (TIB-152, ATCC) was cultured in RPMI-1640 supplied with 10% FBS and 1% penicillin-streptomycin and activated in 3 µg/ml anti-CD3 (300332, BioLgend, San Diego, US), 5 µg/ml anti-human CD28 (302934, BioLgend, San Diego, US) and 100 IU/ml recombinant human IL-2 (589104, BioLgend, San Diego, US) pre-coated wells for 3 days.

### Cell viability assay and colony formation assay

1000 cells per well were seed into a 96-well plate and treated with alive or pasteurized *E. coli* (CFU=10⁶/ml) and *R. intestinalis* (CFU=10⁶/ml), 5% (vol/vol) YCFA, *E. coli* CM, *R. i* CM in DMEM separately. Cell viability was determined by Thiazolyl Blue Tetrazolium Bromide (M5644, Merck KGaA, Daemstadt, DE) 3 days constantly.

For cell colony formation assay, same amounts of cells were seed into 6-well plates, followed with the same treatments as cell viability assay for one to two weeks and changed the culture medium freshly every 3 days. After cold Methanol fixation, cells were stained with 0.5% crystal violet solution. Cell colonies were measured with ImageJ 1.53a.

### Immunocytochemistry (ICC) staining

Human CRC cells seed on poly-L-lysine coated coverslips in 4-well plate and fixed with ice-cold methanol for 10 minutes. Then, cells were blocked with 1% BSA in PBST for 30 minutes and incubated with Ki-67 (8D5) mAb (9449, Cell Signalling Technology, Danvers, US, 1:6400) in a humidified chamber at 4°C overnight and Alexa Fluor 488 goat anti-mouse IgG secondary antibody (A-11001, Thermo Fisher Scientific, Waltham, US, 1µg/mL) for one hour at room temperature in the dark. Cell nuclear was stained with DAPI (P36935, Thermo Fisher Scientific, Waltham, US) for one minute for cell counting before mounting. Images were captured with a confocal laser scanning microscope (TCS SP8, Leica, Buffalo Grove, US). Ki-67 positive cell percentage was Alexa Fluor 488 positive cell amounts in total cell amounts.

### Cell apoptosis and cell cycle

Flow Cytometry was used for cell apoptosis and cell cycle analysis with FACSAria cell sorter (BD Biosciences, Franklin Lakes, US). Data was analyzed by FlowJo 10.4.

Cell apoptosis was detected by FITC Annexin V apoptosis detection kit (556547, BD Biosciences, Franklin Lakes, US). Adherent cells were removed by Trypsin-EDTA solution and washed with cold PBS twice and then resuspend in binding buffer at a concentration of 1 × 10⁶ cells/ml. 100 µl of cell solution was transferred to a 5ml tube, incubated with 5 µl FITC Annexin V and 5 µl PI for 15 minutes at 25 °C in the dark. Cells were added with 400 µl binding buffer to stop staining and analyzed by flow cytometer in one hour.

For cell cycle analysis, cell pellet was washed by PBS and incubated in 70 to 80% ethanol at -20°C for more than two hours. In each test, 10⁶ cells were used for staining in 0.5 ml PI/RNase staining buffer (550825, BD Biosciences, Franklin Lakes, US) for 15 minutes at room temperature in the dark. Tubes were stored at 4 °C protected from light and analyzed on flow cytometer with one hour.

### Patient-derived colorectal cancer organoid culture

CRC patient-derived organoid was obtained from the Princess Margaret Living Biobank (PMLB), originally from a 46-year-old female colorectal adenocarcinoma patient. The minced pathologic specimens were digested and embedded into Matrigel and maintained in Advanced DMEM/F12, supplied with 1% P/S, 10 mM HEPES, 1×GlutaMAX medium containing N2 and B27 supplements, 50% (vol/vol) Wnt3a conditional medium, 10% (vol/vol) R-spondin-1 conditional medium, 100ng/ml Noggin conditional medium, 50ng/ml EGF, 10nM Gastrin, 100ng/ml FGF10, 1.25 mMN-acetylcysteine, 500nM A8301, 1µM SB202190, 10mM Nicotinamide. Treatments containing 5% (vol/vol) YCFA, *E. coli* CM or *R. i* CM were added into the culture medium directly and freshly changed every three days. Surface area of organoid was measured by Image J 1.53a.

### Untargeted metabolomic profiling

Metabolomic profiling was performed by BIOTREE, Shanghai, China. 400µL extract solution containing isotopically labelled internal standard mixture was added into100µL each sample of YCFA<3kDa, *E. coli* CM<3kDa and *R.i* CM<3kDa, sonicated in iced water bath for 10 minutes, and incubated at -40 °C for one hour to precipitate proteins. After centrifuge at 13800 g for 15 minutes at 4 °C, the supernatant was transferred to a new glass vial for liquid chromatography tandem mass spectrometry (LC-MS/MS) analysis using an ultra-high performance liquid chromatography (UHPLC) system (Vanquish, Thermo Fisher Scientific, Waltham, US) with a UPLC BEH Amide column (2.1 mm × 100 mm, 1.7 µm), coupled to Orbitrap Exploris 120 mass spectrometer (Orbitrap MS, Thermo Fisher Scientific) on information-dependent acquisition (IDA) mode controlling by the acquisition software (Xcalibur, Thermo Fisher Scientific). Electrospray ionization (ESI) source conditions were as following: sheath gas flow rate was 50 Arb, Aux gas flow rate was 15 Arb, capillary temperature was 320 °C, full MS resolution was 60000, MS/MS resolution was15000, collision energy was 10/30/60 in NCE mode, spray voltage was 3.8 kV (positive) or -3.4 kV (negative) respectively.

### Targeted short chain fatty acid gas chromatography-mass spectrometry analysis

To confirm the findings from untargeted metabolomic profiling, metabolites were extracted from less than 3 kDa fractions of YCFA, *E. coli* CM and *R.i* CM for analysis with gas chromatography-mass spectrometry (GC-MS, GC2030-QP2020 NX, Shimadzu, Tokyo, JP) utilized with an Agilent HP-FFAP capillary column (30m×250µm×0.25µm, J&W Scientific, Folsom, US). 1 µL aliquot of sample was injected in split mode (5:1). Helium was used as the carrier gas. Front inlet septum purge flow was 3 ml/min, and column flow was 1 ml/min. The program of oven temperature ramp was as follow: 80 °C hold on 1 min; then raised to 200 °C at a rate of 10 °C/min^{,} hold on 5 min; then raise to 240 °C at a rate of 40 °C/min and hold on 1 min. The front injection, transfer line, ion source and quad temperatures were 240 °C, 240 °C, 200°C and 150 °C. The electron energy was -70 eV. The mass spectrometry data were acquired in Scan/SIM mode with the m/z range of 33-150 after a solvent delay of 3.5 min. Standard curves were established with acetic acid (64-19-7, Dr. Ehrenstorfer, Augsburg, DE), propionic acid (79-09-4, Dr. Ehrenstorfer, Augsburg, DE), isobutyric acid (79-31-2, Dr. Ehrenstorfer, Augsburg, DE), butyric acid (107-92-6, Dr. Ehrenstorfer, Augsburg, DE), isovaleric acid (503-47-2, Dr. Ehrenstorfer, Augsburg, DE), valeric acid (109-52-4, Dr. Ehrenstorfer, Augsburg, DE), hexanoic acid (142-62-1, Dr. Ehrenstorfer, Augsburg, DE), heptanoic acid (111-14-8, Dr. Ehrenstorfer, Augsburg, DE), octanoic acid (124-07-2, Dr. Ehrenstorfer, Augsburg, DE), nonanoic acid (112-05-0, Dr. Ehrenstorfer, Augsburg, DE), decanoic acid (334-48-5, Dr. Ehrenstorfer, Augsburg, DE), 2-Methylvaleric acid (97-61-0, Dr. Ehrenstorfer, Augsburg, DE).

### T cell isolation and activation

T cells isolated from healthy human peripheral blood mononuclear cells (PBMC) using immunomagnetic negative selection kit (17951, Stemcell Technologies, Vancouver, CA). Isolated T cells were then cultured in complete RPMI-1640 medium supplied with human CD3/CD28 magnet beads (11161D, Thermo Fisher Scientific, Waltham, US) and 30 U/ml recombinant human IL-2 (589104, BioLgend, San Diego, US) for expiation and activation for three days. Mouse T cells were isolated from the spleen of naive mice and purified with Mouse T cell isolation kit (19851, Thermo Fisher Scientific, Waltham, US), cultured in complete RPMI-1640 medium with mouse CD3/CD28 beads (11456D, Thermo Fisher Scientific, Waltham, US) and 30 U/ml recombinant mouse IL-2 (575404, BioLegend, San Diego, US).

### Immune cell characterization

Cytotoxic CD8⁺ T cell markers Granzyme B, IFN-γ, TNF-α under the *in vitro* treatments of *R.i* CM, NaBu or TLR5 inhibitor TH1020 (HY-116961, MedChemExpress, Monmouth Junction, US) were analyzed with flow cytometry. Isolated human T cells were stimulated with 30ng/ml PMA, 1µg/ml ionomycin and 2.5 µg/ml monensin in complete RPMI-1640 medium at 37 °C for 4 hours. For surface antigens staining, cells were stained with Brilliant Violet 605 anti-human CD45 antibody (2D1) (368524, BioLegend, San Diego, US), PE anti-human CD3 antibody (OKT3) (317308, BioLegend), PE/Cyanine5 anti-human CD4 antibody (RPA-T4) (300510, BioLegend), Brilliant Violet 421 anti-human CD8a antibody (SK1) (344748, BioLegend) in FACS buffer on ice for 30 minutes. After fixing with Transcription Factor Fixation/Permeabilization buffer (00-5521-00, Invitrogen, Waltham, US), intracellular cytokines were stained in 1 × Permeabilization buffer (00-8333-56, Invitrogen, Waltham, US) with PE/Cyanine7 anti-human/mouse Granzyme B recombinant antibody (QA16A02) (372214, BioLegend), Brilliant Violet 711 anti-human IFN-γ antibody (4S.B3) (502540, BioLegend), FITC anti-human TNF-α antibody (Mab11) (502906, BioLegend). Granzyme B, IFN-γ, TNF-α negative cells were gated in CD8⁺ T cells (CD45⁺ CD3⁺ CD8⁺) without intracellular staining.

For tumour-infiltrating immune cell analysis, tumour from MC-38 and CT-26 bearing rectal orthotopic mouse models was minced on ice and digested in PBS buffer containing 0.5mg/mL collagenase IV and 0.25mg/ml DNase I at 37 °C for 30 min. After digestion, tumour tissue was filtered through a 70 µm cell strainers, and then distributed into different panels. For the surface staining of MDSC, cells were stained with Brilliant Violet 605 anti-mouse CD45 antibody (30-F11) (103140, BioLegend), PerCP/Cyanine5.5 anti-mouse/human CD11b antibody (M1/70) (101228, BioLegend), PE anti-mouse Ly-6G/Ly-6C (Gr-1) antibody (RB6-8C5). MDSC was gated in CD45⁺ CD11b⁺ Gr-1⁺. For CD8 functional markers staining, the procedures were similar with the *in vitro* T cell analysis. Antibodies used for staining are as following: Brilliant Violet 605 anti-mouse CD45 antibody (30-F11) (103140, BioLegend), PE anti-mouse CD3 antibody (17A2) (100206, BioLegend), Brilliant Violet anti-mouse CD8a antibody (53-6.7) (100738, BioLegend), PE/Cyanine5 anti-mouse CD4 antibody (GK1.5) (100410, BioLegend), PE/Cyanine7 anti-human/mouse Granzyme B recombinant antibody (QA16A02) (372214, BioLegend), FITC anti-mouse IFN-γ antibody (XMG1.2) (505806, BioLegend), Brilliant Violet 711 anti-moue TNF-α (MP6-XT22) (506349, BioLegend). Flow Cytometry was performed on FACSAria cell sorter (BD Biosciences, Franklin Lakes, US). Data was analyzed by FlowJo 10.4.

### RNA isolation and quantitative real-time PCR (qPCR)

Total RNA was extracted with Trizol (15596018, Invitrogen, Waltham, US) from naive mouse spleen isolated T cells after CD3/CD28/IL-2 activation and *R.i* CM and Butyrate treatments. Then, RNA was reverse transcript into cDNA with RT reagent kit with gDNA eraser (RR047A, Takara, Tokyo, JPN). qPCR was performed using TB Green Premix (RR420A, Takara) in the QuantStudio 7 Flex System (Thermo Fisher Scientific, Waltham, US). The relative RNA expression was normalized to that of *β-actin* as denominators.

### RESULTS

### Roseburia intestinalis is depleted in stool samples of colorectal cancer patients

Besides the cohorts to discover the novel noninvasive CRC diagnosis method in our previous study, the abundance of *R. intestinalis* was evaluated in four other published metagenomic data cohorts from China, Germany, France, and Japan, including 334 CRC patients and 463 healthy individuals as well (**Figure 8A**). Another inhouse Chinese cohort of 110 CRC patients and 112 healthy controls were also recruited from the participants under colonoscopy screening to investigate the *R. intestinalis* abundance (**Figure 8B**). In these five cohorts, *R. intestinalis* was depleted in the faecal samples of CRC patients.

To link the gut microbiota-associated metabolites to colorectal carcinogenesis, metabolomic profiling was performed on faecal samples from 118 CRC patients and normal controls, and butyric acid was depleted in CRC patients (**Figure 8C**). *R. intestinalis* was also depleted in CRC patients in this study. The negative associations of *R. intestinalis* and butyrate with CRC from human faecal metagenomic and metabolomic sequencing data indicate their potential tumour suppressive role on CRC.

After reanalysis of a published metagenomics cohort, we found that *R. intestinalis* was enriched in the stool samples of 75 immune checkpoint inhibitor responders compared to 258 non-responders in non-small-cell lung cancer (**Figure 8D**), which could be used as a biomarker to refine patient stratification to immunotherapy.

### Roseburia intestinalis inhibited colorectal cancer development in murine colorectal cancer models

Two spontaneous mouse models were used to evaluate the tumour suppressive role of *R. intestinalis* on CRC. We started daily gavaging 6-week-old *Apc*^{*Min*/}*⁺ mice R. intestinalis* (*R.i*) or *E. coli* strain MG1655 for continuous 6 weeks (**Figure 9A**). *R. i* significantly reduced the colon tumour incidence (**Figure 9B****,** **9C**), tumour number and size (**Figure 9D**) compared those treated with *E. coli* and PBS. Besides, colon tissues from *R. i* treated mice got lower dysplasia score under histology evaluation and obviously less Ki-67 positive cell proportion was observed in both tumour and non-tumour colon tissues (**Figure 9E**). The mouse body weight, liver (ALT, AST) and renal (BUN, CREA) function were not obviously altered with the dosage and frequency of *R.i* treatment (**Figure 10A and 10B**). We got consistent results in another carcinogen-induced CRC model in which 6-week-old C57BL/6J mice were injected with 6 dosages AOM weekly, following with 30 weeks administration of *R. intestinalis* (**Figure 9F-K**). What's more, *R. intestinalis* enhanced gut barrier homeostasis (**Figure 11**). Lower serum FITC-dextran (4kd) concentration was detected four hours after its orally gavage, indicating that barrier impairment was milder in *R.i* treated mice (**Figure 11A**). Molecularly, tight junction protein ZO-1 and Cluaindin-1 expression lever was significantly higher with *R.i* treatment (**Figure 11B, 11C**). Taking together, *R. intestinalis* protected mice against colorectal carcinogenesis by deducing tumour incidence, decreasing tumour number and tumour size, and enhancing gut barrier functions under a well-tolerated dosage.

### Roseburia intestinalis cultured medium induces colorectal cancer cell apoptosis and cell cycle arrest

Based on the findings in murine models, we then detected whether *R. intestinalis* itself or its producing metabolites have the antitumour effects in vitro. First, colon cancer cell lines HCT116, LoVo, SW480 and normal epithelial cell line NCM460 were cocultured with alive or pasteurized *E. coli* or *R.i* for 4 hours per day for continuous 3 days, but we didn't observe any difference in cell viability among groups, indicating that *R*. *intestinalis* itself has no effects on colon cell viability with this coculture condition (**Figure 12**).

We then moved to the metabolites produced by *R. intestinalis.* The cell viability of HCT116, LoVo and SW480, but not NCM460 was inhibited after 5% (vol/vol) *R. intestinalis* cultured medium (*R.i* CM) treatment (**Figure 13A**). The results were confirmed by colony formation assay (**Figure 13B**) and nuclear proliferating marker Ki-67 immunocytochemistry staining (**Figure 13C**). Moreover, *R.i* CM induced higher apoptosis cell proportion at both early and late stage in CRC cell lines (**Figure 13D**). Apoptosis markers Cleaved Caspase-7 and Cleaved PARP increased in all three CRC cells, while Cleaved Caspase-3 and Cleaved Caspase-9 increased in HCT116 and SW480 at protein level after *R.i* CM treatment (**Figure 13E**). In addition, HCT116, LoVo and SW480 treated with *R.i* CM showed a cell cycle arrest with an increased cell proportion at GO/G1 phase and a decrease of cells in synthesis (S) (**Figure 13F**). The protein level of cell cycle marker Cdk6 was increased and p27 Kip1 was increased after treatment with *R.i* CM, indicating that *R.i* CM induces G1 phase arrest (**Figure 13G**). Besides of cell lines, CRC patient derived organoid was also treated with *R.i* CM and the surface area of it was extremely lower after the treatment (**Figure 13H****).** The results indicate that metabolites producing by *R. intestinalis* mediated the tumour suppressive effects *in vitro.*

### Butyrate is the main tumour suppressive component produced by Roseburia intestinalis

To determine the effective components in *R.i* CM, we separated it into fractions less than 3 kDa and more than 3 kDa. Only *R. i* CM<3 kDa still kept the tumour-suppressive effects on Colon cancer cells, but not *R.i* CM>3 kDa (**Figure 14A**). *R.i* CM<3 kDa was sent for untargeted metabolomics profiling by LC-MS/MS and butyric acid ranked top ones (**Figure 14B**). The accurate concentration of short chain fatty acid *was measured by targeted gas chromatography-*mass spectrometry (GC-MS), and butyric acid *was markedly enriched in R.i* CM<3 kDa fractions (**Figure 14C**). The concentration of butyric acid in *R.i* CM reached to more than 2000 µg/ml (**Figure 14C**). The elevation of butyrate acid was confirmed in *Apc*^{*Min*/}*⁺ and* AOM-induced mouse faecal materials by GC-MS analysis (**Figure 14D**).

The direct suppressive effect of butyrate on colorectal cancer cells was confirmed by cell viability inhibition (**Figure 15A**), apoptosis inducement (**Figure 15B**) and cell cycle arrest (**Figure 15C**) with the treatment of comparable concentration (1mM) of butyrate acid in 5% *R.i* CM.

In another set of untargeted metabolomics profiling by GC-MS, inosine was the most enriched molecular in *R.i* CM>3 kDa (**Figure 16A**), indicating that inosine might be another tumour suppressive component in *R.i* CM and the concentration of inosine in *R.i* CM is around 8µg/ml (**Figure 16B**). But comparable dosage of inosine in *R.i* CM has no anti-tumour effects in vitro (**Figure 17A-C**).

### Roseburia intestinalis cultured medium and butyrate directly boosts cytotoxic CD8+ T cells in vitro

Besides the direct apoptosis inducement and proliferation inhibition of butyrate and *R. intestinalis* metabolites on tumour cells, we also estimated their antitumour response on CD8⁺ T cells, which play a central role in tumour immunity. T cells were isolated from human *peripheral blood mononuclear cell* or mouse spleen and activated by α-CD3/CD28 and IL-2 with or without *R.i* CM or butyrate (1mM) treatment. The results show that both *R.i* CM and butyrate treatment promoted the production of Granzyme B, IFN-γ and TNF-α in CD8⁺ T cells (**Figure 15D****,** **Figure 18****).** To understand how *R. intestinalis* facilitates CD8⁺ T cells by producing butyrate, the mRNA expression level of receptors and transporters on CD8+ T cells, including *Gpr41, Gpr43, Gpr109a, Slcl6a1, Tlr1 to 6, Id2, Il2ra, Il2rb, Il7r, Il12rb1, Il12rb2, Cd8a, Cd8b1, Cd27, Cd28, Ox40, 4-lbb,* were measured by qPCR with or without *R.i* CM treatment (**Figure 15E****).** Unexpectedly, *R. i* CM treatment didn't change the expression of short chain fatty acid receptors or transporters, but other effector T cell-associated genes, like *Id2, Il2ra* and *Cd28,* and pattern recognition receptor *Tlr5* (**Figure 15E****).** Especially, the *Tlr5* mRNA expression level was more than 10 times higher with *R.i* CM treatment (**Figure 15E****).** Similarly, *Tlr5* expression was much higher in CD8⁺ T cells treated with butyrate (**Figure 15F****).** These data led us to hypothesize that *R. intestinalis* may facilitate CD8⁺ T cell response on a TLRS-dependent pathway. TLR5 captured on Chip CMS can bind butyric acid with an affinity of 264 µM as determined by surface plasmon resonance (SPR) (**Figure 15G**). To confirm our hypothesis, a selective TLRS antagonist TH1020 was used to inhibit TLR5 signalling. Obviously, Th1020 abolished the effective promotion of butyrate on Granzyme B, IFN-γ and TNF-α in CD8⁺ T cells (**Figure 15H****).** Molecularly, both *R.i* CM and butyrate increased NF-κB nuclear translocation with higher phospho-NF-xB expression and inhibited by Th1020 in α-CD3/CD28/IL-2 activated Jurkat E6.1 T cells (**Figure 15I**). Overall, butyrate, produced by *R. intestinalis,* boosted cytotoxic CD8⁺ T cells through TLR5-NF-κB activation.

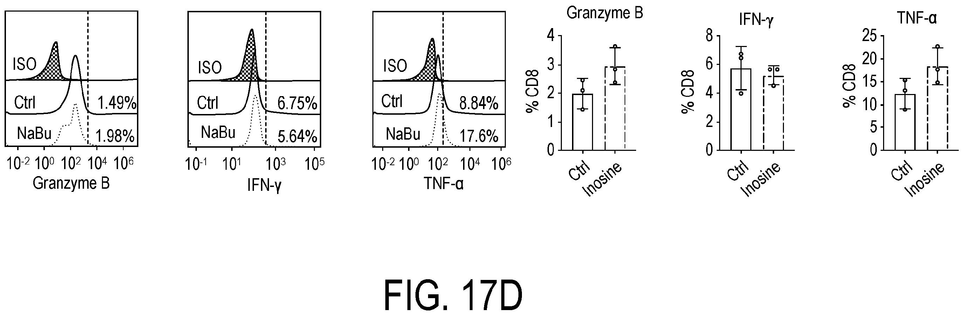

The effect of comparable dosage of inosine (0.4µg/ml) on promotion of Granzyme B, IFN-γ and TNF-α in CD8⁺ T cells was also detected (**Figure 17D****).**

### Supplementation of Roseburia intestinalis and butyrate restricts MC38 and CT-26 orthotopic tumour growth

We next tested whether *R. intestinalis* and butyrate could effectively enhance immunotherapy on colorectal cancer. Treatments of *E. coli, R.i* or sodium butyrate (NaBu) started three days after murine CRC cell MC-38 injection into C57BL/6J mouse's rectum and lasted for three weeks, αPD-1 therapy began after the tumour was established (**Figure 19A****).** *R. intestinalis,* NaBu and αPD-1 treatments inhibited MC38 orthotopic tumour growth (**Figure 19B-D****).** Combination of αPD-1 with *R.i* or NaBu did not enhance the efficiency of αPD-1 therapy; while tumours from *R.i* or NaBu-treated mice are small already (**Figure 19B-D****).** *R*. *intestinalis* or sodium butyrate treatment of MC38 tumours increased infiltrating CD8⁺ T cells (**Figure 19E**) and Granzyme B⁺, IFN-γ⁺ and TNF-α⁺ CD8+ T cells (**Figure 19F**) while decreasing MDSCs (**Figure 19G**). In this αPD-1 sensitive mouse model, *R. intestinalis* and butyrate supplementation is as effective as αPD-1 therapy.

In another MSS-type mouse colorectal tumour model, CT-26 was injected to BALB/c mouse rectum under same experimental design and timeline with last model (**Figure 20A****).** CT-26 was non-sensitive to αPD-1 therapy, but the tumour weight and size are still reduced with *R.i* or NaBu treatment alone compared with αPD-1 therapy and control groups (**Figure 20B-****D).** Moreover, the combination of *R. intestinalis* or sodium butyrate with anti-PD-1 further heightened the anti-tumour effects of *R. intestinalis* or sodium butyrate (**Figure 20B-D**). Consistently, the proportion of infiltrating CD8+ T cells (**Figure 20E**) and Granzyme B⁺, IFN-γ and TNF-α⁺ CD8⁺ T cells was significantly increased in CT26 tumours with monotreatment of *R. intestinalis* or sodium butyrate (**Figure 20F**), and their combination with anti-PD-1 showed additional effects on these immune cells (**Figure 20E-F**). Treatment of *R. intestinalis* or sodium butyrate but not anti-PD-1 monotherapy also reduced the proportion of MDSCs in tumours (**Figure 20G**). *R. intestinalis* and sodium butyrate are effective on facilizing anti-PD-1 therapeutic efficacy against CRC with MSS phenotype.

### DISCUSSION

Faecal butyrate was found closely related to CRC incidence in the inventors' previous study, indicating the potential therapeutic role of butyrate. Detected by LC-MS/MS, the inventors found that the anticancer efficiency from *R. intestinalis* was mainly from butyrate. They then confirmed that the cultured medium of *R. intestinalis* restricted the proliferation of CRC cells but not normal epithelial cells by apoptosis induction and cell cycle arrest. Supplement of sodium butyrate to mice achieved the same anti-tumour response. Butyrate in lumen is the primary energy source for normal colonocytes. Due to the Warburg effect, tumour cells rely on glucose as their primary energy, which lead to the accumulation of butyrate and act as histone deacetylase (HDAC) inhibitor. The Warburg effect dictates the mechanism of butyrate mediated histone acetylation and inhibition of tumour cell proliferation but not normal cells. Directly, by producing butyrate, *R. intestinalis* kills tumour cells to inhibit the progression of CRC.

The promotion role of butyrate producing bacteria *R. intestinalis* on immune checkpoint inhibitor response was confirmed by reanalysing a published metagenomics cohort (**Figure 8D**). Other butyrate producing bacteria such as *Akkermansia muciniphila* and *Faecalibacterium* were also reduced in PD-1 therapy non-responders. Moreover, faecal butyric acid concentration is associated with PD-1 inhibitor efficacy in solid cancer. These association analyses between butyrate concentration or butyrate producing bacteria abundance and immunotherapy response inspired us the therapeutic potential of butyrate on CRC.

Clinically, the major challenge of immunotherapy on CRC patients is the poor response, especially for most patients presenting MSS CRC tumours. Even in MSI-H CRC patients, only 40% of them responded to immune checkpoint inhibitors. In practical mouse experiments, MSS type CT-26 cells are considered as sensitive to immune checkpoint blocking therapy. As illustrated in the results, anti-PD-1 therapies were effective in MC-38 bearing mouse (**Figure 19**), but not CT26 bearing mouse (**Figure 20**). In MC-38 rectum orthotopic mice, both *R. intestinalis* and butyrate were as efficient as anti-PD-1 on tumour shrink. *R*. *intestinalis* and butyrate alone were sufficient to suppress the tumour growth without any additional improvements when combining with anti-PD-1. In CT26 rectum orthotopic mice, the anti-tumour immunity of *R. intestinalis* and butyrate were more obvious when anti-PD-1 therapy was disabled in this model. Thus, *R. intestinalis* provides a promising therapy in CRC patients who are resistant to the current anti-PD-1 therapy.

With a focus on the specific immune cell type, the current immunotherapies are centred on CD8⁺ T cells, which are the most powerful anticancer immune effectors by recognizing and killing tumour cells with the cytotoxic molecules. However, the direct role of microbiota on the CD8⁺ T cells functional regulation needs further study. Different from other existing studies, in which bacteria only served as enhancers of immunotherapy, the inventors found that *R. intestinalis* itself could promote anti-tumour cytotoxic CD8⁺ T cell response directly through its metabolite butyrate, independently from anti-PD1 therapy, especially in MSS-type CT-26 bearing mice.

Molecularly downstream, short chain fatty acids (SCFAs) interact with metabolite-sensing G protein-coupled receptors GPR 41, GPR 43 and GPR109A on epithelial cells. But in the inventors' study, stimulation of butyrate didn't change the mRNA expression of *Gpr 41, Gpr 43* and *Gpr109a,* but *Tlr5* on CD8⁺ T cells. In another study, treating isolated CD8⁺ T cells from GPR43 or GPR109a KO mice with butyrate still induced cytotoxic CD8⁺ T cells and blocking GPR-signaling with pertussis toxin did not impair the effect of butyrate on functional CD8⁺ T cells, which indicating that the promotion of butyrate on CD8⁺ T cell function may not depend on GPR receptors. Butyrate increased the binding of the transcription factor specificity protein 3 to the TLR5 promotor regions to upregulate the transcription of TLR5, which confirm the association of butyrate with TLRS at the transcription level indirectly, but whether butyrate directly binding to TLRS is unknown. The inventors confirmed the binding affinity of butyric acid to TLRS by surface plasmon resonance (SPR), indicating that TLR5 might be a butyrate receptor, at least on CD8⁺ T cells. TLR5, as one of the pathogen-associated molecular patterns (PAMPs), expressed in epithelial cells and immune cells, specifically recognize flagellin on bacteria. Activation of TLRS translocated the nuclear factor NF-κB and stimulated the production of TNF-α. TH1020, identified as an antagonist of TLR5, competed with flagellin and disrupted the association with TLRS based on molecular docking simulation, repressed the expression of downstream TNF-α signaling pathways. In this study, the effects of butyrate on CD8⁺ cells were abolished by TH1020, with the repression of Granzyme B, IFN-γ, TNF-α and NF-κB mobilization. Overall, butyrate directly boosting cytotoxic CD8⁺ T cells through TLRS stimulation and NF-xB signalling activation.

The inventors thus discovered in human faecal sequencing data, *R. intestinalis* and butyrate were depleted in CRC patients compared to healthy controls. By using multiple mouse models, they demonstrated the preclinical efficiency of *R. intestinalis* supplements as monotherapy on restricting colorectal tumour growth through butyrate, especially on MSS-type CT-26 tumours. Molecularly, *R. intestinalis* cultured medium and butyrate suppressed CRC by directly inducing tumour cell apoptosis, arresting cell cycle, and mediating cytotoxic Granzyme B⁺, IFN-γ⁺, TNF-α⁺ CD8⁺ T cells through TLRS dependent NF-xB signalling. *R*. *intestinalis,* as a promising probiotics supplement, opened a new way to expand the CRC treatment efficiency, particularly for immunotherapy resistant patients.

### EXAMPLE 3: Streptococcus salivarius k12 Reprograms Tumour Immune Microenvironment to Synergize with Anti-PD1 to Suppress Colorectal Tumourigenesis

### BACKGROUND OF THE INVENTION

Colorectal cancer is one of the leading cancer deaths worldwide. Immune checkpoint inhibitors (ICI), especially, monoclonal antibodies (mAbs) directed at the PD-1/PD-L1 (programmed cell death protein-1/programmed death ligand-1) axis has revolutionized cancer treatment. However, only a minority of patients respond to these immune-oncology therapies, and undeniably, a multitude of ICI-associated immune-related adverse effects (irAEs) remain a clinical challenge. Identification of optimized therapeutic analogous, immunostimulatory molecules that trigger activations of innate and adaptive immune responses to facilitate the successful clinical immunotherapy, is therefore highly warranted.

In recent years, the "favorable" and "unfavorable" gut microbiota has been highlighted in the immune-oncology study. Several studies have pointed out the involvement of some specific probiotics in modulating the response to immunotherapy. Of note, administration of *Bifidobacterium longum* and *Bifidobacterium breve* cocktail to TAC mice is sufficient to control the tumour growth by increasing anticancer T cells to the same extent as anti-PD-L1 through activating dendritic cell-mediated immune responses, whereas the synergistic effect from combined *Bifidobacterium spp* with PD-1/PD-L1 blockade can almost abolish the tumour growth.

Using shotgun metagenomic sequencing, the inventors identified *S. salivarius* as the most significantly depleted probiotic in the stool samples of patients with CRC, indicating its possible role in preventing colorectal tumourigenesis. As there has been no reported functional role *of S. salivarius* (M18) in suppressing CRC, and due to the strain-specific efficacy of the probiotics, the inventors further evaluated the protective role of *S. salivarius* (K12) and a healthy human isolated strain on colorectal tumourigenesis. They found the specific tumour suppressive effect of *S*. *salivarius* (K12) as compared with *S. salivarius* (M18) and *S. salivarius* (Human). Through bacterial whole genome sequencing (WGS), the inventors found the *S*. *salivarius* (K12) contains a specific gene cluster responsible for exopolysaccharides (EPS) production. They also revealed that *S. salivarius* (K12) can augment the intratumoural effector CD8+ T cells infiltration, which turns the "cold" tumours into "hot" tumours to potentiate the anti-PD1 response in CRC.

### MATERIALS AND METHODS

### Animal experiments

Animal models were established the same as described previously. In brief, C57BL/6J*-ApcMin*/J mice, which develop intestinal polyps spontaneously were used as a model of spontaneous intestinal neoplasia. They were purchased from the Jackson Laboratory (Bar Harbor, ME, USA) and maintained in the animal facility at the Chinese University of Hong Kong. Mice at 4-5 weeks old were divided into 3 groups - 1×10⁸ colony forming units (CFU) *of S. salivarius* or *E. coli* MG1655, or the same volume of BHI was gavaged to them once daily for 10 weeks. *Faecalibacterium prausnitzii,* which was reported to have an immune modulatory effect, was used as a positive control. Colorectal tumour formation was monitored by mouse colonoscopy (Coloview, Karl Stroz, Germany). Colonic tissues were collected for immune profiling.

For the carcinogen-induced CRC mouse model, male C57BL/6 mice at 6 weeks old were intraperitoneally injected with a single dose of 10mg/kg AOM (Merck, Darmstadt, Germany), followed by 2% DSS (MP Biomedicals, Solon, OH) administration for 1 week. After DSS treatment, the same treatment regimen with the *Apc*^{*min*/*+*} mice was used in this AOM/DSS-induced CRC model. Mice were raised to 20 weeks for the evaluation of the probiotic treatment efficacy.

For the xenograft model, male C57BL/6 mice or BALB/c mic at 5 weeks old were pre-treated with 1×10⁸ CFU *S. salivarius. E. coli* MG1655, or the same volume of BHI for 2 weeks, following which, the mice were subcutaneously injected with MC38 (5×10⁵ cells) or CT26 (1×10⁶ cells) into the flank, respectively. Tumour volume was measured with a digital caliper every other day after the outgrowth of the tumour. The anti-mouse programmed cell death protein 1 (anti-PD1) monoclonal antibody (BE0146, Bio X Cell, Lebanon, NH) or IgG isotype control (BE0089, Bio X Cell) was administrated to the CT26 syngeneic mice to evaluate the synergistic effect *of S. salivarius* in improving anti-PD1 response. At the end of the experiment, tumour tissues were collected for evaluating the changes of the tumour immune prefile. All the procedures were performed in accordance with guidelines approved by the Animal Experimentation Ethics Committee of The Chinese University of Hong Kong.

### Bacterial strains and culture conditions

*S. salivarius* (M18 and K12) were purchased from American Type Culture Collection (ATCC; Manassas, VA). *E. coli* MG1655 was obtained from DSMZ. They were cultured in BHI broth (Thermo Fisher Scientific, West Palm Beach, FL) at 37°C under aerobic conditions. The bacteria were centrifuged at 5,000 rpm for 10 minutes to obtain the bacteria pallet and resuspended in BHI before gavaging to mice. The *S. salivarius* conditioned medium (Ss. CM) was obtained by centrifuging the bacteria culture supernatant at 4500g for 15 mins and filtered through a 0.2-µm pore-size filter.

For the *S. salivarius* (human isolation), healthy human faecal samples were collected and suspended in PBS within 30 minutes. After homogenization, the suspension was filtered through a 100µm strainer to remove larger particles. Serial dilution was then performed by using the faecal suspension and the BHI agar plate was further used to incubate the suspension in an aerobic incubator at 37 °C for 24 hours. Colonies with a similar appearance to *S*. *salivarius* were purified by streak plated method. The 16S rDNA for each isolate was amplified by PCR with the university primers 341F (5'- CCTAYGGGRBGCASCAG-3', SEQ ID NO: 1) and 806R (5'-GGACTACNNGGGTATCTAAT-3', SEQ ID NO:2). *S*. *salivarius* (human) was identified by blasting the 16S rDNA in the GenBank.

### Cell culture

Colon cancer cell lines, including HCT116, Lovo, MC38, and CT26, were obtained from American Type Culture Collection (ATCC). Normal colonic epithelial cell line NCM460 was obtained from INCELL Corporation (San Antonio, TX). All the cell lines were cultured in high-glucose Dulbecco's Modified Eagle's Medium (DMEM) (Thermo Fisher Scientific) supplemented with 10% (vol/vol) fetal bovine serum (FBS) (Thermo Fisher Scientific), 1% penicillin/ streptomycin in a humidified atmosphere containing 5% CO₂. For co-culture of epithelial cells with bacteria, the penicillin/streptomycin in the culture medium was removed and cells were exposed to bacteria with a multiplicity of infection (MOI) of 200 for 2 hours. Cell viability was determined by 3-(4,5-dimethylthiazoly-2-yl)-2,5-diphenyltetrazoliumbromide (MTT) (Sigma-Aldrich) assay.

### Multicolor flow cytometry analysis

Tumour tissues were dissected into small pieces and digested with RPMI 1640 with 0.1mg/ml collagenase D and 50U/ml DNase I for 1h at 37°C on a shaking platform. The cell suspension was filtered through a 70-µm cell strainer and centrifuged at 700 g for 10 min. The cell pellet was resuspended in PBS plus 1% bovine serum albumin (BSA) for cell surface marker analysis. For intracellular cytokine staining, stimulation was performed for the isolated cell suspension before surface marker staining. In brief, the primary isolated cells were incubated in RPMI 1640 containing phorbol myristate acetate (PMA), ionomycin, and monensin for 4 hours at 37°C. The stimulated cells were then permeabilized by an intracellular staining kit (eBioscience) and stained with anti-IFNγ, anti-TNF-α, and anti-GZMB overnight before multicolor flow cytometry analysis.

### Bacteria whole genome sequencing (WGS)

The WGS of the 3 bacterial strains was conducted by Beijing Novogene Technology. Bacterial genomic DNAs were extracted by using the ZR Faecal DNA MiniPrep (Zymo Research, Irvine, CA) following the manufacturer's instructions.

### Statistical analysis

Results are expressed as mean ± standard deviation (SD). ANOVA was used to compare differences among multiple groups, and post-hoc analysis was performed by Tukey's multiple comparisons test. P-values < 0.05 indicate statistical significance. Statistical tests were performed in GraphPad Prism (V8.0, GraphPad Software Inc., San Diego, CA).

### RESULTS

### S. salivarius (K12) and its conditioned medium inhibit the viability of colon cancer cells

To evaluate the strain-specific anti-CRC effect of *S. salivarius,* colon cancer cells, including HCT116, Lovo, and colon normal epithelial cell line NCM460 were cocultured with 3 different strains of *S*. *salivarius* (MOI = 200, 2h). *E. coli* strain MG1655 was used as a control. We found that, compared with *S. salivarius* (M18) and *S. salivarius* (Human), only *S. salivarius* (K12) suppressed the CRC cells' viability (**Figure 21A**). Concordantly, the conditioned medium from *S. salivarius* (K12) (K12.CM) also revealed a solely inhibitory effect in suppressing the viability of colon cancer cells, but not the normal epithelial cells (**Figure 21B**), indicating that only the *S. salivarius* (K12) strain contribute to the direct antitumourigenic effect.

We next determined whether such a strain-specific function was attributed to the specific genetic background, 3^{rd} generation sequencing was then performed on the 3 different strains of *S*. *salivarius.* We discovered that *S. salivarius* (K12) contains a gene cluster responsible for EPS production (**Figure 21C**). Those results indicate that *S. salivarius* containing EPS gene cluster can suppress the viability of CRC cells.

### S. salivarius (K12) protects against intestinal tumourigenesis and augments effector CD8+ T-cell infiltration in Apc^{min/+} mice

To investigate the effect of *S. salivarius* (K12) on colorectal tumourigenesis, the inventors gavaged the mice with *S. salivarius* (K12), BHI and a non-tumourigenic *E. coli* strain MG1655 were used as plain culture broth and bacteria control, respectively (**Figure 22A**). After 10-week gavage, they observed that administration of *S. salivarius* (K12) significantly reduced the tumour number (**Figure 22B**) and tumour size (**Figure 22C**) in the colon and small intestine of *Apc*^{*min*/*+*} mice. They also examined the change of immune landscape induced by *S*. *salivarius* (K12). It was found that tumour-infiltrating cytotoxic T cells (CD8+) were significantly enhanced in the *S. salivarius* (K12)-treated *Apc*^{*min*/*+*} mice as compared with the BHI groups. The CD8+ effector T cells, granzyme B (GZMB)+ CD8+ T cells also enhanced significantly in the *S. salivarius* (K12)-treated *Apc*^{*min*/*+*} mice (**Figure 22D**), indicating the *S*. *salivarius* (K12) can abrogate intestinal tumourigenesis and modulate immune microenvironment in *Apc*^{*min*/*+*} mice.

### S. salivarius (K12) protects against intestinal tumourigenesis and augments effector CD8+ T-cell infiltration in AOM/DSS-induced CRC mice

To validate the tumour-suppressive and immune modulatory effect of *S. salivarius* (K12) on colorectal tumourigenesis, the inventors established a colitis-associated CRC mouse model, in which DSS-promoted, AOM-induced CRC mice were treated with *S. salivarius* (K12). BHI and a non-tumourigenic *E. coli* strain MG1655 were used as plain culture broth and bacteria control, respectively. *F. prausnitzii,* a CRC immune-modulating bacteria, was used as a positive control (**Figure 23A**). After 14-week gavage, colonoscopy identified that colon tumour sizes in *S. salivarius* (K12) treated group were visually smaller than the *E. coli* MG1655 or broth control groups (**Figure 23B**). In accordance with this observation, the inventors observed consistent results as in the *Apc*^{*min*/*+*} mice (**Figure 23C and Figure 23D**).

### S. salivarius (K12) protects against intestinal tumourigenesis and augments effector CD8+ T-cell infiltration in MC38 syngeneic mouse model

MC38 syngeneic model is an immunoresponsive murine tumour model. The inventors administered *S. salivarius* (K12) to the MC38 (MSI-H) syngeneic mouse model. BHI was used as broth control, *F. prausnitzii* was used as a positive control (**Figure 24A**). *S*. *salivarius* (K12) significantly alleviate tumour growth and tumour weight (**Figure 24B**). Of note, the suppressive effect was similar to *F*. *prausnitzii* (**Figure 24C**). Moreover, the *S*. *salivarius* (K12) monotherapy also enhanced the tumour-infiltrating effector CD8+ cytotoxic T cells (**Figure 24D**). These results indicate that *S*. *salivarius* (K12) also functions in the MC38 syngeneic model to strengthen antitumour immunity.

### S. salivarius (K12) reprograms the tumour immune microenvironment to synergize with anti-PD1 to suppress colorectal tumourigenesis in the CT26 syngeneic model

CT26 (MSS CRC cell line) syngeneic model is an immunotherapy resistance murine tumour model. No single-agent anti-PD1/PD-L1 therapy has shown any efficacy against this tumour. To investigate whether *S. salivarius* (K12) plus anti-PD1 exerts a synergistic CRC-suppressive effect, the CT26 syngeneic model was treated with *S. salivarius* (K12). BHI and *E.coli* were used as control. After 2 weeks of pre-treatment, the CT 26 mouse CRC cells were transplanted to the flank of the mice. When the tumour developed, anti-PD1 antibody or IgG isotype control was intraperitoneally injected into the mice (**Figure 25A and Figure 25B**). Monotherapy of *S. salivarius* (K12) slightly decreased the tumour size and tumour weight (**Figure 25C**). Most strikingly, combined *S. salivarius* (K12) with anti-PD1 led to an obvious reduction of the tumour size and tumour weight **(****Figure 25C****)** as compared with *S. salivarius* (K12) alone or *BHI*/*E. coli* + anti-PD 1, indicating combined *S. salivarius* (K12) with anti-PD synergistically abrogates CT26 syngeneic model. The inventors also evaluated the tumour-infiltrating CD8+ cytotoxic T cells, they found the combination therapy led to the significant induction of effector CD8+ T cells **(****Figure 25D****).** Collectively, these results indicate that *S. salivarius* (K12) enhances antitumour immunity in CRC.

### DISCUSSION

In this study, the inventors demonstrate for the first time the strain-specific anti-CRC effect of *S. salivarius.* Oral administration of the *S. salivarius* K12, which contains the EPS production gene cluster suppresses tumour formation in 3 murine models of CRC (*Apc*^{*min*/}*⁺,* AOM/DSS, and MC38 syngeneic models). The tumour-infiltrating effector CD8+ T cells were also enhanced in the *S. salivarius* K12-treated CRC mice. Furthermore, *S. salivarius* K12 synergistically with anti-PD1 in the immunotherapy resistance murine tumour model (CT26 syngeneic model) to suppress the tumour size and enhance the effector CD8+ T cells infiltration. These results strongly illustrate *S. salivarius* K12 as a prophylactic for modulating antitumour immunity and improving anti-PD1 response.

All patents, patent applications, and other publications, including GenBank Accession Numbers and equivalents, cited in this application are incorporated by reference in the entirety for all purposes.

## Claims

1. A composition for use in enhancing efficacy of immunotherapy for colorectal cancer in a subject in need thereof, comprising an effective amount of (1) any one or more of *Lactobacillus gallinarum, Roseburia intestinalis,* and *Streptococcus salivarius* K12; and (2) a physiologically acceptable excipient.

2. The composition of claim 1, comprising an effective amount of two or three of *Lactobacillus gallinarum, Roseburia intestinalis,* and *Streptococcus salivarius* K12.

3. The composition of claim 1, comprising a total of about 10⁶ to about 10¹⁴ colony-forming unit (CFU) of any one or any two or all three of *Lactobacillus gallinarum, Roseburia intestinalis,* and *Streptococcus salivarius* K12.

4. The composition of claim 1, further comprising one or more of: a total of about 10⁶ to about 10¹⁴ colony-forming unit (CFU) of any one or any two or all three of *Lactobacillus gallinarum, Roseburia intestinalis,* and *Streptococcus salivarius* K12; and optionally further comprising one or more of: indole-3-carboxylic acid (ICA) and/or butyrate.

5. The composition of claim 1, further comprising one or more of: a total of about 10⁶ to about 10¹⁴ colony-forming unit (CFU) of any one or any two or all three of *Lactobacillus gallinarum, Roseburia intestinalis,* and *Streptococcus salivarius* K12; indole-3-carboxylic acid (ICA); and butyrate, and wherein the composition is formulated for oral ingestion.

6. The composition of claim 5, which is in the form of a food or beverage item.

7. The composition of claim 1, wherein the composition is formulated in a daily dosage comprising about 5 × 10⁹ CFU of *L. gallinarum* per kg recipient bodyweight and/or formulated in a daily dosage comprising about 5 × 10⁹ CFU of *R. intestinalis* per kg recipient bodyweight and/or formulated in a daily dosage comprising about 5 × 10⁹ CFU of*S*. *salivarius* K12 per kg recipient bodyweight.

8. A method for augmenting immunotherapy efficacy in the treatment of colorectal cancer in a subject, comprising administering to the subject an effective amount of a composition containing an effective amount of (1) any one or more of *Lactobacillus gallinarum, Roseburia intestinalis,* and *Streptococcus salivarius* K12; and (2) one or more physiologically acceptable excipients.

9. The method of claim 8, wherein the immunotherapy comprises administration of an effective amount of a PD1 inhibitor or a PDL1 inhibitor.

10. The method of claim 9, wherein the PD1 inhibitor is an anti-PD1 antibody.

11. The method of 9, wherein the PD1 inhibitor or PDL1 inhibitor is administered via injection.

12. The method of claim 11, wherein the injection is intravenous or intraperitoneal.

13. The method of claim 8, comprising administering to the subject one composition comprising any one or more of *Lactobacillus gallinarum, Roseburia intestinalis,* and *Streptococcus salivarius* K12.

14. The method of claim 8, comprising administering to the subject two or more compositions comprising any two or all three of *Lactobacillus gallinarum, Roseburia intestinalis,* and *Streptococcus salivarius* K12, optionally further comprising ICA and/or butyrate.

15. The method of claim 8, wherein the administering step comprises oral ingestion of the composition, and wherein the oral ingestion is optionally prior to or with food intake.

16. A kit for enhancing efficacy of immunotherapy for colorectal cancer in a subject, comprising two compositions, the first composition comprising an effective amount of any one or two or three of *Lactobacillus gallinarum, Roseburia intestinalis,* and *Streptococcus salivarius* K12, and the second composition comprising an effective amount of a PD-1 inhibitor or a PD-1L inhibitor.

17. The kit of claim 16, wherein the first composition is formulated for oral ingestion and/or wherein the second composition is formulated for injection.

18. The kit of claim 16, wherein the first composition is formulated in a daily dosage comprising about 5 × 10⁹ CFU of *S. salivarius* K12 per kg recipient bodyweight.
